# EUROPÄISCHE PATENTANMELDUNG

(11) **EP 2 298 305 A1**
(43) Veröffentlichungstag der Anmeldung: **23.03.2011**
(21) Anmeldenummer: 10180130.6
(22) Anmeldetag: 25.03.2003
(51) Int. Cl.: A61K 31/517, C07D 239/94, C07D 405/12, C07D 401/12, C07D 413/12, C07D 403/12, C07D 498/08, C07D 491/08, A61P 35/00

(54) **4-(N-phenylamino)-chinazoline/chinoline als tyrosinkinaseinhibitoren**

(30) Priorität: 30.03.2002 DE 10214412
(62) Teilanmeldung aus: 03745271.1
(71) Anmelder: Boehringer Ingelheim Pharma GmbH & Co. KG, 55216 Ingelheim am Rhein (DE)
(72) Erfinder: Himmelsbach, Frank, 55216, INGELHEIM AM RHEIN (DE); Jung, Birgit, 55216, INGELHEIM AM RHEIN (DE); Solca, Flavio, 55216, INGELHEIM AM RHEIN (DE)
(74) Vertreter: Hammann, Heinz

(57) **Zusammenfassung**

Die vorliegende Erfindung betrifft bicyclische Heterocyclen der allgemeinen Formel 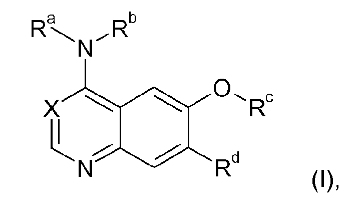 in der
R^{a}, R^{b}, R^{c}, R^{d} und X wie im Anspruch 1 definiert sind, deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

## Beschreibung

Gegenstand der vorliegenden Erfindung sind bicyclische Heterocyclen der allgemeinen Formel 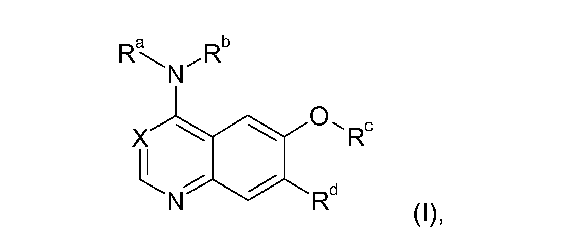 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

In der obigen allgemeinen Formel I bedeutet
R^{a} ein Wasserstoffatom oder eine C₁₋₄-Alkylgruppe,
R^{b} eine Phenyl- oder 1-Phenylethylgruppe, in denen der Phenylkern jeweils durch die Reste R¹ bis R³ substituiert ist, wobei
   R¹ und R², die gleich oder verschieden sein können, jeweils ein Wasserstoff-, Fluor-, Chlor-, Brom- oder Jodatom,
   eine C₁₋₄-Alkyl-, Hydroxy-, C₁₋₄-Alkoxy-, C₂₋₃-Alkenyl- oder C₂₋₃-Alkinylgruppe,
   eine Aryl-, Aryloxy-, Arylmethyl- oder Arylmethoxygruppe,
   eine Heteroaryl-, Heteroaryloxy-, Heteroarylmethyl- oder Heteroarylmethoxygruppe,
   eine durch 1 bis 3 Fluoratome substituierte Methyl- oder Methoxygruppe oder eine Cyano-, Nitro- oder Aminogruppe, und
   R³ ein Wasserstoff-, Fluor-, Chlor- oder Bromatom oder
   eine Methyl- oder Trifluormethylgruppe darstellen,
R^{c} eine Cyclobutyl-, Cyclopentyl- oder Cyclohexylgruppe, die jeweils durch eine Gruppe R⁴-N-R⁵ substituiert ist, wobei
   - R⁴: ein Wasserstoffatom oder eine C₁₋₃-Alkylgruppe und
   - R⁵: ein Wasserstoffatom oder eine C₁₋₃-Alkylgruppe,

   eine Aminocarbonyl-C₁₋₃-alkyl-, C₁₋₃-Alkylaminocarbonyl-C₁₋₃-alkyl-, Di-(C₁₋₃-alkyl)aminocarbonyl-C₁₋₃-alkyl-, Pyrrolidin-1-ylcarbonyl-C₁₋₃-alkyl-, Piperidin-1-ylcarbonyl-C₁₋₃-alkyl-, Homopiperidin-1-ylcarbonyl-C₁₋₃-alkyl-, Morpholin-4-yl-carbonyl-C₁₋₃-alkyl-, Homomorpholin-4-ylcarbonyl-C₁₋₃-alkyl-, Piperazin-1-ylcarbo-nyl-C₁₋₃-alkyl-, 4-C₁₋₃-Alkyl-piperazin-1-ylcarbonyl-C₁₋₃-alkyl-, Homopiperazin-1-yl-carbonyl-C₁₋₃-alkyl- oder eine 4-C₁₋₃-Alkyl-homopiperazin-1-ylcarbonyl-C₁₋₃-alkylgruppe,
   eine Hydroxy-C₂₋₄-alkyl-, C₁₋₃-Alkyloxy-C₂₋₄-alkyl-, C₁₋₄-Alkyloxy-carbonylamino-C₂₋₄-alkyl-, Amino-C₂₋₄-alkyl-, C₁₋₃-Alkylamino-C₂₋₄-alkyl-, Di-(C₁₋₃-alkyl)amino-C₂₋₄-alkyl-, C₁₋₃-Alkylcarbonylamino-C₂₋₄-alkyl-, Aminocarbonylamino-C₂₋₄-al kyl-, C₁₋₃-Alkylaminocarbonylamino-C₂₋₄-alkyl-, D i-(C₁₋₃-alkyl)amino-carbonylamino-C₂₋₄-alkyl-, Pyrrolidin-1-ylcarbonylamino-C₂₋₄-alkyl-, Piperidin-1-ylcarbonylamino-C₂₋₄-alkyl-, Morpholin-4-ylcarbonylamino-C₂₋₄-alkyl-, C₁₋₃-Alkylsulfonyl-C₂₋₄-alkyl- oder eine C₁₋₃-Alkylsulfonylamino-C₂₋₄-alkylgruppe,
   eine (2-Oxo-pyrrolidin-1-yl)-C₂₋₄-alkyl-, (2-Oxopiperidin-1-yl)-C₂₋₄-alkyl-, (3-Oxo-morpholin-4-yl)-C₂₋₄-alkyl-, (2-Oxo-imidazolidin-1-yl)-C₂₋₄-alkyl-, (2-Oxo-3-C₁₋₃-alkyl-imidazolidin-1-yl)-C₂₋₄-alkyl-, (2-Oxo-hexahydropyrimidin-1-yl)-C₂₋₄-alkyl- oder eine (2-Oxo-3-C₁₋₃-alkyl-hexahydropyrimidin-1-yl)-C₂₋₄-alkylgruppe,
   eine C₁₋₄-Alkylsulfonyl-, Chlor-C₁₋₄-alkylsulfonyl-, Brom-C₁₋₄-alkylsulfonyl-, Amino-C₁₋₄-alkylsulfonyl-, C₁₋₃-Alkylamino-C₁₋₄-alkylsulfonyl-, Di-(C₁₋₃-alkyl)amino-C₁₋₄-alkylsulfonyl-, (Pyrrolidin-1-yl)-C₁₋₄-alkylsulfonyl-, (Piperidin-1-yl)-C₁₋₄-alkylsulfonyl-, (Homopiperidin-1-yl)-C₁₋₄-alkylsulfonyl-, (Morpholin-4-yl)-C₁₋₄-alkylsulfonyl-, (Homomorpholin-4-yl)-C₁₋₄-alkylsulfonyl-, (Piperazin-1-yl)-C₁₋₄-alkylsulfonyl-, (4-C₁₋₃-Alkyl-piperazin-1-yl)-C₁₋₄-alkylsulfonyl-, (Homopiperazin-1-yl)-C₁₋₄-alkylsulfonyl- oder eine (4-C₁₋₃-Alkyl-homopiperazin-1-yl)-C₁₋₄-alkylsulfonylgruppe,
   eine C₁₋₄-Alkyloxycarbonylgruppe,
   eine Formyl-, C₁₋₄-Alkyl-carbonyl-, C₁₋₃-Alkyloxy-C₁₋₄-alkyl-carbonyl-, Tetrahydrofuranylcarbonyl-, Tetrahydropyranylcarbonyl-, Amino-C₁₋₄-alkylcarbonyl-, C₁₋₃-Alkylamino-C₁₋₄-alkyl-carbonyl-, Di-(C₁₋₃-alkyl)amino-C₁₋₄-alkylcarbonyl-, Pyrrolidin-1-yl-C₁₋₄-alkyl-carbonyl-, Piperidin-1-yl-C₁₋₄-alkyl-carbonyl-, (Homopiperidin-1-yl)-C₁₋₄-alkyl-carbonyl-, Morpholin -4-yl-C₁₋₄-alkyl-carbonyl-, (Homomorpholin-4-yl)-C₁₋₄-alkyl-carbonyl-, (Piperazin-1-yl)-C₁₋₄-alkyl-carbonyl-, (4-C₁₋₃-Alkyl-piperazin-1-yl)-C₁₋₄-alkyl-carbonyl-, (Homopiperazin-1-yl)-C₁₋₄-alkylcarbonyl-, (4-C₁₋₃-Alkyl-homopiperazin-1-yl)-C₁₋₄-alkyl-carbonyl- oder eine C₁₋₃-Alkylsulfonyl-C₁₋₄-alkyl-carbonylgruppe,
   eine Cyano-, Aminocarbonyl-, C₁₋₃-Alkyl-aminocarbonyl-, Di-(C₁₋₃-alkyl)aminocarbonyl-, (C₁-₃-Alkyloxy-C₂₋₄-alkyl)aminocarbonyl-, N-(C₁₋₃-Alkyl)-N-(C₁₋₃-alkyloxy-C₂₋₄-alkyl)aminocarbonyl-, Arylaminocarbonyl-, Pyrrolidin-1-ylcarbonyl-, Piperidin-1-ylcarbonyl-, Homopiperidin-1-ylcarbonyl-, Morpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, Piperazin-1-ylcarbonyl-, 4-C₁₋₃-Alkyl-piperazin-1-ylcarbonyl-, Homopiperazin-1-ylcarbonyl-, 4-C₁₋₃-Alkyl-homopiperazin-1-ylcarbonyl-, Aminosulfonyl-, C₁₋₃-Alkyl-aminosulfonyl-, D i-(C₁₋₃-alkyl)amino-sulfonyl-, Pyrrolidin-1-yl-sulfonyl-, Piperidin-1-ylsulfonyl-, Homopiperidin-1-ylsulfonyl-, Morpholin-4-ylsulfonyl-, Homomorpholin-4-ylsulfonyl-, Piperazin-1-ylsulfonyl-, 4-C₁₋₃-Alkyl-piperazin-1-ylsulfonyl-, Homopiperazin-1-ylsulfonyl- oder eine 4-C₁₋₃-Alkyl-homopiperazin-1-ylsulfonylgruppe darstellen,
eine Cyclobutyl-, Cyclopentyl- oder Cyclohexylgruppe, die jeweils durch eine Gruppe R⁶ substituiert ist, wobei
   R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-C₁₋₃-alkyl-imidazolidin-1-yl-, 2-Oxo-hexahydropyrimidin-1-yl- oder eine 2-Oxo-3-C₁₋₃-alkyl-hexahydropyrimidin-1-ylgruppe darstellt,
eine Azetidin-3-ylgruppe, die in 1-Stellung durch den Rest R⁵ substituiert ist, wobei R⁵ wie vorstehend erwähnt definiert ist,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist,
oder eine Tetrahydrofuran-3-yl-, Tetrahydropyran-3-yl- oder Tetrahydropyran-4-yl-gruppe,
R^{d} ein Wasserstoffatom oder ein Fluor-, Chlor- oder Bromatom,
eine Hydroxygruppe,
eine C₁₋₄-Alkyloxygruppe,
eine durch 1 bis 3 Fluoratome substituierte Methoxygruppe,
eine durch 1 bis 5 Fluoratome substituierte Ethyloxygruppe,
eine C₂₋₄-Alkyloxygruppe, die durch einen Rest R⁶ oder R⁷ substituiert ist, wobei
   R⁶ wie vorstehend erwähnt definiert ist und
   R⁷ eine Hydroxy-, C₁₋₃-Alkyloxy-, C₃₋₆-Cycloalkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo-[3.2.1]oct-3-yl-, Piperazin-1-yl-, 4-C₁₋₃-Alkyl-piperazin-1-yl-, Homopiperazin-1-yl- oder C₁₋₃-Alkyl-homopiperazin-1-ylgruppe, oder
   eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino-, Morpholin-4-ylcarbonylamino- oder eine C₁₋₄-Alkylsulfonylamino-Gruppe darstellt,
eine C₃₋₇-Cycloalkyloxy- oder C₃₋₇-Cycloalkyl-C₁₋₄-alkyloxygruppe,
eine Tetrahydrofuran-3-yloxy-, Tetrahydropyran-3-yloxy- oder Tetrahydropyran-4-yloxy-gruppe,
eine Tetrahydrofuranyl-C₁₋₄-alkyloxy- oder Tetrahydropyranyl-C₁₋₄-alkyloxygruppe,
eine C₁₋₄-Alkoxygruppe, die durch eine in 1-Stellung durch den Rest R⁸ substituierte Pyrrolidinyl-, Piperidinyl- oder Homopiperidinylgruppe substituiert ist, wobei
   R⁸ ein Wasserstoffatom oder eine C₁₋₃-Alkylgruppe darstellt,
oder eine C₁₋₄-Alkoxygruppe, die durch eine in 4-Stellung durch den Rest R⁸ substituierte Morpholinylgruppe substituiert ist, wobei R⁸ wie vorstehend erwähnt definiert ist, und
X eine durch eine Cyanogruppe substituierte Methingruppe oder ein Stickstoffatom bedeuten, und
wobei unter den bei der Definition der vorstehend genannten Reste erwähnten Arylgruppen jeweils eine Phenylgruppe zu verstehen ist, die durch R⁹ mono- oder disubstituiert ist, wobei die Substituenten gleich oder verschieden sein können und
   R⁹ ein Wasserstoffatom, ein Fluor-, Chlor-, Brom- oder lodatom oder eine C₁₋₃-Alkyl-, Hydroxy-, C₁₋₃-Alkyloxy-, Difluormethyl- Trifluormethyl-, Difluormethoxy-, Trifluormethoxy- oder Cyanogruppe darstellt,
unter den bei der Definition der vorstehend genannten Reste erwähnten Heteroarylgruppen eine Pyridyl-, Pyridazinyl-, Pyrimidinyl- oder Pyrazinylgruppe zu verstehen ist, wobei die vorstehend erwähnten Heteroarylgruppen jeweils durch den Rest R⁹ mono- oder disubstituiert sind, wobei die Substituenten gleich oder verschieden sein können und R⁹ wie vorstehend erwähnt definiert ist, und
die vorstehend erwähnten Pyrrolidinyl-, Piperidinyl-, Piperazinyl- und Morpholinylgruppen jeweils durch eine oder zwei C₁₋₃-Alkylgruppen substituiert sein können, und
soweit nichts anderes erwähnt wurde, die vorstehend erwähnten Alkylgruppen geradkettig oder verzweigt sein können,
mit der Maßgabe, daß die Verbindung 4-[(3-Chlor-4-fluor-phenyl)amino]-6-((S)-tetrahydrofuran-3-yloxy)-7-hydroxy-chinazolin ausgeschlossen ist.

Bevorzugte Verbindungen der obigen allgemeinen Formel I sind diejenigen, in denen
R^{a} ein Wasserstoffatom,
R^{b} eine durch die Reste R¹ bis R³ substituierte Phenylgruppe, wobei
   R¹ ein Wasserstoff-, Fluor-, Chlor- oder Bromatom,
   eine Methyl-, Trifluormethyl- oder Ethinylgruppe,
   eine Phenyloxy- oder Phenylmethoxygruppe, wobei der Phenylteil der vorstehend erwähnten Gruppen gegebenenfalls durch ein Fluor- oder Chloratom substituiert ist, oder
   eine Pyridyloxy- oder Pyridinylmethoxygruppe, wobei der Pyridinylteil der vorstehend erwähnten Gruppen gegebenenfalls durch eine Methyl- oder Trifluormethylgruppe substituiert ist,
   R² ein Wasserstoff-, Fluor- oder Chloratom oder eine Methylgruppe und
   R³ ein Wasserstoffatom darstellen,
R^{c} eine Cyclopentylgruppe, die in 3-Stellung durch eine Gruppe R⁴-N-R⁵ substituiert ist, wobei
   - R⁴: ein Wasserstoffatom oder eine C₁₋₃-Alkylgruppe und
   - R⁵: ein Wasserstoffatom oder eine C₁₋₃-Alkylgruppe,

   eine Aminocarbonyl-C₁₋₃-alkyl-, C₁₋₃-Alkylaminocarbonyl-C₁₋₃-alkyl-, Di-(C₁₋₃-alkyl)aminocarbonyl-C₁₋₃-alkyl-, Pyrrolidin-1-ylcarbonyl-C₁₋₃-alkyl-, Piperidin-1-yl-carbonyl-C₁₋₃-alkyl-, Piperazin-1-ylcarbonyl-C₁₋₃-alkyl-, 4-C₁₋₃-Alkyl-piperazin-1-yl-carbonyl-C₁₋₃-alkyl- oder Morpholin-4-ylcarbonyl-C₁₋₃-alkylgruppe,
   eine Hydroxy-C₂₋₄-alkyl-, C₁₋₃-Alkyloxy-C₂₋₄-alkyl-, C₁₋₄-Alkyloxy-carbonylamino-C₂₋₄-alkyl-, Amino-C₂₋₄-alkyl-, C₁₋₃-Alkylamino-C₂₋₄-alkyl-, Di-(C₁₋₃-alkyl)amino-C₂₋₄-alkyl-, C₁₋₃-Alkylcarbonylamino-C₂₋₄-alkyl-, Aminocarbonylamino-C₂₋₄-alkyl-, C₁₋₃-Alkylaminocarbonylamino-C₂₋₄-alkyl-, Di-(C₁₋₃-alkyl)amino-carbonylamino-C₂₋₄-alkyl-, Morpholin-4-ylcarbonylamino-C₂₋₄-alkyl-, C₁₋₃-Alkylsulfonyl-C₂₋₄-alkyl- oder C₁₋₃-Alkylsulfonylamino-C₂₋₄-alkylgruppe,
   eine (2-Oxo-pyrrolidin-1-yl)-C₂₋₄-alkyl-, (2-Oxopiperidin-1-yl)-C₂₋₄-alkyl-, (3-Oxo-morpholin-4-yl)-C₂₋₄-alkyl-, (2-Oxo-imidazolidin-1-yl)-C₂₋₄-alkyl-, (2-Oxo-3-methyl-imidazolidin-1-yl)-C₂₋₄-alkyl-, (2-Oxo-hexahydropyrimidin-1-yl)-C₂₋₄-alkyl- oder (2-Oxo-3-methyl-hexahydropyrimidin-1-yl)-C₂₋₄-alkylgruppe,
   eine C₁₋₃-Alkylsulfonyl-, Chlor-C₂₋₄-alkylsulfonyl-, Brom-C₂₋₄-alkylsulfonyl-, Amino-C₂₋₄-alkylsulfonyl-, C₁₋₃-Alkylamino-C₂₋₄-alkylsulfonyl-, Di-(C₁₋₃-alkyl)amino-C₂₋₄-alkylsulfonyl-, (Pyrrolidin-1-yl)-C₂₋₄-alkylsulfonyl-, (Piperidin-1-yl)-C₂₋₄-alkylsulfonyl- oder (Morpholin-4-yl)-C₂₋₄-alkylsulfonylgruppe,
   eine C₁₋₄-Alkyloxy-carbonylgruppe,
   eine Formyl-, C₁₋₃-Alkyl-carbonyl-, C₁₋₃-Alkyloxy-C₁₋₃-alkyl-carbonyl-, Tetrahydrofuranylcarbonyl-, Tetrahydropyranylcarbonyl-, Amino-C₁₋₃-alkylcarbonyl-, C₁₋₃-Alkylamino-C₁₋₃-alkyl-carbonyl-, Di-(C₁₋₃-alkyl)amino-C₁₋₃-alkylcarbonyl-, Pyrrolidin-1-yl-C₁₋₃-alkyl-carbonyl-, Piperidin-1-yl-C₁₋₃-alkyl-carbonyl-, Piperazin-1-yl-C₁₋₃-alkyl-carbonyl-, 4-C₁₋₃-Alkyl-piperazin-1-yl-C₁₋₃-alkyl-carbonyl-, Morpholin-4-yl-C₁₋₃-alkyl-carbonyl- oder eine C₁₋₃-Alkylsulfonyl-C₁₋₃-alkylcarbonylgruppe,
   eine Cyano-, Aminocarbonyl-, C₁₋₃-Alkyl-aminocarbonyl-, Di-(C₁₋₃-alkyl)aminocarbonyl-, (C₁₋₃-Alkyloxy-C₂₋₄-alkyl)aminocarbonyl-, N-(C₁₋₃-Alkyl)-N-(C₁₋₃-Alkyloxy-C₂₋₄-alkyl)aminocarbonyl-, Phenylaminocarbonyl-, Pyrrolidin-1-ylcarbonyl-, Piperidin-1-ylcarbonyl, Morpholin-4-ylcarbonyl-, C₁₋₃-Alkyl-morpholin-4-ylcarbonyl-, D i-(C₁₋₃-alkyl)morpholin-4-ylcarbonyl-, Homomorpholin-4-ylcarbonyl-, 2-Oxa-5-aza-bicyclo[2.2.1 ]hept-5-ylcarbonyl-, 3-Oxa-8-aza-bicyclo[3.2.1 ]oct-8-ylcarbonyl-, 8-Oxa-3-aza-bicyclo[3.2.1]oct-3-ylcarbonyl-, Piperazin-1-ylcarbonyl-, 4-(C₁₋₃-alkyl)-piperazin-1-ylcarbonyl-, Aminosulfonyl-, C₁₋₃-Alkyl-aminosulfonyl-, Di-(C₁₋₃-alkyl)amino-sulfonyl-, Pyrrolidin-1-yl-sulfonyl-, Piperidin-1-ylsulfonyl- oder eine Morpholin-4-ylsulfonylgruppe darstellen, oder
eine Cyclopentylgruppe, die in 3-Stellung durch eine Gruppe R⁶ substituiert ist, wobei
   R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydropyrimidin-1-yl- oder eine 2-Oxo-3-methyl-hexahydropyrimidin-1-ylgruppe darstellt,
eine Cyclohexylgruppe, die in 3-Stellung oder in 4-Stellung durch eine Gruppe R⁴-N-R⁵ substituiert ist, wobei R⁴ und R⁵ wie vorstehend erwähnt definiert sind,
eine Cyclohexylgruppe, die in 3-Stellung oder in 4-Stellung durch eine Gruppe R⁶ substituiert ist, wobei R⁶ wie vorstehend erwähnt definiert ist,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist, oder
eine Tetrahydrofuran-3-yl-, Tetrahydropyran-3-yl- oder Tetrahydropyran-4-ylgruppe,
R^{d} ein Wasserstoffatom,
eine C₁₋₃-Alkyloxygruppe,
eine Methoxygruppe, die durch ein bis drei Fluoratome substituiert ist,
eine Ethyloxygruppe, die in 2-Stellung durch einen Rest R⁶ oder R⁷ substituiert ist, wobei R⁶ wie vorstehend erwähnt definiert ist und
   R⁷ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Morpholin-4-yl-, Homomorpholin-4-yl-, 2-Oxa-5-aza-bicyclo[2.2.1]hept-5-yl-, 3-Oxa-8-aza-bicyclo-[3.2.1]oct-8-yl-, 8-Oxa-3-aza-bicyclo[3.2.1]oct-3-yl-, Piperazin-1-yl- oder eine 4-C₁₋₃-Alkyl-piperazin-1-ylgruppe, oder
   eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino- Morpholin-4-ylcarbonylamino- oder eine C₁₋₄-Alkylsulfonylamino-Gruppe darstellt,
eine Propyloxygruppe, die in 3-Stellung durch einen Rest R⁶ oder R⁷ substituiert ist, wobei R⁶ und R⁷ wie vorstehend erwähnt definiert sind, oder
eine Butyloxygruppe, die in 4-Stellung durch einen Rest R⁶ oder R⁷ substituiert ist, wobei R⁶ und R⁷ wie vorstehend erwähnt definiert sind, und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Besonders bevorzugte Verbindungen der obigen allgemeinen Formel I sind diejenigen, in denen
R^{a} ein Wasserstoffatom,
R^{b} eine 3-Ethinylphenyl-, 3-Bromphenyl-, 3,4-Difluorphenyl- oder 3-Chlor-4-fluorphenylgruppe,
eine 3-Chlor-4-benzyloxy-phenyl-, 3-Chlor-4-[(3-fluor-benzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{c} eine Cyclohexylgruppe, die in 3-Stellung oder in 4-Stellung durch eine Gruppe R⁴-N-R⁵ substituiert ist, wobei
   R⁴ ein Wasserstoffatom, eine Methyl- oder Ethylgruppe und
   R⁵ ein Wasserstoffatom, eine Methyl-, Aminocarbonylmethyl-, Methylaminocarbonylmethyl-, Dimethylaminocarbonylmethyl-, Pyrrolidin-1-ylcarbonylmethyl-, Piperidin-1-ylcarbonylmethyl-, Piperazin-1-ylcarbonylmethyl-, 4-Methylpiperazin-1-ylcarbonylmethyl-, Morpholin-4-ylcarbonylmethyl-, 2-(Morpholin-4-yl-carbonyl)ethyl- oder 3-(Morpholin-4-yl-carbonyl)propylgruppe,
   eine Ethyl-, Propyl-, 2-Hydroxyethyl-, 3-Hydroxypropyl-, 2-Methoxyethyl-, 3-Methoxypropyl-, 2-(Butyloxycarbonylamino)-ethyl-, 2-Aminoethyl-, 3-Aminopropyl-, 2-(Acetylamino)ethyl-, 3-(Acetylamino)propyl-, 2-(Ethylcarbonylamino)ethyl-, 3-(Ethylcarbonylamino)propyl-, 2-(Propylcarbonylamino)ethyl-, 3-(Propylcarbonylamino)propyl-, 2-(Ethylaminocarbonylamino)ethyl-, 3-(Ethylaminocarbonylamino)propyl-, 2-(Dimethylaminocarbonylamino)ethyl-, 3-(Dimethylaminocarbonylamino)propyl-, 2-(Morpholin-4-ylcarbonylamino)ethyl-, 3-(Morpholin-4-ylcarbonylamino)propyl-, 2-(Methylsulfonyl)ethyl-, 3-(Methylsulfonyl)propyl-, 2-(Methylsulfonylamino)ethyl-oder eine 3-(Methylsulfonylamino)propylgruppe,
   eine 2-(2-Oxo-pyrrolidin-1-yl)ethyl-, 2-(2-Oxopiperidin-1-yl)ethyl-, 2-(3-Oxo-morpholin-4-yl)ethyl-, 2-(2-Oxo-imidazolidin-1-yl)ethyl-, 2-(2-Oxo-3-methyl-imidazolidin-1-yl)ethyl-, 2-(2-Oxo-hexahydropyrimidin-1-yl)ethyl- oder eine 2-(2-Oxo-3-methyl-hexahydropyrimidin-1-yl)ethylgruppe,
   eine 3-(2-Oxo-pyrrolidin-1-yl)propyl-, 3-(2-Oxopiperidin-1-yl)propyl-, 3-(3-Oxo-morpholin-4-yl)propyl-, 3-(2-Oxo-imidazolidin-1-yl)propyl-, 3-(2-Oxo-3-methyl-imidazolidin-1-yl)propyl-, 3-(2-Oxo-hexahydropyrimidin-1-yl)propyl- oder eine 3-(2-Oxo-3-methyl-hexahydropyrimidin-1-yl)propylgruppe,
   eine Methylsulfonyl-, Ethylsulfonyl-, 3-Chlorpropylsulfonyl-, 2-(Morpholin-4-yl)-ethylsulfonyl- oder eine 3-(Morpholin-4-yl)-propylsulfonylgruppe,
   eine Propyloxycarbonyl- oder Butyloxycarbonylgruppe,
   eine Formyl-, Acetyl-, Ethylcarbonyl-, Propylcarbonyl-, Methoxyacetyl-, (2-Methoxyethyl)carbonyl-, (3-Methoxypropyl)carbonyl-, Tetrahydrofuran-2-ylcarbonyl-, Tetrahydropyran-4-ylcarbonyl-, Aminoacetyl-, Methylaminoacetyl-, Dimethylaminoacetyl-, Morpholin-4-ylacetyl-, [2-(Morpholin-4-yl)ethyl]carbonyl-, [3-(Morpholin-4-yl)propyl]carbonyl- oder eine Methylsulfonylacetylgruppe,
   eine Cyano-, Aminocarbonyl-, Methylaminocarbonyl-, Dimethylaminocarbonyl-, Ethylaminocarbonyl-, Diethylaminocarbonyl-, Propylaminocarbonyl-, (2-Methoxyethyl)aminocarbonyl-, N-Methyl-N-(2-methoxyethyl)-aminocarbonyl-, (3-Methoxypropyl)aminocarbonyl-, N-Methyl-N-(3-methoxypropyl)-aminocarbonyl-, Phenylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, 4-Methylpiperazin-1-ylcarbonyl-, Aminosulfonyl-, Methylaminosulfonyl-, Dimethylaminosulfonyl- oder eine Morpholin-4-ylsulfonylgruppe darstellen,
eine Cyclohexylgruppe, die in 3-Stellung oder in 4-Stellung durch eine Gruppe R⁶ substituiert ist, wobei
   R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydropyrimidin-1-yl- oder eine 2-Oxo-3-methyl-hexahydropyrimidin-1-ylgruppe darstellt,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist,
eine Tetrahydrofuran-3-yl-, Tetrahydropyran-3-yl- oder Tetrahydropyran-4-ylgruppe,
R^{d} ein Wasserstoffatom,
eine Methoxy-, Difluormethoxy- oder Ethyloxygruppe,
eine Ethyloxygruppe, die in 2-Stellung durch einen Rest R⁶ oder R⁷ substituiert ist, wobei R⁶ wie vorstehend erwähnt definiert ist und
   R⁷ eine Hydroxy-, Methoxy-, Ethoxy-, Amino-, Dimethylamino-, Diethylamino-, Bis-(2-methoxyethyl)-amino-, Pyrrolidin-1-yl-, Piperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-Methylpiperazin-1-yl- oder 4-Ethylpiperazin-1-ylgruppe, oder
   eine Acetylamino-, Ethylcarbonylamino-, Propylcarbonylamino-, Butylcarbonylamino-, Methoxyacetylamino-, Butyloxycarbonylamino-, Ethylaminocarbonylamino-, Dimethylaminocarbonylamino-, Pyrrolidin-1-ylcarbonylamino-, Piperidin-1-ylcarbonylamino-, Morpholin-4-ylcarbonylamino-, Methylsulfonylamino-, Ethylsulfonylamino- oder Butylsulfonylamino-Gruppe darstellt,
eine Propyloxygruppe, die in 3-Stellung durch einen Rest R⁶ oder R⁷ substituiert ist, wobei R⁶ und R⁷ wie vorstehend erwähnt definiert sind, oder
eine Butyloxygruppe, die in 4-Stellung durch einen Rest R⁶ oder R⁷ substituiert ist, wobei R⁶ und R⁷ wie vorstehend erwähnt definiert sind, und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Ganz besonders bevorzugte Verbindungen der allgemeinen Formel I sind diejenigen, in denen
R^{a} ein Wasserstoffatom,
R^{b} eine 3-Bromphenyl-, 3,4-Difluorphenyl-, 3-Chlor-4-fluor-phenyl- oder eine 3-Ethinylphenylgruppe, oder
eine 3-Chlor-4-benzyloxy-phenyl-, 3-Chlor-4-[(3-fluorbenzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{c} eine Cyclohexylgruppe, die in 3-Stellung durch eine Amino-, Acetylamino-, tert.-Butyloxycarbonylamino- oder Methylsulfonylaminogruppe substituiert ist,
eine Cyclohexylgruppe, die in 4-Stellung durch eine Amino-, Methylamino-, Ethylamino, Dimethylamino-, Aminocarbonylmethylamino-, Methylaminocarbonylmethylamino-, Dimethylaminocarbonylmethylamino-, Morpholin-4-ylcarbonylmethylamino-, [3-(Morpholin-4-ylcarbonyl)propyl]amino-, [2-(Methylsulfonyl)ethyl]amino-, [3-(Methylsulfonyl)propyl]amino- oder [2-(Methylsulfonylamino)ethyl]amino-Gruppe substituiert ist,
eine Cyclohexylgruppe, die in 4-Stellung durch eine [2-(2-Oxo-pyrrolidin-1-yl)ethyl]amino-, [2-(2-Oxopiperidin-1-yl)ethyl]amino-, [2-(2-Oxo-imidazolidin-1-yl)ethyl]amino-, [2-(2-Oxo-3-methyl-imidazolidin-1-yl)ethyl]amino-, [2-(2-Oxo-hexahydropyrimidin-1-yl)ethyl]amino- oder [2-(2-Oxo-3-methyl-hexahydropyrimidin-1-yl)ethyl]amino-Gruppe substituiert ist,
eine Cyclohexylgruppe, die in 4-Stellung durch eine [3-(2-Oxo-pyrrolidin-1-yl)propyl]amino-, [3-(2-Oxopiperidin-1-yl)propyl]amino-, [3-(2-Oxo-imidazolidin-1-yl)propyl]amino-, [3-(2-Oxo-3-methyl-imidazolidin-1-yl)propyl]amino-, [3-(2-Oxo-hexahydropyrimidin-1-yl)propyl]amino- oder [3-(2-Oxo-3-methyl-hexahydropyrimidin-1-yl)propyl]amino-Gruppe substituiert ist,
eine Cyclohexylgruppe, die in 4-Stellung durch eine Acetylamino-, N-(Acetyl)-methylamino-, Aminomethylcarbonylamino-, Methylaminomethylcarbonylamino-, Dimethylaminomethylcarbonylamino-, Morpholin-4-ylmethylcarbonylamino-, Methoxyacetylamino-, N-(Methoxyacetyl)-methylamino-, Tetrahydropyran-4-ylcarbonylamino-, N-(Tetrahydropyran-4-ylcarbonyl)-methylamino-, tert.-Butyloxycarbonylamino-, N-(tert.-Butyloxycarbonyl)-methylamino-, Aminocarbonylamino-, Methylaminocarbonylamino-, N-(Ethylaminocarbonyl)-methylamino-, Dimethylaminocarbonylamino-, N-(Dimethylaminocarbonyl)-methylamino-, N-(Piperidin-1-ylcarbonyl)-methylamino-, Morpholin-4-ylcarbonylamino-, N-(Morpholin-4-ylcarbonyl)-methylamino- oder N-(4-Methylpiperazin-1-ylcarbonyl)-methylamino-Gruppe substituiert ist,
eine Cyclohexylgruppe, die in 4-Stellung durch eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydropyrimidin-1-yl- oder eine 2-Oxo-3-methyl-hexahydropyrimidin-1-yl-Gruppe substituiert ist,
eine Cyclohexylgruppe, die in 4-Stellung durch eine Methylsulfonylamino-, N-(Methylsulfonyl)-methylamino-, Ethylsulfonylamino-, N-(Ethylsulfonyl)-methylamino-, Dimethylaminosulfonylamino-, N-(Dimethylaminosulfonyl)-methylamino-, Morpholin-4-ylsulfonylamino-, N-(Morpholin-4-ylsulfonyl)-methylamino- 3-Chlorpropylsulfonylamino-, [2-(Morpholin-4-yl)-ethyl]sulfonylamino- oder [3-(Morpholin-4-yl)-propyl]sulfonylamino-gruppe substituiert ist,
eine Pyrrolidin-3-ylgruppe,
eine Pyrrolidin-3-ylgruppe, die in 1-Stellung durch eine Methyl-, Acetyl-, Methoxyacetyl-, tert.-Butyloxycarbonyl-, Morpholin-4-ylcarbonyl- oder Methylsulfonylgruppe substituiert ist,
eine Piperidin-3-ylgruppe,
eine Piperidin-3-ylgruppe, die in 1-Stellung durch eine Methyl-, Acetyl-, Methoxyacetyl-, tert.-Butyloxycarbonyl-, Morpholin-4-ylcarbonyl- oder Methylsulfonylgruppe substituiert ist,
eine Piperidin-4-ylgruppe, die in 1-Stellung durch eine Methyl-, Ethyl-, Propyl-, Isopropyl-, 2-Hydroxyethyl-, 2-Methoxyethyl-, 3-Methoxypropyl-, 2-(Methylsulfonyl)-ethyl-, 3-(Methylsulfonyl)-propyl-, 2-(tert.-Butyloxycarbonylamino)-ethyl-, 2-Aminoethyl-, 2-(Acetylamino)-ethyl-, 2-(Ethylcarbonylamino)-ethyl-, 2-(Propylcarbonylamino)-ethyl-, 2-(Ethylaminocarbonylamino)-ethyl-, 2-(Dimethylaminocarbonylamino)-ethyl-, 2-(Morpholin-4-ylcarbonylamino)-ethyl-, 3-(Acetylamino)-propyl-, 3-(Ethylcarbonylamino)-propyl-, 3-(Propylcarbonylamino)-propyl-, 3-(Ethylaminocarbonylamino)-propyl-, 3-(Dimethylaminocarbonylamino)-propyl-, 3-(Morpholin-4-ylcarbonylamino)-propyl-, 2-(Methylsulfonylamino)-ethyl-, 3-(Methylsulfonylamino)-propyl-, (Aminocarbonyl)methyl-, (Methylaminocarbonyl)methyl-, (Dimethylaminocarbonyl)methyl-, (Pyrrolidin-1-ylcarbonyl)methyl-, (Morpholin-4-ylcarbonyl)methyl-, 2-(Morpholin-4-ylcarbonyl)-ethyl-oder 3-(Morpholin-4-ylcarbonyl)-propyl-Gruppe substituiert ist,
eine Piperidin-4-ylgruppe, die in 1-Stellung durch eine 2-(2-Oxo-pyrrolidin-1-yl)-ethyl-, 2-(2-Oxopiperidin-1-yl)-ethyl-, 2-(3-Oxomorpholin-4-yl)-ethyl-, 2-(2-Oxo-imidazolidin-1-yl)-ethyl-, 2-(2-Oxo-3-methyl-imidazolidin-1-yl)-ethyl-, 2-(2-Oxo-hexahydropyrimidin-1-yl)-ethyl- oder 2-(2-Oxo-3-methyl-hexahydropyrimidin-1-yl)-ethyl-Gruppe substituiert ist,
eine Piperidin-4-ylgruppe, die in 1-Stellung durch eine 3-(2-Oxo-pyrrolidin-1-yl)-propyl-, 3-(2-Oxopiperidin-1-yl)-propyl-, 3-(3-Oxomorpholin-4-yl)-propyl-, 3-(2-Oxo-imidazolidin-1-yl)-propyl-, 3-(2-Oxo-3-methyl-imidazolidin-1-yl)-propyl-, 3-(2-Oxo-hexahydropyrimidin-1-yl)-propyl- oder 3-(2-Oxo-3-methyl-hexahydropyrimidin-1-yl)-propyl-Gruppe substituiert ist,
eine Piperidin-4-ylgruppe, die in 1-Stellung durch eine Formyl-, Acetyl-, Methoxyacetyl-, (2-Methoxyethyl)carbonyl-, (3-Methoxypropyl)carbonyl-, Methylsulfonylacetyl-, Aminoacetyl-, Methylaminoacetyl-, (Dimethylamino)acetyl-, (Morpholin-4-yl)acetyl-, [2-(Morpholin-4-yl)-ethyl]carbonyl-, [3-(Morpholin-4-yl)-propyl]carbonyl-, Tetrahydrofuran-2-ylcarbonyl- oder Tetrahydropyran-4-ylcarbonyl-Gruppe substituiert ist,
eine Piperidin-4-ylgruppe, die in 1-Stellung durch eine Cyano-, Aminocarbonyl-, Methylaminocarbonyl-, Ethylaminocarbonyl-, (2-Methoxyethyl)aminocarbonyl-, N-Methyl-N-(2-methoxyethyl)-aminocarbonyl-, (3-Methoxypropyl)aminocarbonyl-, N-Methyl-N-(3-methoxypropyl)-aminocarbonyl-, Isopropylaminocarbonyl-, Phenylaminocarbonyl-, Dimethylaminocarbonyl-, Diethylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, 4-Methylpiperazin-1-ylcarbonyl-, Isopropyloxycarbonyl- oder tert.-Butyloxycarbonyl-Gruppe substituiert ist,
eine Piperidin-4-ylgruppe, die in 1-Stellung durch eine Methylsulfonyl-, Ethylsulfonyl-, [2-(Morpholin-4-yl)-ethyl]sulfonyl-, [3-(Morpholin-4-yl)-propyl]sulfonyl-, Aminosulfonyl-, Methylaminosulfonyl-, Dimethylaminosulfonyl- oder Morpholin-4-ylsulfonylgruppe substituiert ist, oder
eine Tetrahydrofuran-3-yl-, Tetrahydropyran-3-yl- oder Tetrahydropyran-4-ylgruppe,
R^{d} ein Wasserstoffatom,
eine Methoxy-, Difluormethoxy- oder Ethyloxygruppe,
eine 2-(Morpholin-4-yl)ethyloxy-, 3-(Morpholin-4-yl)propyloxy- oder 4-(Morpholin-4-yl)butyloxygruppe,
eine 3-(Dimethylamino)propyloxy-, 3-(Diethylamino)propyloxy-, 3-[Bis-(2-methoxyethyl)-amino]propyloxy-, 3-(Piperazin-1-yl)propyloxy-, 3-(4-Methylpiperazin-1-yl)propyloxy- oder 3-(4-Ethylpiperazin-1-yl)propyloxy-Gruppe,
eine 3-(Homomorpholin-4-yl)-propyloxy-, 3-(2-Oxa-5-aza-bicyclo[2.2.1]hept-5-yl)-propyloxy-, 3-(3-Oxa-8-aza-bicyclo[3.2.1]oct-8-yl)-propyloxy- oder 3-(8-Oxa-3-aza-bicyclo[3.2.1]oct-3-yl)-propyloxy-Gruppe,
eine 2-(2-Oxo-pyrrolidin-1-yl)-ethyloxy-, 2-(2-Oxopiperidin-1-yl)-ethyloxy-, 2-(3-Oxomorpholin-4-yl)-ethyloxy-, 2-(2-Oxo-imidazolidin-1-yl)-ethyloxy-, 2-(2-Oxo-3-methyl-imidazolidin-1-yl)-ethyloxy-, 2-(2-Oxo-hexahydropyrimidin-1-yl)-ethyloxy- oder 2-(2-Oxo-3-methyl-hexahydropyrimidin-1-yl)-ethyloxy-Gruppe,
eine 3-(2-Oxo-pyrrolidin-1-yl)-propyloxy-, 3-(2-Oxopiperidin-1-yl)-propyloxy-, 3-(3-Oxomorpholin-4-yl)-propyloxy-, 3-(2-Oxo-imidazolidin-1 -yl)-propyloxy-, 3-(2-Oxo-3-methyl-imidazolidin-1-yl)-propyloxy-, 3-(2-Oxo-hexahydropyrimidin-1-yl)-propyloxy- oder 3-(2-Oxo-3-methyl-hexahydropyrimidin-1-yl)-propyloxy-Gruppe,
eine 2-(Methoxy)-ethyloxy-, 2-(tert.-Butyloxycarbonylamino)-ethyloxy-, 2-(Amino)-ethyloxy-, 2-(Acetylamino)-ethyloxy-, 2-(Ethylcarbonylamino)-ethyloxy-, 2-(Propylcarbonylamino)-ethyloxy-, 2-(Isobutylcarbonylamino)-ethyloxy-, 2-(Methoxyacetylamino)-ethyloxy-, 2-(Ethylaminocarbonylamino)-ethyloxy-, 2-(Dimethylaminocarbonylamino)-ethyloxy-, 2-(Pyrrolidin-1-ylcarbonylamino)-ethyloxy-, 2-(Piperidin-1-ylcarbonylamino)-ethyloxy-, 2-(Morpholin-4-ylcarbonylamino)-ethyloxy-, 2-(Methylsulfonylamino)-ethyloxygruppe, 2-(Ethylsulfonylamino)-ethyloxy- oder 2-(Butylsulfonylamino)-ethyloxy-Gruppe, oder
eine 3-(tert.-Butyloxycarbonylamino)-propyloxy-, 3-(Amino)-propyloxy-, 3-(Acetylamino)-propyloxy- oder 3-(Methylsulfonylamino)-propyloxy-Gruppe,
   und
X ein Stickstoffatom bedeuten, deren Tautomere, deren Stereoisomere, deren Gemische und deren Salze.

Insbesondere bevorzugte Verbindungen der allgemeinen Formel I sind diejenigen, in denen
R^{a} ein Wasserstoffatom,
R^{b} vorzugsweise eine 3-Chlor-4-fluor-phenylgruppe oder auch eine 3-Ethinylphenylgruppe,
R^{c} eine Cyclohexylgruppe, die in 3-Stellung durch eine Amino-, Acetylamino-, tert.-Butyloxycarbonylamino- oder Methylsulfonylaminogruppe substituiert ist,
eine Cyclohexylgruppe, die in 4-Stellung durch eine Amino-, Methylamino-, Dimethylamino-, Acetylamino-, N-(Acetyl)-methylamino-, Methoxyacetylamino-, N-(Methoxyacetyl)-methylamino-, Tetrahydropyran-4-ylcarbonylamino-, N-(Tetrahydropyran-4-ylcarbonyl)-methylamino-, tert.-Butyloxycarbonylamino-, N-(tert.-Butyloxycarbonyl)-methylamino-, N-(Ethylaminocarbonyl)-methylamino-, Dimethylaminocarbonylamino-, N-(Dimethylaminocarbonyl)-methylamino-, N-(Piperidin-1-ylcarbonyl)-methylamino-, Morpholin-4-ylcarbonylamino-, N-(Morpholin-4-ylcarbonyl)-methylamino-, N-(4-Methylpiperazin-1-ylcarbonyl)-methylamino-, Methylsulfonylamino-, N-(Methylsulfonyl)-methylamino-, Ethylsulfonylamino-, N-(Ethylsulfonyl)-methylamino-, Dimethylaminosulfonylamino-, N-(Dimethylaminosulfonyl)-methylamino-, Morpholin-4-ylsulfonylamino-, N-(Morpholin-4-ylsulfonyl)-methylamino-, 3-Chlorpropylsulfonylamino-, oder [3-(Morpholin-4-yl)-propyl]sulfonylaminogruppe substituiert ist,
eine Pyrrolidin-3-ylgruppe,
eine Pyrrolidin-3-ylgruppe, die in 1-Stellung durch eine tert.-Butyloxycarbonyl- oder Methylsulfonylgruppe substituiert ist,
eine Piperidin-3-ylgruppe,
eine Piperidin-3-ylgruppe, die in 1-Stellung durch eine tert.-Butyloxycarbonyl- oder Methylsulfonylgruppe substituiert ist,
eine Piperidin-4-ylgruppe,
eine Piperidin-4-ylgruppe, die in 1-Stellung durch eine Methyl-, (Aminocarbonyl)methyl-, (Dimethylaminocarbonyl)methyl-, (Morpholin-4-ylcarbonyl)methyl-, 2-(tert.-Butyloxycarbonylamino)ethyl-, 2-Aminoethyl-, 2-(Acetylamino)ethyl-, 2-(Methylsulfonylamino)ethyl-, Cyano-, Acetyl-, Methoxyacetyl-, (Dimethylamino)acetyl-, (Morpholin-4-yl)acetyl-, Tetrahydropyran-4-ylcarbonyl-, Ethylaminocarbonyl-, Isopropylaminocarbonyl-, Phenylaminocarbonyl-, Dimethylaminocarbonyl-, Diethylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 4-Methylpiperazin-1-ylcarbonyl-, Isopropyloxycarbonyl-, tert.-Butyloxycarbonyl-, Methylsulfonyl-, Dimethylaminosulfonyl- oder Morpholin-4-ylsulfonylgruppe substituiert ist, oder
eine Tetrahydrofuran-3-yl-, Tetrahydropyran-3-yl- oder Tetrahydropyran-4-ylgruppe,
R^{d} ein Wasserstoffatom,
eine Methoxy- oder Ethyloxygruppe,
eine 2-(Morpholin-4-yl)ethyloxy-, 3-(Morpholin-4-yl)propyloxy- oder 4-(Morpholin-4-yl)butyloxygruppe,
eine 2-(3-Methyl-2-oxo-hexahydropyrimidin-1-yl)-ethyloxygruppe,
eine 2-(Methoxy)-ethyloxy-, 2-(tert.-Butyloxycarbonylamino)-ethyloxy-, 2-Aminoethyloxy-, 2-(Acetylamino)-ethyloxy- oder 2-(Methylsulfonylamino)-ethyloxygruppe oder eine 3-(tert.-Butyloxycarbonylamino)-propyloxy-, 3-Amino-propyloxy-, 3-(Acetylamino)-propyloxy- oder 3-(Methylsulfonylamino)-propyloxygruppe,
   und
X ein Stickstoffatom bedeuten,
deren Tautomere, deren Stereoisomere, deren Gemische und deren Salze.
Unter den vorstehend beschriebenen bicyclischen Heterocyclen der allgemeinen Formel I sowie den jeweils als bevorzugt, besonders bevorzugt, ganz besonders bevorzugt und insbesondere bevorzugt gekennzeichneten Untergruppen sind jeweils diejenigen Verbindungen besonders hervorzuheben, in denen
   - (a): R^{c} eine in 4-Stellung substituierte Cyclohexylgruppe darstellt,
   - (b): R^{c} eine gegebenenfalls in 1-Stellung substituierte Pyrrolidin-3-ylgruppe darstellt,
   - (c): R^{c} eine gegebenenfalls in 1-Stellung substituierte Piperidin-3-ylgruppe darstellt,
   - (d): R^{c} eine gegebenenfalls in 1-Stellung substituierte Piperidin-4-ylgruppe darstellt,
   - (e): R^{c} eine Tetrahydrofuran-3-ylgruppe darstellt,
   - (f): R^{c} eine Tetrahydropyran-3-ylgruppe darstellt, oder
   - (g): R^{c} eine Tetrahydropyran-4-ylgruppe darstellt,
   wobei R^{a}, R^{b}, R^{d} und X jeweils wie vorstehend erwähnt definiert sind.

Beispielsweise seien folgende besonders bevorzugte Verbindungen der allgemeinen Formel I erwähnt:
- (1): 4-[(3-Chlor-4-fluor-phenyl)amino]-6-((*S*)-tetrahydrofuran-3-yloxy)-7-methoxy- chinazolin,
- (2): 4-[(3-Chlor-4-fluor-phenyl)amino]-6-(tetrahydropyran-4-yloxy)-7-methoxy- chinazolin,
- (3): 4-[(3-Chlor-4-fluor-phenyl)amino]-6-((*R*)-tetrahydrofuran-3-yloxy)-7-methoxy- chinazolin,
- (4): 4-[(3-Chlor-4-fluor-phenyl)amino]-6-(trans-4-amino-cyclohexan-1-yloxy)-7- methoxy-chinazolin,
- (5): 4-[(3-Chlor-4-fluor-phenyl)amino]-6-(trans-4-methansulfonylamino-cyclohexan-1- yloxy)-7-methoxy-chinazolin,
- (6): 4-[(3-Chlor-4-fluor-phenyl)amino]-6-(piperidin-4-yloxy)-7-methoxy-chinazolin,
- (7): 4-[(3-Chlor-4-fluor-phenyl)amino]-6-(1-methansulfonyl-piperidin-4-yloxy)-7- methoxy-chinazolin,
- (8): 4-[(3-Chlor-4-fluor-phenyl)amino]-6-(cis-4-{[3-(morpholin-4-yl)-propyl]sulfonyl- aminol}-cyclohexan-1-yloxy)-7-methoxy-chinazolin,
- (9): 4-[(3-Chlor-4-fluor-phenyl)amino]-6-(tetrahydropyran-3-yloxy)-7-methoxy- chinazolin,
- (10): 4-[(3-Chlor-4-fluor-phenyl)amino]-6-(trans-4-{[3-(morpholin-4-yl)-propyl]sulfonyl- amino}-cyclohexan-1-yloxy)-7-methoxy-chinazolin,
- (11): 4-[(3-Chlor-4-fluor-phenyl)amino]-6-(1-methyl-piperidin-4-yloxy)-7-methoxy- chinazolin,
- (12): 4-[(3-Chlor-4-fluor-phenyl)amino]-6-{1-[(morpholin-4-yl)carbonyl]-piperidin-4-yl- oxy}-7-methoxy-chinazolin,
- (13): 4-[(3-Chlor-4-fluor-phenyl)amino]-6-{1-[(methoxymethyl)carbonyl]-piperidin-4-yl- oxy}-7-methoxy-chinazolin,
- (14): 4-[(3-Chlor-4-fluor-phenyl)amino]-6-(1-cyano-piperidin-4-yloxy)-7-methoxy- chinazolin,
- (15): 4-[(3-Chlor-4-fluor-phenyl)amino]-6-{1-[(morpholin-4-yl)sulfonyl]-piperidin-4-yl- oxy}-7-methoxy-chinazolin,
- (16): 4-[(3-Chlor-4-fluor-phenyl)amino]-6-[1-(2-acetylamino-ethyl)-piperidin-4-yloxy]-7- methoxy-chinazolin,
- (17): 4-[(3-Chlor-4-fluor-phenyl)amino]-6-{trans-4-[(dimethylamino)sulfonylamino]- cyclohexan-1-yloxy}-7-methoxy-chinazolin,
- (18): 4-[(3-Chlor-4-fluor-phenyl)amino]-6-{trans-4-[(morpholin-4-yl)carbonylamino]- cyclohexan-1-yloxy}-7-methoxy-chinazolin,
- (19): 4-[(3-Chlor-4-fluor-phenyl)amino]-6-{trans-4-[(morpholin-4-yl)sulfonylamino]- cyclohexan-1-yloxy}-7-methoxy-chinazolin,
- (20): 4-[(3-Chlor-4-fluor-phenyl)amino]-6-(tetrahydropyran-4-yloxy)-7-(2-acetylamino- ethoxy)-chinazolin,
- (21): 4-[(3-Chlor-4-fluor-phenyl)amino]-6-(tetrahydropyran-4-yloxy)-7-(2- methansulfonylamino-ethoxy)-chinazolin und
- (22): 4-[(3-Chlor-4-fluor-phenyl)amino]-6-(tetrahydropyran-4-yloxy)-7-(2-methoxy- ethoxy)-chinazolin,
sowie deren Salze.

Die Verbindungen der allgemeinen Formel I lassen sich beispielsweise nach folgenden Verfahren herstellen:
a) Umsetzung einer Verbindung der allgemeinen Formel 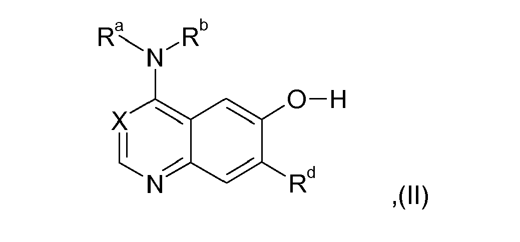 in der
   R^{a}, R^{b}, R^{d} und X wie eingangs erwähnt definiert sind, mit einer Verbindung der allgemeinen Formel

      Z¹-R^{c}, (III)

      in der
   R^{c} wie eingangs erwähnt definiert ist und Z¹ eine Austrittsgruppe wie ein Halogenatom, z.B. ein Chlor- oder Bromatom, eine Sulfonyloxygruppe wie eine Methansulfonyloxy- oder p-Toluolsulfonyloxygruppe oder eine Hydroxygruppe darstellt.
   Mit einer Verbindung der allgemeinen Formel III, in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether bei Temperaturen zwischen -50 und 150°C, vorzugsweise jedoch bei Temperaturen zwischen -20 und 80°C, durchgeführt.
b) Zur Herstellung von Verbindungen der allgemeinen Formel I, in der R^{d} eine der eingangs erwähnten, gegebenenfalls substituierten Alkyloxygruppen darstellt:
   Umsetzung einer Verbindung der allgemeinen Formel 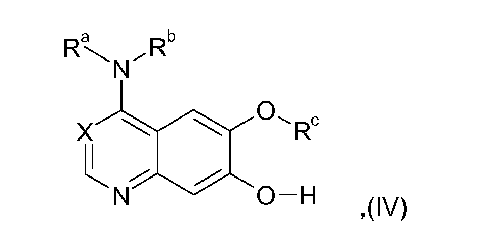 in der R^{a}, R^{b}, R^{c} und X wie eingangs erwähnt definiert, sind mit einer mit einer Verbindung der allgemeinen Formel

      Z²-R^{d'}, (V)

      in der R^{d'} eine C₁-₄-Alkylgruppe, eine durch 1 bis 3 Fluoratome substituierte Methylgruppe, eine durch 1 bis 5 Fluoratome substituierte Ethylgruppe, eine durch einen Rest R⁶ oder R⁷ substituiert C₂₋₄-Alkylgruppe, wobei R⁶ und R⁷ wie eingangs erwähnt definiert sind, eine C₁-₄-Alkylgruppe, die durch eine in 1-Stellung durch den Rest R⁸ substituierte Pyrrolidinyl-, Piperidinyl- oder Homopiperidinylgruppe substituiert ist, oder eine C₁-₄-Alkylgruppe, die durch eine in 4-Stellung durch den Rest R⁸ substituierte Morpholinylgruppe substituiert ist, wobei R⁸ jeweils wie eingangs erwähnt definiert ist, darstellt und
   Z² eine Austrittsgruppe wie ein Halogenatom, eine Alkylsulfonyloxy-, Arylsulfonyloxy- oder eine Hydroxygruppe darstellt.
   Handelt es sich bei der Austrittsgruppe um ein Halogenatom wie ein Chlor-, Brom- oder lodatom oder um eine Alkylsulfonyloxy- oder Arylsulfonyloxygruppe wie die die Methansulfonyloxy oder p-Toluolsulfonyloxygruppe, wird die Reaktion vorzugsweise in Gegenwart einer organischen oder anorganischen Base wie Kaliumcarbonat, Natriumhydrid oder N-Ethyl-diisopropylamin durchgeführt. Handelt es sich bei der Austrittsgruppe um eine Hydroxygruppe, so wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester durchgeführt.
c) Zur Herstellung von Verbindungen der allgemeinen Formel I, in der R^{d} eine der eingangs erwähnten Alkyloxygruppen darstellt, die durch eine gegebenenfalls substituierte Amino-, Alkylamino- oder Dialkylaminogruppe oder durch eine gegebenenfalls substituierte, über ein Iminostickstoffatom gebundene heterocyclischen Gruppe substituiert ist:Umsetzung einer Verbindung der allgemeinen Formel 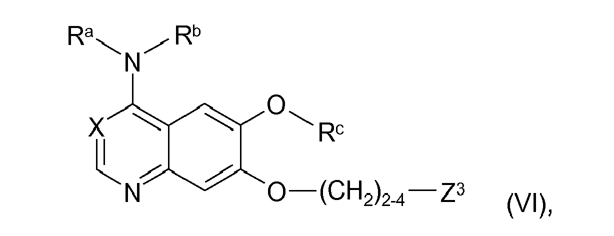 in der R^{a}, R^{b}, R^{c} und X wie eingangs erwähnt definiert sind und Z³ eine Austrittsgruppe wie ein Halogenatom, z.B. ein Chlor- oder Bromatom oder eine Sulfonyloxygruppe wie eine Methansulfonyloxy- oder p-Toluolsulfonyloxygruppe darstellt, mit
   Ammoniak, einem entsprechenden, gegebenenfalls substituierten Alkylamin, Dialkylamin oder einer Iminoverbindung oder deren geeigneten Salzen oder Derivaten, wie beispielsweise Morpholin.
d) Zur Herstellung von Verbindungen der allgemeinen Formel I, in der R^{d} eine Hydroxygruppe darstellt:
   Abspaltung eines Schutzrestes von einer Verbindung der allgemeinen Formel 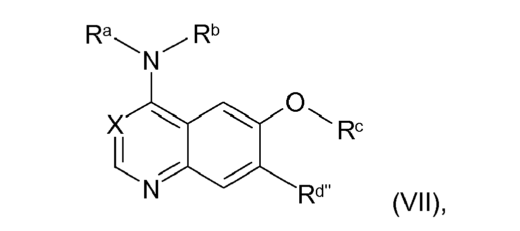 in der R^{a}, R^{b}, R^{c} und X wie eingangs erwähnt definiert sind und R^{d"} eine in eine Hydroxygruppe überführbare Gruppe darstellt, beispielsweise eine gegebenenfalls substituierte Benzyloxygruppe, eine Trimethylsilyloxy-, Acetyloxy-, Benzoyloxy-, Methoxy-, Ethoxy-, tert-Butoxy- oder Trityloxygruppe.
   Die Abspaltung des Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.
   Die Abspaltung eines Benzyl- oder Methoxybenzylrestes erfolgt beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.
   Die Abspaltung eines tert.-Butyl- oder Benzylrestes erfolgt beispielsweise durch Behandlung mit einer Säure wie Trifluoressigsäure, Salzsäure oder Bromwasserstoffsäure oder durch Behandlung mit Jodtrimethylsilan gegebenenfalls unter Verwendung eines Lösungsmittels wie Methylenchlorid, Dioxan, Methanol oder Diethylether.
e) Zur Herstellung von Verbindungen der allgemeinen Formel I, in der R^{c} eine -NH-Gruppe enthält:
   Abspaltung eines Schutzrestes von einer Verbindung der allgemeinen Formel 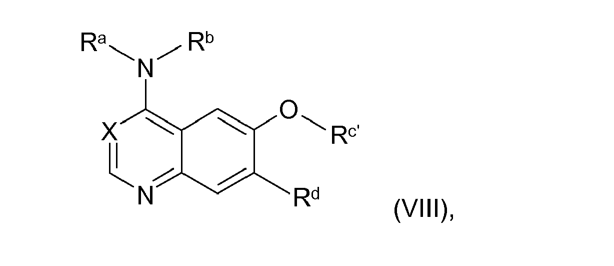 in der R^{a}, R^{b}, R^{d} und X wie eingangs erwähnt definiert sind und R^{c'} mit der Maßgabe die eingangs für R^{c} erwähnten Bedeutungen besitzt, daß R^{c} ein geschütztes Stickstoffatom enthält.
   Übliche Schutzreste für eine Amino-, Alkylamino- oder Iminogruppe sind beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe , wobei für die Aminogruppe zusätzlich die Phthalylgruppe in Betracht kommt.
   Die Abspaltung des Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.
   Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.
   Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.
   Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.
   Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.
f) Zur Herstellung von Verbindungen der allgemeinen Formel I, in der R^{c} eine durch eine gegebenenfalls substituierte Amino-, Alkylamino- oder Dialkyaminogruppe oder durch eine über ein Stickstoffatom gebundenene, gegebenenfalls substituierte heterocyclische Gruppe substituierte Alkylgruppe enthält:
   Umsetzung einer Verbindung der allgemeinen Formel 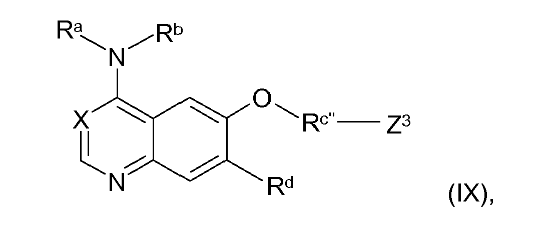 in der R^{a}, R^{b}, R^{d} und X wie eingangs erwähnt definiert sind, Z³ eine Austrittsgruppe darstellt, beispielsweise ein Halogenatom wie ein Chlor- oder Bromatom, oder eine Sulfonyloxygruppe wie eine Methansulfonyloxy- oder p-Toluolsulfonyloxygruppe, und R^{c"} mit der Maßgabe, daß ein an ein aliphatisches Kohlenstoffatom gebundenes Wasserstoffatom durch die Gruppe Z³ ersetzt ist, die für R^{c} eingangs erwähnten Bedeutungen besitzt,
   mit Ammoniak, einem entsprechenden, gegenenfalls substituierten Alkylamin, Dialkylamin oder einer Iminoverbindung oder deren geeigneten Salzen oder Derivaten, wie beispielsweise Morpholin.

Erhält man erfindungsgemäß eine Verbindung der allgemeinen Formel I, die eine Amino-, Alkylamino- oder Iminogruppe enthält, so kann diese mittels Acylierung, Cyanierung oder Sulfonylierung in eine entsprechende Acyl-, Cyano- oder Sulfonylverbindung der allgemeinen Formel I übergeführt werden, wobei als Acylierungsmittel beispielsweise Isocyanate, Carbamoylchloride, Carbonsäurehalogenide, Carbonsäureanhydride und Carbonsäuren mit Aktivierungsmitteln wie N,N'-Carbonyldiimidazol, N,N'-Dicyclohexylcarbodiimid oder O-(Benzotriazol-1-yl)-N,N,N'N'-tetramethyluronium-tetrafluoroborat, als Sulfonylierungsmittel Sulfonylhalogenide und als Cyanierungsmittel Chlor- oder Bromcyan in Frage kommen, und/oder
eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden und/oder
eine Verbindung der allgemeinen Formel I, die eine Chlor-C₁₋₄-alkylsulfonyl- oder Brom-C₁-₄-alkylsulfonylgruppe enthält, so kann diese durch Umsetzung mit einem Amin in eine entsprechende Amino-C₁₋₄-alkylsulfonylverbindung übergeführt werden und/oder
eine Verbindung der allgemeinen Formel I, die eine tert.-Butyloxycarbonylamino-, N-Alkyl-N-(tert.-butyloxycarbonyl)amino- oder eine N-tert.-Butyloxycarbonyliminogruppe enthält, so kann diese mittels Behandlung mit einer Säure wie Salzsäure oder Trifluoressigsäure in eine entsprechende Amino-, Alkylamino- oder Iminoverbindung der allgemeinen Formel I übergeführt werden.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis IX sind teilweise literaturbekannt oder können nach an sich literaturbekannten Verfahren (siehe Beispiele I bis XXII) oder den vorstehend beschriebenen Verfahren, gegebenenfalls unter zusätzlicher Einführung von Schutzresten (z.B. Verbindungen der Formel IV bzw. VII und VIII), erhalten werden.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:
Die Hemmung der humanen EGF-Rezeptorkinase wurde mit Hilfe der cytoplasmatischen Tyrosinkinase-Domäne (Methionin 664 bis Alanin 1186 basierend auf der in Nature 309 (1984), 418 publizierten Sequenz) bestimmt. Hierzu wurde das Protein in Sf9 Insektenzellen als GST-Fusionsprotein unter Verwendung des Baculovirus-Expressionssystems exprimiert.

Die Messung der Enzymaktivität wurde in Gegenwart oder Abwesenheit der Testverbindungen in seriellen Verdünnungen durchgeführt. Das Polymer pEY (4:1) von SIGMA wurde als Substrat verwendet. Biotinyliertes pEY (bio-pEY) wurde als Tracer-Substrat zugesetzt. Jede 100 µl Reaktionslösung enthielt 10 µl des Inhibitors in 50% DMSO, 20 µl der Substrat-Lösung (200 mM HEPES pH 7.4, 50 mM Magnesiumacetat, 2.5 mg/ml poly(EY), 5 µg/ml bio-pEY) und 20 µl Enzympräparation. Die Enzymreaktion wurde durch Zugabe von 50µl einer 100 µM ATP Lösung in 10 mM Magnesiumchlorid gestartet. Die Verdünnung der Enzympräparation wurde so eingestellt, daß der Phosphat-Einbau in das bio-pEY hinsichtlich Zeit und Enzymmenge linear war. Die Enzympräparation wurde in 20 mM HEPES pH 7.4, 1 mM EDTA, 130 mM Kochsalz, 0.05% Triton X-1 00, 1 mM DTT und 10% Glycerin verdünnt.

Die Enzymassays wurden bei Raumtemperatur über einen Zeitraum von 30 Minuten ausgeführt und durch Zugabe von 50 µl einer Stopplösung (250 mM EDTA in 20 mM HEPES pH 7.4) beendet. 100 µl wurden auf eine Streptavidin-beschichtete Mikrotiterplatte gebracht und 60 Minuten bei Raumtemperatur inkubiert. Danach wurde die Platte mit 200 µl einer Waschlösung (50 mM Tris, 0.05% Tween 20) gewaschen. Nach Zugabe von 100 µl eines HRPO-gelabelten anti-PY Antikörpers (PY20H Anti-PTyr:HRP von Transduction Laboratories, 250 ng/ml) wurde 60 Minuten inkubiert. Danach wurde die Mikrotiterplatte dreimal mit je 200 µl Waschlösung gewaschen. Die Proben wurden dann mit 100 µl einer TMB-Peroxidase-Lösung (A:B =1:1, Kirkegaard Perry Laboratories) versetzt. Nach 10 Minuten wurde die Reaktion gestoppt. Die Extinktion wurde bei OD₄₅₀ₙₘ mit einem ELISA-Leser gemessen. Alle Datenpunkte wurden als Triplikate bestimmt.

Die Daten wurden mittels einer iterativen Rechnung unter Verwendung eines Analysenprogrammes für sigmoidale Kurven (Graph Pad Prism Version 3.0) mit variabler Hill-Steigung angepaßt. Alle freigegebenen Iterationsdaten wiesen einen Korrelationskoeffizienten von über 0.9 auf und die Ober- und Unterwerte der Kurven zeigten eine Spreizung von mindestens einem Faktor von 5. Aus den Kurven wurde die Wirkstoffkonzentration abgeleitet, die die Aktivität der EGF-Rezeptorkinase zu 50% hemmt (IC₅₀).

Folgende Ergebnisse wurden erhalten:

| Verbindung (Beispiel Nr.) | Hemmung der EGF-Rezeptorkinase IC₅₀ [nM] |
|---|---|
| 1 | 0.13 |
| 1(1) | 0.12 |
| 1(2) | 2 |
| 1(3) | 1.1 |
| 1(4) | 0.6 |
| 1(5) | 0.6 |
| 1(6) | 0.69 |
| 1(7) | 1.6 |
| 2 | 4.5 |
| 2(1) | 0.16 |
| 2(2) | 0.22 |
| 3 | 0.9 |
| 3(1) | 0.14 |
| 3(2) | 0.22 |
| 3(7) | 0.7 |
| 3(8) | 0.6 |
| 3(9) | 0.2 |
| 3(11) | 0.1 |
| 3(15) | 1 |
| 3(16) | 1 |
| 3(17) | 0.3 |
| 3(18) | 0.4 |
| 3(20) | 1 |
| 3(21) | 0.4 |
| 4 | 0.41 |
| 4(1) | 0.16 |
| 7(5) | 1 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome.

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch (z.B. Ambroxol, N-acetylcystein), broncholytisch (z.B. Tiotropium oder Ipratropium oder Fenoterol, Salmeterol, Salbutamol) und/oder entzündungshemmend (z.B. Theophylline oder Glucocorticoide) wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

### 4-[(3-Chlor-4-fluor-phenyl)amino]-6-(tetrahydropyran-4-yloxy)-7-benzyloxy-chinazolin-hydrochlorid

Ein Gemisch aus 10.84 g 4-Chlor-6-(tetrahydropyran-4-yloxy)-7-benzyloxy-chinazolin und 4.50 g 3-Chlor-4-fluoranilin in 300 ml Isopropanol wird vier Stunden unter Rückfluß erhitzt und anschließend über Nacht bei Raumtemperatur stehengelassen. Der entstandene Niederschlag wird abgesaugt, mit Isopropanol nachgewaschen und mit 150 ml Methanol verrührt. Die Suspension wird noch eine halbe Stunde bei Raumtemperatur gerührt und anschließend abgesaugt. Der Filterkuchen wird mehrfach mit Methanol nachgewaschen und getrocknet.
Ausbeute: 9.07 g (60 % der Theorie)
R_{f}-Wert: 0.27 (Kieselgel, Cyclohexan/Essigester = 1:1)
Massenspektrum (ESI⁻): m/z = 478, 480 [M-H]⁻

Analog Beispiel I werden folgende Verbindungen erhalten:
(1) 4-[(3-Chlor-4-fluor-phenyl)amino]-6-((*S*)-tetrahydrofuran-3-yloxy)-7-benzyloxy-chinazolin-hydrochlorid
   R_{f}-Wert: 0.34 (Kieselgel, Cyclohexan/Essigester = 1:1)
   Massenspektrum (ESI⁺): m/z = 466, 468 [M+H]⁺
(2) 4-[(3-Chlor-4-fluor-phenyl)amino]-6-(1-trifluoracetyl-piperidin-4-yloxy)-chinazolin-hydrochlorid
   R_{f}-Wert: 0.17 (Reversed Phase DC-Fertigplatte (E. Merck), Acetonitril/Wasser/ Trifluoressigsäure = 50:50:1)
   Massenspektrum (ESI⁺): m/z = 469, 471 [M+H]⁺
(3) 4-[(3-Chlor-4-fluor-phenyl)amino]-6-(1-trifluoracetyl-piperidin-4-yloxy)-7-acetoxy-chinazolin-hydrochlorid
   R_{f}-Wert: 0.70 (Kieselgel, Methylenchlorid/Methanol/konz. wässriges Ammoniak = 90:10:1)
   Massenspektrum (ESI⁺): m/z = 527, 529 [M+H]⁺
(4) 4-[(3-Ethinyl-phenyl)amino]-6-acetoxy-7-methoxy-chinazolin
   R_{f}-Wert: 0.59 (Kieselgel, Essigester)
   Massenspektrum (ESI⁺): m/z = 334 [M+H]⁺
(5) 4-({3-Chlor-4-[(3-fluor-benzyl)oxy]-phenyl}amino)-6-(acetyloxy)-7-methoxy-chinazolin
   R_{f}-Wert: 0.20 (Reversed Phase DC-Fertigplatte (E. Merck), Acetonitril/Wasser/ Trifluoressigsäure = 50:50:1)
   Massenspektrum (ESI⁻): m/z = 466, 468 [M-H]⁻
(6) 4-{[3-Methyl-4-(pyridin-3-yloxy)-phenyl]amino}-6-(acetyloxy)-7-methoxy-chinazolin Das eingesetzte 3-Methyl-4-(pyridin-3-yloxy)-anilin (R_{f}-Wert: 0.30 (Kieselgel, Cyclohexan/Essigester = 1:1)) wurde durch Umsetzung des Natriumsalzes von 3-Hydroxypyridin mit 3-Methyl-4-fluor-nitrobenzol in Dimethylformamid und anschließender Hydrierung des so erhaltenen 3-Methyl-4-(pyridin-3-yloxy)-nitrobenzols (R_{f}-Wert: 0.58 (Kieselgel, Cyclohexan/Essigester = 1:1)) in Gegenwart von Palladium auf Aktivkohle hergestellt.
   R_{f}-Wert: 0.50 (Reversed Phase DC-Fertigplatte (E. Merck), Acetonitril/Wasser/ Trifluoressigsäure = 50:50:1)
   Massenspektrum (ESI⁻): m/z = 415 [M-H]⁻

### Beispiel II

### 4-Chlor-6-(tetrahydropyran-4-yloxy)-7-benzyloxy-chinazolin

Hergestellt durch Umsetzung von 6-(Tetrahydropyran-4-yloxy)-7-benzyloxy-3H-chinazolin-4-on mit Thionylchlorid in Gegenwart von N,N-Dimethylformamid in Acetonitril unter Rückfluß.
R_{f}-Wert: 0.90 (Kieselgel, Essigester/Methanol = 9:1)

Analog Beispiel II werden folgende Verbindungen erhalten:
(1) 4-Chlor-6-((S)-tetrahydrofuran-3-yloxy)-7-benzyloxy-chinazolin R_{f}-Wert: 0.85 (Kieselgel, Essigester/Methanol = 9:1)
(2) 4-Chlor-6-(1-trifluoracetyl-piperidin-4-yloxy)-chinazolin R_{f}-Wert: 0.92 (Kieselgel, Essigester)
(3) 4-Chlor-6-(1-trifluoracetyl-piperidin-4-yloxy)-7-acetoxy-chinazolin

### Beispiel III

### 6-(Tetrahydropyran-4-yloxy)-7-benzyloxy-3H-chinazolin-4-on

Ein Gemisch aus 15.08 g 2-Amino-4-benzyloxy-5-(tetrahydropyran-4-yloxy)-benzoesäure und 14.40 g Formamidinacetat in 250 ml absolutem Ethanol wird über Nacht unter Rückfluß erhitzt. Das abgekühlte Reaktionsgemisch wird mit 250 ml Wasser versetzt. Der ausgefallene Niederschlag wird abgesaugt und bei 70°C im Trockenschrank getrocknet.
Ausbeute: 10.00 g (65 % der Theorie)
R_{f}-Wert: 0.40 (Reversed Phase DC-Fertigplatte (E. Merck), Acetonitril/Wasser/ Trifluoressigsäure = 50:50:1)
Massenspektrum (ESI⁺): m/z = 353 [M+H]⁺

Analog Beispiel III werden folgende Verbindungen erhalten:
(1) 6-((S)-Tetrahydrofuran-3-yloxy)-7-benzyloxy-3H-chinazolin-4-on
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 339 [M+H]⁺
(2) 6-[1-(tert.-Butyloxycarbonyl)-piperidin-4-yloxy]-3H-chinazolin-4-on
   R_{f}-Wert: 0.48 (Kieselgel, Essigester/Methanol = 9:1)
   Massenspektrum (ESI⁺): m/z = 346 [M+H]⁺
(3) 6-[1-(tert.-Butyloxycarbonyl)-piperidin-4-yloxy]-7-hydroxy-3H-chinazolin-4-on
   R_{f}-Wert: 0.35 (Kieselgel, Methylenchlorid/Methanol = 9:1)
   Massenspektrum (ESI⁺): m/z = 362 [M+H]⁺

### Beispiel IV

### 2-Amino-4-benzyloxy-5-(tetrahydropyran-4-yloxy)-benzoesäure

16.40 g 2-Nitro-4-benzyloxy-5-(tetrahydropyran-4-yloxy)-benzoesäure werden in Gegenwart von 1.64 g Raney-Nickel in 800 ml Methanol bei 55°C hydriert, bis die berechnete Menge Wasserstoff aufgenommen ist. Der Katalysator wird abfiltriert und das Filtrat eingeengt, wobei das gewünschte Produkt auskristallisiert.
Ausbeute: 15.08 g (100 % der Theorie)
R_{f}-Wert: 0.60 (Reversed Phase DC-Fertigplatte (E. Merck), Acetonitril/Wasser/ Trifluoressigsäure = 50:50:1)

Analog Beispiel IV werden folgende Verbindungen erhalten:
(1) 2-Amino-4-benzyloxy-5-((S)-tetrahydrofuran-3-yloxy)-benzoesäure-benzylester
   R_{f}-Wert: 0.70 (Kieselgel, Cyclohexan/Essigester = 1:1)
   Massenspektrum (ESI⁺): m/z = 420 [M+H]⁺
(2) 2-Amino-5-[1-(tert.-butyloxycarbonyl)-piperidin-4-yloxy]-benzoesäure
   R_{f}-Wert: 0.43 (Kieselgel, Methylenchlorid/Methanol = 9:1)
   Massenspektrum (ESI⁺): m/z = 337 [M+H]⁺
(3) 2-Amino-4-hydroxy-5-[1-(tert.-butyloxycarbonyl)-piperidin-4-yloxy]-benzoesäure
   R_{f}-Wert: 0.23 (Kieselgel, Methylenchlorid/Methanol/Essigsäure = 90:10:1)

### Beispiel V

### 2-Nitro-4-benzyloxy-5-(tetrahydropyran-4-yloxy)-benzoesäure

Hergestellt durch Verseifung von 2-Nitro-4-benzyloxy-5-(tetrahydropyran-4-yloxy)-benzoesäure-benzylester mit 1 N Natronlauge in Methanol bei Raumtemperatur.
R_{f}-Wert: 0.20 (Reversed Phase DC-Fertigplatte (E. Merck), Acetonitril/Wasser/ Trifluoressigsäure = 50:50:1)
Massenspektrum (ESI⁺): m/z = 374 [M+H]⁺

### Beispiel VI

### 2-Nitro-4-benzyloxy-5-(tetrahydro-pyran-4-yloxy)-benzoesäure-benzylester

Zu 38 ml Tetrahydrofuran-4-ol in 228 ml N,N-Dimethylformamid werden unter Eisbad-Kühlung 42.60 g Kalium-tert.-butanolat gegeben. Das Gemisch wir eine Stunde bei Raumtemperatur gerührt, dann werden 22.90 g 6-Nitro-benzo[1,3]dioxol-5-carbonsäure zugegeben. Nach 1.5 Stunden ist die Umsetzung laut Dünnschichtchromatogramm vollständig und es werden 28.94 ml Benzylbromid unter Eisbad-Kühlung zugetropft. Das Reaktionsgemisch wird über Nacht bei Raumtemperatur gerührt, mit 100 ml 10%iger Zitronensäure versetzt und einen weiteren Tag bei Raumtemperatur gerührt. Anschließend wird das Reaktionsgemisch im Vakuum bei 60 °C eingeengt und auf 800 ml Eiswasser gegeben. Die wässrige Phase wird mit Essigester extrahiert und die vereinten Extrakte werden mit Wasser und gesättigter Natriumchloridlösung gewaschen, über Magnesiumsulfat getrocknet und eingeengt.

Der Rückstand wird mit Diethylether verrührt, wobei als Nebenprodukt 2-Nitro-4-benzyloxy-5-(tetrahydropyran-4-yloxy)-benzoesäure auskristallisiert. Diese wird abfiltriert und das Filtrat eingeengt. Als Hauptprodukt bleibt 2-Nitro-4-benzyloxy-5-(tetrahydro-pyran-4-yloxy)-benzoesäure-benzylester zurück, welcher ohne weitere Reinigung zur Carbonsäure verseift wird (siehe Beispiel V).

Analog Beispiel VI werden folgende Verbindungen erhalten:
(1) 2-Nitro-4-benzyloxy-5-((S)-tetrahydrofuran-3-yloxy)-benzoesäure-benzylester
   R_{f}-Wert: 0.75 (Kieselgel, Cyclohexan/Essigester = 1:1)
   Massenspektrum (ESI⁺): m/z = 450 [M+H]⁺
(2) 2-Nitro-4-hydroxy-5-[1-(tert.-butyloxycarbonyl)-piperidin-4-yloxy]-benzoesäure Auf die Umsetzung mit Benzylbromid wird verzichtet.
   R_{f}-Wert: 0.40 (Kieselgel, Methylenchlorid/Methanol/Essigsäure = 90:10:1)
   Massenspektrum (ESI⁻): m/z = 381 [M-H]⁻

### Beispiel VII

### 4-[(3-Chlor-4-fluor-phenyl)amino]-6-{1-[2-(tert.-butyloxycarbonylamino)-ethyl]-piperidin-4-yloxy}-7-methoxy-chinazolin

Ein Gemisch aus 410 mg 4-[(3-Chlor-4-fluor-phenyl)amino]-6-(piperidin-4-yloxy)-7-methoxy-chinazolin-dihydrochlorid, 240 mg N-(tert.-Butyloxycarbonyl)-2-brom-ethylamin und 360 mg Kaliumcarbonat in 5 ml N,N-Dimethylformamid wird über Nacht bei Raumtemperatur gerührt. Dann werden nochmals 80 mg N-(tert.-Butyloxycarbonyl)-2-brom-ethylamin zugesetzt und das Reaktionsgemisch wird weitere vier Stunden bei Raumtemperatur gerührt. Zur Aufarbeitung wird es mit Wasser verdünnt und mit Essigester extrahiert. Die vereinigten organischen Phasen werden mit gesättigter Natriumchloridlösung gewaschen, über Magnesiumsulfat getrocknet und eingeengt. Der Rückstand wird über eine Kieselgelsäule mit Essigester/Methanol (95:5 auf 90:1) als Laufmittel chromatographiert.
Ausbeute: 370 mg (79 % der Theorie)
R_{f}-Wert: 0.33 (Reversed Phase DC-Fertigplatte (E. Merck), Acetonitril/Wasser/ Trifluoressigsäure = 50:50:1)
Massenspektrum (ESI⁻): m/z = 544, 546 [M-H]⁻

Analog Beispiel VII wird folgende Verbindung erhalten:
(1) 4-[(3-Chlor-4-fluor-phenyl)amino]-6-{1-[2-(tert.-butyloxycarbonylamino)-ethyl]-piperidin-4-yloxy}-chinazolin
   R_{f}-Wert: 0.38 (Reversed Phase DC-Fertigplatte (E. Merck), Acetonitril/Wasser/ Trifluoressigsäure = 50:50:1)
   Massenspektrum (ESI⁺): m/z = 516, 518 [M+H]⁺

### Beispiel VIII

### 4-[(3-Chlor-4-fluor-phenyl)amino]-6-(piperidin-4-yloxy)-7-methoxy-chinazolin-dihydrochlorid

Hergestellt durch Behandlung von 4-[(3-Chlor-4-fluor-phenyl)amino]-6-[1-(tert.-butyloxycarbonyl)-piperidin-4-yloxy]-7-methoxy-chinazolin mit konzentrierter Salzsäure in Dioxan bei Raumtemperatur.
R_{f}-Wert: 0.53 (Reversed Phase DC-Fertigplatte (E. Merck), Acetonitril/Wasser/ Trifluoressigsäure = 50:50:1)
Massenspektrum (ESI⁺): m/z = 403, 405 [M+H]⁺

Analog Beispiel VIII werden folgende Verbindungen erhalten:
(1) 6-(Piperidin-4-yloxy)-3*H*-chinazolin-4-on x 2 Trifluoressigsäure
   Durchführung mit Trifluoressigsäure in Methylenchlorid.
   Massenspektrum (ESI⁺): m/z = 246 [M+H]⁺
(2) 6-(Piperidin-4-yloxy)-7-hydroxy-3*H*-chinazolin-4-on
   Durchführung mit Trifluoressigsäure in Methylenchlorid.
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 262 [M+H]⁺

### Beispiel IX

### 4-[(3-Chlor-4-fluor-phenyl)amino]-6-[1-(tert.-butyloxycarbonyl)-piperidin-4-yloxy]-7-methoxy-chinazolin

Zu einem Gemisch aus 10.00 g 4-[(3-Chlor-4-fluor-phenyl)amino]-6-hydroxy-7-methoxy-chinazolin und 9.40 g 1-(tert.-Butyloxycarbonyl)-4-hydroxy-piperidin und 12.40 g Triphenylphosphin in 400 ml Methylenchlorid wird bei Raumtemperatur eine Lösung aus 7.80 ml Azodicarbonsäurediethylester in 100 ml Methylenchlorid getropft. Die Suspension wird drei Tage bei Raumtemperatur gerührt und anschließend abgesaugt. Das Filtrat wird eingeengt und über eine Kieselgelsäule mit Methylenchlorid/Methanol (98:2 auf 95:5) als Laufmittel chromatographiert. Das erhaltene Rohprodukt wird mit Diisopropylether versetzt, über Nacht darin gerührt, abgesaugt und getrocknet.
Ausbeute: 5.34 g (34 % der Theorie)
R_{f}-Wert: 0.46 (Kieselgel, Essigester/Methanol = 9:1)
Massenspektrum (ESI⁺): m/z = 503, 505 [M+H]⁺

### Beispiel X

### 4-[(3-Chlor-4-fluor-phenyl)amino]-6-(tetrahydropyran-4-yloxy)-7-(4-brom-butyloxy)-chinazolin

Ein Gemisch aus 500 mg 4-[(3-Chlor-4-fluor-phenyl)amino]-6-(tetrahydropyran-4-yloxy)-7-hydroxy-chinazolin, 165 µl 1-Brom-4-chlor-propan und 360 mg Kaliumcarbonat in 5 ml N,N-Dimethylformamid wird über Nacht bei 80°C gerührt. Zur Aufarbeitung wird das Reaktionsgemisch mit Wasser verdünnt und mit Essigester extrahiert. Die vereinigten organischen Phasen werden mit gesättigter Natriumchloridlösung gewaschen, über Magnesiumsulfat getrocknet und eingeengt. Das Rohpodukt wird ohne weitere Reinigung weiter umgesetzt.
Ausbeute: 650 mg (97 % der Theorie)

Analog Beispiel X werden folgende Verbindungen erhalten:
(1) 4-[(3-Chlor-4-fluor-phenyl)amino]-6-((*S*)-tetrahydrofuran-3-yloxy)-7-(4-brom-butyloxy)-chinazolin
   R_{f}-Wert: 0.84 (Kieselgel, Essigester/Methanol = 9:1)
(2) 4-[(3-Chlor-4-fluor-phenyl)amino]-6-(1-trifluoracetyl-piperidin-4-yloxy)-7-ethoxy-chinazolin
   Massenspektrum (ESI⁺): m/z = 513, 515 [M+H]⁺
(3) 4-[(3-Chlor-4-fluor-phenyl)amino]-6-(1-trifluoracetyl-piperidin-4-yloxy)-7-(2-methoxy-ethoxy)-chinazolin
   R_{f}-Wert: 0.38 (Kieselgel, Methylenchlorid/Methanol = 9:1)
   Massenspektrum (ESI⁺): m/z = 543, 545 [M+H]⁺

### Beispiel XI

### 1-(2-Hydroxy-ethyl)-3-methyl-tetrahydropyrimidin-2-on

Hergestellt durch hydrogenolytische Spaltung von 1-(2-Benzyloxy-ethyl)-3-methyl-tetrahydropyrimidin-2-on in Gegenwart von Palladium auf Aktivkohle in Methanol bei Raumtemperatur.
R_{f}-Wert: 0.23 (Kieselgel, Essigester/Methanol/konz. wässriges Ammoniak = 90:10:1)
Massenspektrum (ESI⁺): m/z = 159 [M+H]⁺

### Beispiel XII

### 1-(2-Benzyloxy-ethyl)-3-methyl-tetrahydropyrimidin-2-on

Hergestellt durch Umsetzung von 1-(2-Benzyloxy-ethyl)-tetrahydropyrimidin-2-on mit Methyliodid in Gegenwart von Kalium-tert.-butanolat in N,N-Dimethylformamid bei Raumtemperatur.
R_{f}-Wert: 0.62 (Kieselgel, Essigester/Methanol/konz. wässriges Ammoniak = 90:10:1)
Massenspektrum (ESI⁺): m/z = 249 [M+H]⁺

### Beispiel XIII

### 1-(2-Benzyloxy-ethyl)-tetrahydropyrimidin-2-on

Hergestellt durch Behandeln von 1-(2-Benzyloxy-ethyl)-3-(3-chlor-propyl)-harnstoff mit Kalium-tert.-butanolat in N,N-Dimethylformamid bei Raumtemperatur.
R_{f}-Wert: 0.42 (Kieselgel, Essigester/Methanol/konz. wässriges Ammoniak = 90:10:1)
Massenspektrum (ESI⁺): m/z = 235 [M+H]⁺

### Beispiel XIV

### 1-(2-Benzyloxy-ethyl)-tetrahydropyrimidin-2-on

Hergestellt durch Umsetzung von 2-Benzyloxy-ethylamin mit 3-Chlor-propyl-isocyanat in Tetradydrofuran.
R_{f}-Wert: 0.73 (Kieselgel, Essigester/Methanol/konz. wässriges Ammoniak = 90:10:1)
Massenspektrum (ESI⁺): m/z = 271, 273 [M+H]⁺

### Beispiel XV

### 3-(tert.-Butyloxycarbonylamino)-cyclohexanol

Hergestellt durch Umsetzung von 3-Amino-cyclohexanol mit Pyrokohlensäure-di-tert.-butylester in Gegenwart von Triethylamin in einem Gemisch aus Dioxan/Wasser (2:1) bei 50°C.
R_{f}-Wert: 0.34 (Kieselgel, Methylenchlorid/Methanol/konz. wässriges Ammoniak = 90:10:1)
Massenspektrum (ESI⁻): m/z = 214 [M-H]⁻

Analog Beispiel XV werden folgende Verbindungen erhalten:
(1) cis-4-[N-(tert.-Butyloxycarbonyl)-N-methyl-amino]-cyclohexanol Die Umsetzung erfolgt in Methanol.
   R_{f}-Wert: 0.70 (Kieselgel, Essigester)
   Massenspektrum (ESI⁺): m/z = 230 [M+H]⁺

### Beispiel XVI

### 6-(1-Trifluoracetyl-piperidin-4-yloxy)-3H-chinazolin-4-on

Hergestellt durch Umsetzung von 6-(Piperidin-4-yloxy)-3*H*-chinazolin-4-o n x 2 Trifluoressigsäure mit Trifluoressigsäureanhydrid in Gegenwart von Triethylamin in Tetrahydrofuran.
R_{f}-Wert: 0.48 (Kieselgel, Essigester/Methanol = 9:1)
Massenspektrum (ESI⁺): m/z = 342 [M+H]⁺

Analog Beispiel XVI werden folgende Verbindungen erhalten:
(1) 6-(1-Trifluoracetyl-piperidin-4-yloxy)-7-hydroxy-3*H*-chinazolin-4-on Durchführung mit Trifluoressigsäuremethylester in Gegenwart von Hünigbase in Methanol.
   R_{f}-Wert: 0.80 (Kieselgel, Methylenchlorid/Methanol = 4:1)
   Massenspektrum (ESI⁺): m/z = 358 [M+H]⁺

### Beispiel XVII

### 2-Nitro-5-[1-(tert.-butyloxycarbonyl)-piperidin-4-yloxy]-benzoesäure

Zu 25.14 g 1-(tert.-Butyloxycarbonyl)-piperidin-4-ol in 120 ml N,N-Dimethylformamid werden unter Eisbad-Kühlung portionsweise 21.00 g Kalium-tert.-butanolat gegeben, wobei die Temperatur unter 10 °C gehalten wird. Das Gemisch wird noch 30 Minuten unter Eisbad-Kühlung nachgerührt, dann werden 11.60 g 5-Fluor-2-nitro-benzoesäure zugegeben. Nach weiteren drei Stunden wird das Reaktionsgemisch auf Wasser gegossen, mit konz. Salzsäure auf pH 1 eingestellt und mit Essigester extrahiert. Die vereinigten organischen Phasen werden mit verdünnter Zitronensäure-Lösung gewaschen, über Magnesiumsulfat getrocknet und eingeengt. Der Rückstand wird mit Diethylether verrieben, abgesaugt und getrocknet. Aus dem Filtrat kristallisiert nach längerem Stehenlassen weiteres Produkt aus, welches ebenfalls abgesaugt und getrocknet wird.
Ausbeute: 9.58 g (42 % der Theorie)
R_{f}-Wert: 0.43 (Kieselgel, Methylenchlorid/Methanol/Essigsäure = 90:10:1)
Massenspektrum (ESI⁺): m/z = 367[M+H]⁺

### Beispiel XVIII

### 4-[(3-Chlor-4-fluor-phenyl)amino]-6-(1-bromacetyl-piperidin-4-yloxy)-chinazolin und 4-[(3-Chlor-4-fluor-phenyl)amino]-6-(1-chloracetyl-piperidin-4-yloxy)-chinazolin

Hergestellt durch Umsetzung von 4-[(3-Chlor-4-fluor-phenyl)amino]-6-(piperidin-4-yloxy)-chinazolin mit Bromessigsäurechlorid in Gegenwart von Hünigbase in Tetrahydrofuran bei Raumtemperatur. Man erhält ein Gemisch aus Brom- und Chlor-Verbindung.
R_{f}-Wert: 0.43 (Kieselgel, Methylenchlorid/Methanol = 9:1)
Massenspektrum (ESI⁺): m/z = 493, 495, 497 [M1+H]⁺und 449, 451, 453 [M2+H]⁺

Analog Beispiel XVIII werden folgende Verbindungen erhalten:
(1) 4-[(3-Chlor-4-fluor-phenyl)amino]-6-(1-chloracetyl-piperidin-4-yloxy)-7-methoxy-chinazolin
   Die Umsetzung erfolgt mit Chloracetylchlorid.
   R_{f}-Wert: 0.59 (Kieselgel, Essigester/Methanol/konz. wässriges Ammoniak = 90:10:1)
   Massenspektrum (ESI⁻): m/z = 477, 479, 481 [M-H]⁻

### Beispiel XIX

### 1-Methyl-3-[([1,4]oxazepan-4-yl)carbonyl]-3H-imidazol-1-ium-iodid

Hergestellt durch Umsetzung von 3-[([1,4]Oxazepan-4-yl)carbonyl]-3*H*-imidazol mit Methyliodid in Acetonitril bei Raumtemperatur. Das Rohprodukt wird ohne weitere Reinigung weiter umgesetzt.

Analog Beispiel XIX werden folgende Verbindungen erhalten:
(1) 1-Methyl-3-[(cis-2,6-dimethyl-morpholin-4-yl)carbonyl]-3*H*-imidazol-1-ium-iodid
   R_{f}-Wert: 0.12 (Kieselgel, Essigester/Methanol/konz. wässriges Ammoniak = 90:10:1)
(2) 1-Methyl-3-[(2-methyl-morpholin-4-yl)carbonyl]-3*H*-imidazol-1-ium-iodid
   R_{f}-Wert: 0.02 (Kieselgel, Essigester/Methanol/konz. wässriges Ammoniak = 90:10:1)
(3) 1-Methyl-3-[(S,S)-(2-oxa-5-aza-bicyclo[2.2.1]hept-5-yl)carbonyl]-3H-imidazol-1-ium-iodid
   R_{f}-Wert: 0.01 (Kieselgel, Essigester/Methanol/konz. wässriges Ammoniak = 90:10:1)
(4) 1-Methyl-3-[(N-methyl-N-2-methoxyethyl-amino)carbonyl]-3H-imidazol-1-ium-iodid
   R_{f}-Wert: 0.03 (Kieselgel, Essigester/Methanol/konz. wässriges Ammoniak = 90:10:1)
(5) 1-Methyl-3-[(N-methyl-N-3-methoxypropyl-amino)carbonyl]-3H-imidazol-1-ium-iodid
   R_{f}-Wert: 0.12 (Kieselgel, Methylenchlorid/Methanol/konz. wässriges Ammoniak = 90:10:1)

### Beispiel XX

### 3-[([1,4]Oxazepan-4-yl)carbonyl]-3H-imidazol

Hergestellt durch Umsetzung von [1,4]Oxazepan mit N,N'-Carbonyldiimidazol in Gegenwart von Triethylamin in Tetrahydrofuran bei Raumtemperatur.
R_{f}-Wert: 0.30 (Kieselgel, Essigester/Methanol/konz. wässriges Ammoniak = 90:10:1)
Massenspektrum (ESI⁺): m/z = 196 [M+H]⁺

Analog Beispiel XX werden folgende Verbindungen erhalten:
(1) 3-[(cis-2,6-Dimethyl-morpholin-4-yl)carbonyl]-3*H*-imidazol
   R_{f}-Wert: 0.46 (Kieselgel, Essigester/Methanol/konz. wässriges Ammoniak = 90:10:1)
(2) 3-[(2-Methyl-morpholin-4-yl)carbonyl]-3*H*-imidazol
   R_{f}-Wert: 0.43 (Kieselgel, Essigester/Methanol/konz. wässriges Ammoniak = 90:10:1)
(3) 3-[(S,S)-(2-Oxa-5-aza-bicyclo[2.2.1]hept-5-yl)carbonyl]-3H-imidazol
   R_{f}-Wert: 0.59 (Reversed Phase DC-Fertigplatte (E. Merck), Acetonitril/Wasser/ Trifluoressigsäure = 50:50:1)
(4) 3-[(N-Methyl-N-2-methoxyethyl-amino)carbonyl]-3H-imidazol
   R_{f}-Wert: 0.32 (Kieselgel, Essigester/Methanol/konz. wässriges Ammoniak = 90:10:1)
(5) 3-[(N-Methyl-N-3-methoxypropyl-amino)carbonyl]-3*H*-imidazol
   R_{f}-Wert: 0.36 (Kieselgel, Essigester/Methanol/konz. wässriges Ammoniak = 90:10:1)

### Beispiel XXI

### 4-[(3-Chlor-4-fluor-phenyl)amino]-6-(1-trifluoracetyl-piperidin-4-yloxy)-7-hydroxy-chinazolin

Hergestellt durch Behandlung von 4-[(3-Chlor-4-fluor-phenyl)amino]-6-(1-trifluoracetyl-piperidin-4-yloxy)-7-acetoxy-chinazolin-hydrochlorid mit gesättigter Natriumhydrogencarbonat-Lösung in Methanol bei Raumtemperatur. Neben dem gewünschten Produkt wird als Nebenprodukt auch etwas 4-[(3-Chlor-4-fluor-phenyl)amino]-6-(piperidin-4-yloxy)-7-hydroxy-chinazolin isoliert.
R_{f}-Wert: 0.20 (Kieselgel, Methylenchlorid/Methanol = 20:1)
Massenspektrum (ESI⁻): m/z = 483, 485 [M-H]⁻

Analog Beispiel XXI werden folgende Verbindungen erhalten:
(1) 4-[(3-Ethinyl-phenyl)amino]-6-hydroxy-7-methoxy-chinazolin
   Durchführung mit 40 %iger Natronlauge in Ethanol.
   R_{f}-Wert: 0.32 (Kieselgel, Essigester)
   Massenspektrum (ESI⁺): m/z = 292 [M+H]⁺
(2) 4-({3-Chlor-4-[(3-fluor-benzyl)oxy]-phenyl}amino)-6-hydroxy-7-methoxy-chinazolin
   R_{f}-Wert: 0.70 (Kieselgel, Methylenchlorid/Methanol = 9:1)
   Massenspektrum (ESI⁻): m/z = 424, 426 [M-H]⁻
(3) 4-{[3-Methyl-4-(pyridin-3-yloxy)-phenyl]amino}-6-hydroxy-7-methoxy-chinazolin
   R_{f}-Wert: 0.23 (Kieselgel, Methylenchlorid/Methanol = 9:1)
   Massenspektrum (ESI⁻): m/z = 373 [M-H]⁻

### Beispiel XXII

### 6-(1-Trifluoracetyl-piperidin-4-yloxy)-7-acetoxy-3H-chinazolin-4-on

Hergestellt durch Umsetzung von 6-(1-Trifluoracetyl-piperidin-4-yloxy)-7-hydroxy-3*H-*chinazolin-4-on mit Acetanhydrid in Pyridin bei 80°C.
R_{f}-Wert: 0.60 (Kieselgel, Methylenchlorid/Methanol = 9:1)
Massenspektrum (ESI⁺): m/z = 400 [M+H]⁺

### Beispiel XXIII

### 4-[(3-Chlor-4-fluor-phenyl)amino]-6-{1-[(4-nitrophenyloxy)-carbonyl]-piperidin-4-yloxy}-7-methoxy-chinazolin

Hergestellt durch Umsetzung von 4-[(3-Chlor-4-fluor-phenyl)amino]-6-(piperidin-4-yloxy)-7-methoxy-chinazolin (Beispiel 2(2)) mit Chlorameisensäure-(4-nitrophenyl)-ester.
R_{f}-Wert: 0.48 (Kieselgel, Methylenchlorid/Methanol/konz. wässriges Ammoniak = 90:10:1)

### Herstellung der Endverbindungen:

### Beispiel 1

### 4-[(3-Chlor-4-fluor-phenyl)amino]-6-((S)-tetrahydrofuran-3-yloxy)-7-methoxy-chinazolin

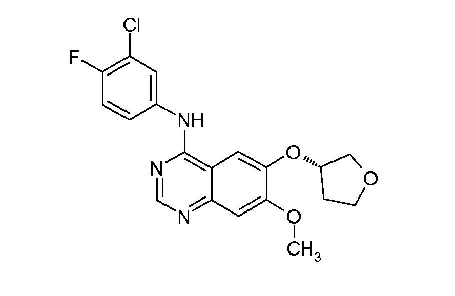

300 mg 4-[(3-Chlor-4-fluor-phenyl)amino]-6-hydroxy-7-methoxy-chinazolin in 6 ml Acetonitril werden mit 114 µl (R)-3-Hydroxy-tetrahydrofuran und 370 mg Triphenylphosphin versetzt. Anschließend werden 234 µl Azodicarbonsäurediethylester zugegeben und das Reaktionsgemisch wird über Nacht bei Raumtemperatur gerührt. Zur Aufarbeitung wird das Reaktionsgemisch filtriert und das Filtrat im Vakuum eingeengt. Das Rohprodukt wird chromatographisch über eine Kieselgelsäule mit Essigester/- Methanol (95:5) als Laufmittel gereinigt.
Ausbeute: 53 mg (15 % der Theorie)
Schmelzpunkt: 178°C
Massenspektrum (ESI⁺): m/z = 390, 392 [M+H]⁺

Analog Beispiel 1 werden folgende Verbindungen erhalten:
(1) 4-[(3-Chlor-4-fluor-phenyl)amino]-6-(tetrahydropyran-4-yloxy)-7-methoxy-chinazolin 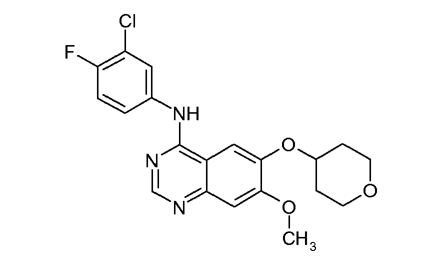
   R_{f}-Wert: 0.54 (Kieselgel, Essigester/Methanol = 9:1)
   Massenspektrum (ESI⁺): m/z = 404, 406 [M+H]⁺
(2) 4-[(3-Chlor-4-fluor-phenyl)amino]-6-[cis-4-(tert.-butyloxycarbonylamino)-cyclohexan-1-yloxy]-7-methoxy-chinazolin 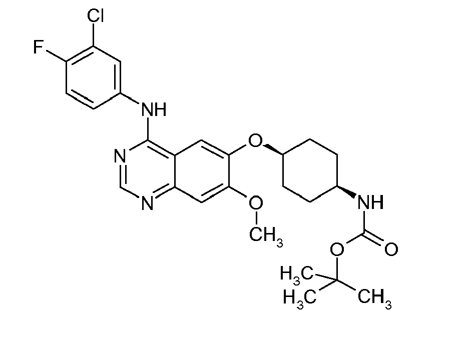
   R_{f}-Wert: 0.70 (Kieselgel, Essigester/Methanol = 9:1)
   Massenspektrum (ESI⁺): m/z = 517, 519 [M+H]⁺
(3) 4-[(3-Chlor-4-fluor-phenyl)amino]-6-((*R*)-tetrahydrofuran-3-yloxy)-7-methoxy-chinazolin 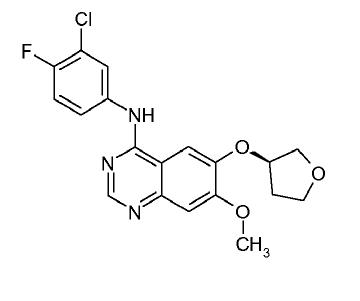
   R_{f}-Wert: 0.64 (Kieselgel, Essigester/Methanol = 9:1)
   Massenspektrum (ESI⁺): m/z = 390, 392 [M+H]⁺
(4) 4-[(3-Chlor-4-fluor-phenyl)amino]-6-[trans-4-(tert.-butyloxycarbonylamino)-cyclohexan-1-yloxy]-7-methoxy-chinazolin 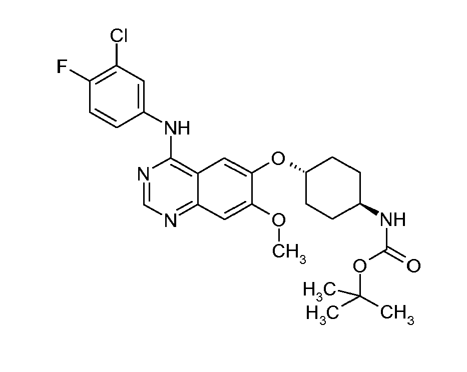
   R_{f}-Wert: 0.65 (Kieselgel, Essigester/Methanol = 9:1)
   Massenspektrum (ESI⁺): m/z = 517, 519 [M+H]⁺
(5) 4-[(3-Chlor-4-fluor-phenyl)amino]-6-[1-(tert.-butyloxycarbonyl)-piperidin-4-yloxy]-7-methoxy-chinazolin 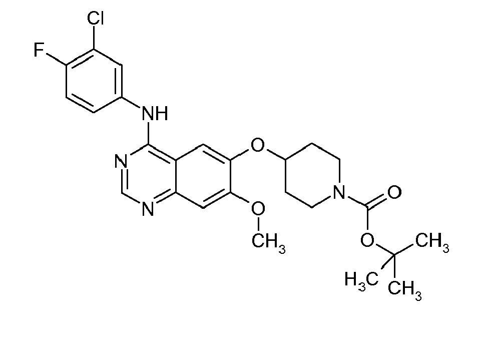
   Schmelzpunkt: 184°C
   Massenspektrum (ESI⁺): m/z = 503, 505 [M+H]⁺
(6) 4-[(3-Chlor-4-fluor-phenyl)amino]-6-(tetrahydropyran-3-yloxy)-7-methoxy-chinazolin 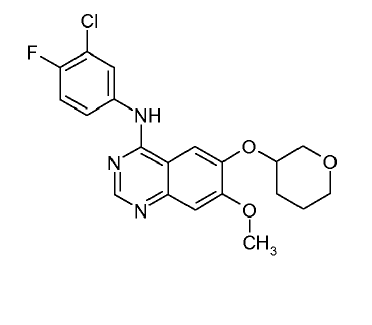
   R_{f}-Wert: 0.52 (Kieselgel, Methylenchlorid/Methanol = 9:1)
   Massenspektrum (ESI⁺): m/z = 404, 406 [M+H]⁺
(7) 4-[(3-Chlor-4-fluor-phenyl)amino]-6-(1-methyl-piperidin-4-yloxy)-7-methoxy-chinazolin 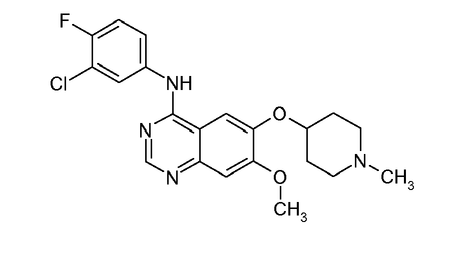
   Schmelzpunkt: 218°C
   Massenspektrum (ESI⁺): m/z = 417, 419 [M+H]⁺
(8) 4-[(3-Chlor-4-fluor-phenyl)amino]-6-[(S)-1-(tert.-butyloxycarbonyl)-pyrrolidin-3-yloxy]-7-methoxy-chinazolin
   Durchführung mit Azodicarbonsäurediisopropylester in Methylenchlorid.
   R_{f}-Wert: 0.51 (Kieselgel, Methylenchlorid/Methanol = 9:1)
   Massenspektrum (ESI⁺): m/z = 489, 491 [M+H]⁺
(9) 4-[(3-Chlor-4-fluor-phenyl)amino]-6-[1-(tert.-butyloxycarbonyl)-piperidin-3-yloxy]-7-methoxy-chinazolin
   Durchführung mit Azodicarbonsäurediisopropylester in Methylenchlorid.
   R_{f}-Wert: 0.56 (Kieselgel, Methylenchlorid/Methanol = 9:1)
   Massenspektrum (ESI⁻): m/z = 501, 503 [M-H]⁻
(10) 4-[(3-Chlor-4-fluor-phenyl)amino]-6-((S)-tetrahydrofuran-3-yloxy)-7-[2-(3-methyl-2-oxo-hexahydropyrimidin-1-yl)-ethoxy]-chinazolin
   Durchführung mit Azodicarbonsäurediisopropylester in Methylenchlorid.
   Schmelzpunkt: 235°C
   Massenspektrum (ESI⁺): m/z = 516, 518 [M+H]⁺
(11) 4-[(3-Chlor-4-fluor-phenyl)amino]-6-[3-(tert.-butyloxycarbonylamino)-cyclohexan-1-yloxy]-7-methoxy-chinazolin
   Durchführung mit Azodicarbonsäurediisopropylester in Methylenchlorid.
   R_{f}-Wert: 0.68 (Kieselgel, Essigester/Methanol = 9:1)
   Massenspektrum (ESI⁻): m/z = 515, 517 [M-H]⁻
(12) 4-[(3-Chlor-4-fluor-phenyl)amino]-6-{cis-4-[N-(tert.-butyloxycarbonyl)-N-methylamino]-cyclohexan-1-yloxy}-7-methoxy-chinazolin
   Durchführung mit Azodicarbonsäurediisopropylester in Methylenchlorid.
   R_{f}-Wert: 0.37 (Kieselgel, Methylenchlorid/Methanol = 9:1)
   Massenspektrum (ESI⁺): m/z = 531, 533 [M+H]⁺
(13) 4-[(3-Chlor-4-fluor-phenyl)amino]-6-{trans-4-[N-(tert.-butyloxycarbonyl)-N-methylamino]-cyclohexan-1-yloxy}-7-methoxy-chinazolin
   Durchführung mit Azodicarbonsäurediisopropylester in Methylenchlorid.
   Schmelzpunkt: 231°C
   Massenspektrum (ESI⁺): m/z = 531, 533 [M+H]⁺

### Beispiel 2

### 4-[(3-Chlor-4-fluor-phenyl)amino]-6-(cis-4-amino-cyclohexan-1-yloxy)-7-methoxy-chinazolin x Trifluoressigsäure

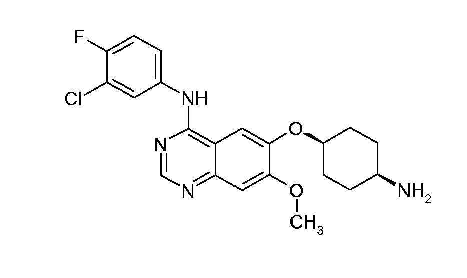

Hergestellt durch Behandlung von 4-[(3-Chlor-4-fluor-phenyl)amino]-6-[cis-4-(tert.-butyloxycarbonylamino)-cyclohexan-1-yloxy]-7-methoxy-chinazolin mit Trifluoressigsäure in Methylenchlorid bei Raumtemperatur.
Schmelzpunkt: 221°C
Massenspektrum (ESI⁺): m/z = 417, 419 [M+H]⁺

Analog Beispiel 2 werden folgende Verbindungen erhalten:
(1) 4-[(3-Chlor-4-fluor-phenyl)amino]-6-(trans-4-amino-cyclohexan-1-yloxy)-7-methoxy-chinazolin 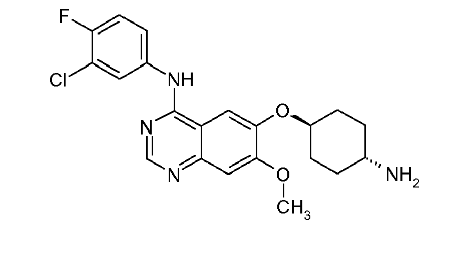
   Massenspektrum (ESI⁺): m/z = 417, 419 [M+H]⁺
(2) 4-[(3-Chlor-4-fluor-phenyl)amino]-6-(piperidin-4-yloxy)-7-methoxy-chinazolin x Trifluoressigsäure 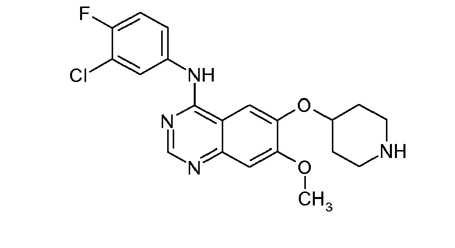
   Schmelzpunkt: 232°C
   Massenspektrum (ESI⁺): m/z = 403, 405 [M+H]⁺

### Beispiel 3

### 4-[(3-Chlor-4-fluor-phenyl)amino]-6-(cis-4-methansulfonylamino-cyclohexan-1-yloxy)-7-methoxy-chinazolin

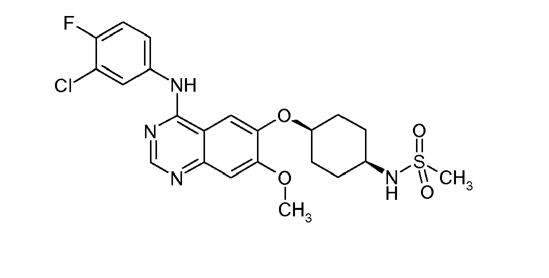

Hergestellt durch Umsetzung von 4-[(3-Chlor-4-fluor-phenyl)amino]-6-(cis-4-amino-cyclohexan-1-yloxy)-7-methoxy-chinazolin x Trifluoressigsäure mit Methansulfonsäurechlorid in Gegenwart von Hünigbase in Tetrahydrofuran bei Raumtemperatur.
R_{f}-Wert: 0.77 (Kieselgel, Essigester/Methanol/konz. wässriges Ammoniak = 40:10:1)
Massenspektrum (ESI⁺): m/z = 495, 497 [M+H]⁺

Analog Beispiel 3 werden folgende Verbindungen erhalten:
(1) 4-[(3-Chlor-4-fluor-phenyl)amino]-6-(trans-4-methansulfonylamino-cyclohexan-1-yloxy)-7-methoxy-chinazolin 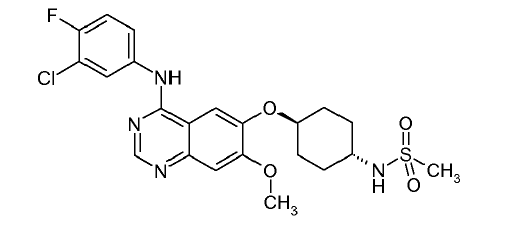
   R_{f}-Wert: 0.20 (Kieselgel, Essigester)
   Massenspektrum (ESI⁺): m/z = 495, 497 [M+H]⁺
(2) 4-[(3-Chlor-4-fluor-phenyl)amino]-6-(1-methansulfonyl-piperidin-4-yloxy)-7-methoxy-chinazolin 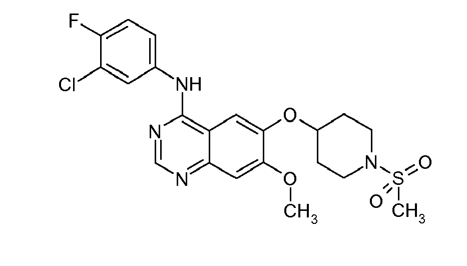
   R_{f}-Wert: 0.59 (Kieselgel, Essigester/Methanol/konz. wässriges Ammoniak = 90:10:1)
   Massenspektrum (ESI⁺): m/z = 481, 483 [M+H]⁺
(3) 4-[(3-Chlor-4-fluor-phenyl)amino]-6-{cis-4-[(3-chlor-propyl)sulfonylamino]-cyclohexan-1-yloxy}-7-methoxy-chinazolin 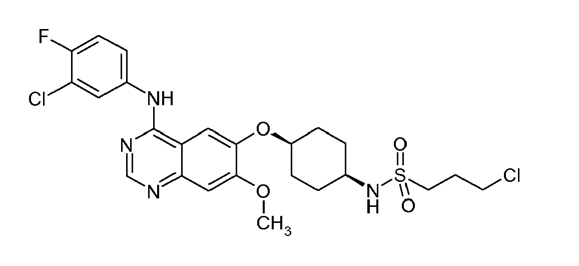
   Die Umsetzung erfolgt mit 3-Chlorpropansulfonylchlorid.
   R_{f}-Wert: 0.79 (Kieselgel, Essigester/Methanol = 9:1)
   Massenspektrum (ESI⁻): m/z = 555, 557, 559 [M-H]⁻
(4) 4-[(3-Chlor-4-fluor-phenyl)amino]-6-{trans-4-[(3-chlor-propyl)sulfonylamino]-cyclohexan-1-yloxy}-7-methoxy-chinazolin 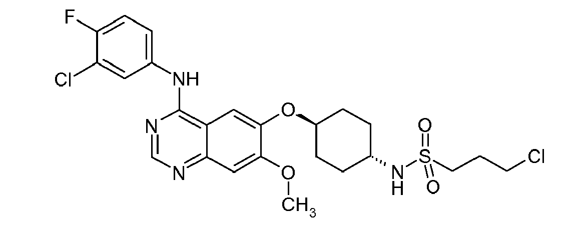
   Die Umsetzung erfolgt mit 3-Chlorpropansulfonylchlorid.
   R_{f}-Wert: 0.42 (Kieselgel, Essigester)
   Massenspektrum (ESI⁺): m/z = 557, 559, 561 [M+H]⁺
(5) 4-[(3-Chlor-4-fluor-phenyl)amino]-6-(1-methylcarbonyl-piperidin-4-yloxy)-7-methoxy-chinazolin 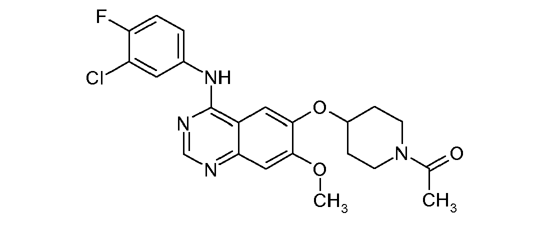
   Die Umsetzung erfolgt mit Acetanhydrid.
   Schmelzpunkt: 216°C
   Massenspektrum (ESI⁺): m/z = 445, 447 [M+H]⁺
(6) 4-[(3-Chlor-4-fluor-phenyl)amino]-6-{1-[(dimethylamino)carbonyl]-piperidin-4-yl-oxy}-7-methoxy-chinazolin 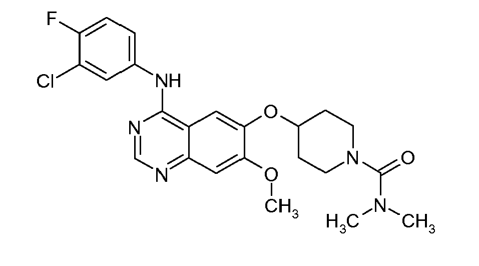
   Die Umsetzung erfolgt mit N,N-Dimethylcarbamoylchlorid.
   R_{f}-Wert: 0.28 (Reversed Phase DC-Fertigplatte (E. Merck), Acetonitril/Wasser/ Trifluoressigsäure = 50:50:1)
   Massenspektrum (ESI⁺): m/z = 474, 476 [M+H]⁺
(7) 4-[(3-Chlor-4-fluor-phenyl)amino]-6-{1-[(morpholin-4-yl)carbonyl]-piperidin-4-yl-oxy}-7-methoxy-chinazolin 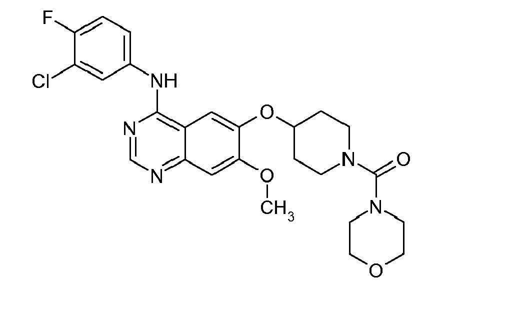
   Die Umsetzung erfolgt mit (Morpholin-4-yl)carbonylchlorid in Acetonitril.
   R_{f}-Wert: 0.37 (Reversed Phase DC-Fertigplatte (E. Merck), Acetonitril/Wasser/ Trifluoressigsäure = 50:50:1)
   Massenspektrum (ESI⁺): m/z = 516, 518 [M+H]⁺
(8) 4-[(3-Chlor-4-fluor-phenyl)amino]-6-{1-[(methoxymethyl)carbonyl]-piperidin-4-yl-oxy}-7-methoxy-chinazolin 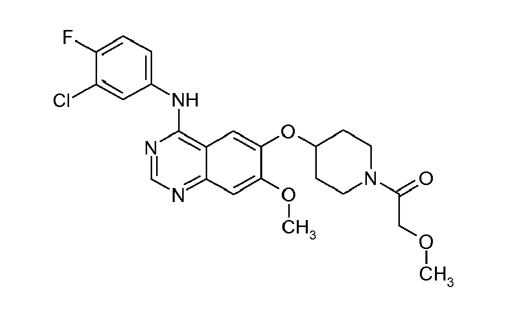
   Die Umsetzung erfolgt mit Methoxyessigsäurechlorid.
   R_{f}-Wert: 0.80 (Kieselgel, Methylenchlorid/Methanol = 9:1)
   Massenspektrum (ESI⁺): m/z = 475, 477 [M+H]⁺
(9) 4-[(3-Chlor-4-fluor-phenyl)amino]-6-(1-cyano-piperidin-4-yloxy)-7-methoxy-chinazolin 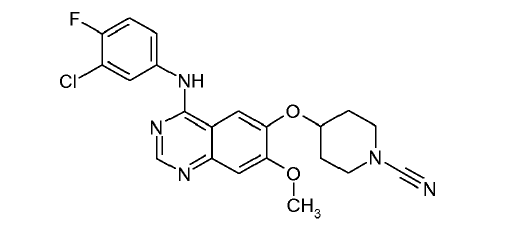
   Die Umsetzung erfolgt mit Bromcyan in Methylenchlorid.
   R_{f}-Wert: 0.40 (Reversed Phase DC-Fertigplatte (E. Merck), Acetonitril/Wasser/ Trifluoressigsäure = 50:50:1)
   Massenspektrum (ESI⁺): m/z = 428, 430 [M+H]⁺
(10) 4-[(3-Chlor-4-fluor-phenyl)amino]-6-{1-[(dimethylamino)sulfonyl]-piperidin-4-yloxy}-7-methoxy-chinazolin 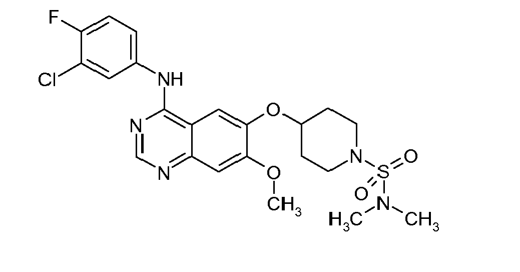
   Die Umsetzung erfolgt mit N,N-Dimethylsulfamoylchlorid in Acetonitril.
   R_{f}-Wert: 0.24 (Reversed Phase DC-Fertigplatte (E. Merck), Acetonitril/Wasser/ Trifluoressigsäure = 50:50:1)
   Massenspektrum (ESI⁺): m/z = 510, 512 [M+H]⁺
(11) 4-[(3-Chlor-4-fluor-phenyl)amino]-6-{1-[(morpholin-4-yl)sulfonyl]-piperidin-4-yloxy}-7-methoxy-chinazolin 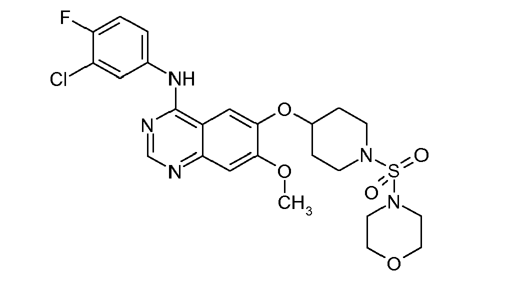
   Die Umsetzung erfolgt mit (Morpholin-4-yl)sulfonylchlorid in Acetonitril.
   R_{f}-Wert: 0.29 (Reversed Phase DC-Fertigplatte (E. Merck), Acetonitril/Wasser/ Trifluoressigsäure = 50:50:1)
   Massenspektrum (ESI⁺): m/z = 552, 554 [M+H]⁺
(12) 4-[(3-Chlor-4-fluor-phenyl)amino]-6-(1-methansulfonyl-piperidin-3-yloxy)-7-methoxy-chinazolin
   R_{f}-Wert: 0.33 (Reversed Phase DC-Fertigplatte (E. Merck), Acetonitril/Wasser/ Trifluoressigsäure = 50:50:1)
   Massenspektrum (ESI⁺): m/z = 481,483 [M+H]⁺
(13) 4-[(3-Chlor-4-fluor-phenyl)amino]-6-((S)-1-methansulfonyl-pyrrolidin-3-yloxy)-7-methoxy-chinazolin
   Schmelzpunkt: 249°C
   Massenspektrum (ESI⁺): m/z = 467, 469 [M+H]⁺
(14) 4-[(3-Chlor-4-fluor-phenyl)amino]-6-[1-(2-methansulfonylamino-ethyl)-piperidin-4-yloxy]-7-methoxy-chinazolin
   R_{f}-Wert: 0.49 (Reversed Phase DC-Fertigplatte (E. Merck), Acetonitril/Wasser/ Trifluoressigsäure = 50:50:1)
   Massenspektrum (ESI⁺): m/z = 524, 526 [M+H]⁺
(15) 4-[(3-Chlor-4-fluor-phenyl)amino]-6-[1-(2-acetylamino-ethyl)-piperidin-4-yloxy]-7-methoxy-chinazolin
   Die Umsetzung erfolgt mit Acetanhydrid.
   R_{f}-Wert: 0.51 (Reversed Phase DC-Fertigplatte (E. Merck), Acetonitril/Wasser/ Trifluoressigsäure = 50:50:1)
   Massenspektrum (ESI⁺): m/z = 488, 490 [M+H]⁺
(16) 4-[(3-Chlor-4-fluor-phenyl)amino]-6-{trans-4-[(dimethylamino)sulfonylamino]-cyclohexan-1-yloxy}-7-methoxy-chinazolin
   Die Umsetzung erfolgt mit N,N-Dimethylsulfamoylchlorid in Acetonitril.
   R_{f}-Wert: 0.69 (Kieselgel, Essigester/Methanol/konz. wässriges Ammoniak = 90:10:1)
   Massenspektrum (ESI⁺): m/z = 524, 526 [M+H]⁺
(17) 4-[(3-Chlor-4-fluor-phenyl)amino]-6-{trans-4-[(morpholin-4-yl)carbonylamino]-cyclohexan-1-yloxy}-7-methoxy-chinazolin
   Die Umsetzung erfolgt mit (Morpholin-4-yl)carbonylchlorid in Acetonitril.
   R_{f}-Wert: 0.38 (Kieselgel, Essigester/Methanol = 9:1)
   Massenspektrum (ESI⁺): m/z = 530, 532 [M+H]⁺
(18) 4-[(3-Chlor-4-fluor-phenyl)amino]-6-{trans-4-[(morpholin-4-yl)sulfonylamino]-cyclohexan-1-yloxy}-7-methoxy-chinazolin
   Die Umsetzung erfolgt mit (Morpholin-4-yl)sulfonylchlorid in Acetonitril.
   Schmelzpunkt: 237°C
   Massenspektrum (ESI⁻): m/z = 564, 566 [M-H]⁻
(19) 4-[(3-Chlor-4-fluor-phenyl)amino]-6-(3-methansulfonylamino-cyclohexan-1-yloxy)-7-methoxy-chinazolin
   R_{f}-Wert: 0.66 (Kieselgel, Essigester/Methanol = 9:1)
   Massenspektrum (ESI⁻): m/z = 493, 495 [M-H]⁻
(20) 4-[(3-Chlor-4-fluor-phenyl)amino]-6-(tetrahydropyran-4-yloxy)-7-(2-acetylamino-ethoxy)-chinazolin
   Die Umsetzung erfolgt mit Acetylchlorid in Acetonitril.
   Schmelzpunkt: 224°C
   Massenspektrum (ESI⁺): m/z = 475, 477 [M+H]⁺
(21) 4-[(3-Chlor-4-fluor-phenyl)amino]-6-(tetrahydropyran-4-yloxy)-7-(2-methansulfonylamino-ethoxy)-chinazolin
   Schmelzpunkt: 227°C
   Massenspektrum (ESI⁺): m/z = 511, 513 [M+H]⁺
(22) 4-[(3-Chlor-4-fluor-phenyl)amino]-6-(cis-3-acetylamino-cyclohexan-1-yloxy)-7-methoxy-chinazolin
   Die Umsetzung erfolgt mit Acetylchlorid in Acetonitril. Cis- und trans-Isomer werden chromatographisch über eine Kieselgelsäule getrennt.
   R_{f}-Wert: 0.43 (Kieselgel, Methylenchlorid/Methanol/konz. wässriges Ammoniak = 90:10:1)
   Massenspektrum (ESI⁺): m/z = 459, 461 [M+H]⁺
(23) 4-[(3-Chlor-4-fluor-phenyl)amino]-6-(trans-3-acetylamino-cyclohexan-1-yloxy)-7-methoxy-chinazolin
   Die Umsetzung erfolgt mit Acetylchlorid in Acetonitril. Cis- und trans-Isomer werden chromatographisch über eine Kieselgelsäule getrennt.
   R_{f}-Wert: 0.49 (Kieselgel, Methylenchlorid/Methanol/konz. wässriges Ammoniak = 90:10:1)
   Massenspektrum (ESI⁺): m/z = 459, 461 [M+H]⁺
(24) 4-[(3-Chlor-4-fluor-phenyl)amino]-6-(tetrahydropyran-4-yloxy)-7-(3-acetylamino-propyloxy)-chinazolin
   Die Umsetzung erfolgt mit Acetylchlorid.
   Schmelzpunkt: 225°C
   Massenspektrum (ESI⁺): m/z = 489, 491 [M+H]⁺
(25) 4-[(3-Chlor-4-fluor-phenyl)amino]-6-(tetrahydropyran-4-yloxy)-7-(3-methansulfonylamino-propyloxy)-chinazolin
   Schmelzpunkt: 222°C
   Massenspektrum (ESI⁺): m/z = 525, 527 [M+H]⁺
(26) 4-[(3-Chlor-4-fluor-phenyl)amino]-6-(1-methansulfonyl-piperidin-4-yloxy)-chinazolin
   R_{f}-Wert: 0.44 (Kieselgel, Methylenchlorid /Methanol = 9:1)
   Massenspektrum (ESI⁺): m/z = 451, 453 [M+H]⁺
(27) 4-[(3-Chlor-4-fluor-phenyl)amino]-6-{1-[(morpholin-4-yl)carbonyl]-piperidin-4-yloxy}-chinazolin
   Die Umsetzung erfolgt mit (Morpholin-4-yl)carbonylchlorid in Acetonitril.
   R_{f}-Wert: 0.40 (Kieselgel, Methylenchlorid /Methanol = 9:1)
   Massenspektrum (ESI⁺): m/z = 486, 488 [M+H]⁺
(28) 4-[(3-Chlor-4-fluor-phenyl)amino]-6-(1-acetyl-piperidin-4-yloxy)-chinazolin
   Die Umsetzung erfolgt mit Acetanhydrid.
   R_{f}-Wert: 0.50 (Kieselgel, Methylenchlorid /Methanol = 9:1)
   Massenspektrum (ESI⁺): m/z = 415, 417 [M+H]⁺
(29) 4-[(3-Chlor-4-fluor-phenyl)amino]-6-{1-[(dimethylamino)carbonyl]-piperidin-4-yloxy}-chinazolin
   Die Umsetzung erfolgt mit N,N-Dimethylcarbamoylchlorid.
   R_{f}-Wert: 0.47 (Kieselgel, Methylenchlorid /Methanol = 9:1)
   Massenspektrum (ESI⁺): m/z = 444, 446 [M+H]⁺
(30) 4-[(3-Chlor-4-fluor-phenyl)amino]-6-{trans-4-acetylamino-cydohexan-1-yloxy}-7-methoxy-chinazolin
   Die Umsetzung erfolgt mit Acetanhydrid.
   R_{f}-Wert: 0.50 (Kieselgel, Methylenchlorid/Methanol = 9:1)
   Massenspektrum (ESI⁺): m/z = 459, 461 [M+H]⁺
(31) 4-[(3-Chlor-4-fluor-phenyl)amino]-6-{trans-4-[(dimethylamino)carbonylamino]-cyclohexan-1-yloxy}-7-methoxy-chinazolin
   Die Umsetzung erfolgt mit N,N-Dimethylcarbamoylchlorid.
   R_{f}-Wert: 0.40 (Kieselgel, Methylenchlorid/Methanol = 9:1)
   Massenspektrum (ESI⁺): m/z = 488, 490 [M+H]⁺
(32) 4-[(3-Chlor-4-fluor-phenyl)amino]-6-[trans-4-(2-methoxy-acetylamino)-cyclohexan-1-yloxy]-7-methoxy-chinazolin
   Die Umsetzung erfolgt mit Methoxyessigsäurechlorid.
   R_{f}-Wert: 0.35 (Kieselgel, Methylenchlorid/Methanol = 9:1)
   Massenspektrum (ESI⁺): m/z = 489, 491 [M+H]⁺
(33) 4-[(3-Chlor-4-fluor-phenyl)amino]-6-[1-(2-methoxy-acetyl)-piperidin-4-yloxy]-chinazolin
   Die Umsetzung erfolgt mit Methoxyessigsäurechlorid.
   R_{f}-Wert: 0.41 (Kieselgel, Methylenchlorid/Methanol = 9:1)
   Massenspektrum (ESI⁺): m/z = 445, 447 [M+H]⁺
(34) 4-[(3-Chlor-4-fluor-phenyl)amino]-6-(1-isopropyloxycarbonyl-piperidin-4-yloxy)-7-methoxy-chinazolin
   Die Umsetzung erfolgt mit Chlorameisensäureisopropylester.
   R_{f}-Wert: 0.67 (Kieselgel, Essigester/Methanol/konz. wässriges Ammoniak = 98:2:1)
   Massenspektrum (ESI⁺): m/z = 489, 491 [M+H]⁺
(35) 4-[(3-Chlor-4-fluor-phenyl)amino]-6-(1-cyano-piperidin-4-yloxy)-chinazolin
   Die Umsetzung erfolgt mit Bromcyan in Methylenchlorid.
   R_{f}-Wert: 0.49 (Kieselgel, Methylenchlorid/Methanol = 9:1)
   Massenspektrum (ESI⁻): m/z = 396, 398 [M-H]⁻
(36) 4-[(3-Chlor-4-fluor-phenyl)amino]-6-{1-[(dimethylamino)sulfonyl]-piperidin-4-yloxy}-chinazolin
   Die Umsetzung erfolgt mit N,N-Dimethylsulfamoylchlorid in Acetonitril.
   R_{f}-Wert: 0.34 (Kieselgel, Methylenchlorid/Methanol = 9:1)
   Massenspektrum (ESI⁺): m/z = 480, 482 [M+H]⁺
(37) 4-[(3-Chlor-4-fluor-phenyl)amino]-6-{1-[(morpholin-4-yl)sulfonyl]-piperidin-4-yloxy}-chinazolin
   Die Umsetzung erfolgt mit (Morpholin-4-yl)sulfonylchlorid in Acetonitril.
   R_{f}-Wert: 0.15 (Kieselgel, Cyclohexan/Essigester = 1:1)
   Massenspektrum (ESI⁺): m/z = 522, 524 [M+H]⁺
(38) 4-[(3-Chlor-4-fluor-phenyl)amino]-6-[1-(2-acetylamino-ethyl)-piperidin-4-yloxy]-chinazolin
   Die Umsetzung erfolgt mit Acetanhydrid in Acetonitril.
   Schmelzpunkt: 221°C
   Massenspektrum (ESI⁺): m/z = 458, 460 [M+H]⁺
(39) 4-[(3-Chlor-4-fluor-phenyl)amino]-6-(1-[(diethylamino)carbonyl]-piperidin-4-yloxy)-7-methoxy-chinazolin
   Die Umsetzung erfolgt mit N,N-Diethylcarbamoylchlorid.
   R_{f}-Wert: 0.40 (Kieselgel, Essigester/Methanol/konz. wässriges Ammoniak = 95:5:1)
   Massenspektrum (ESI⁺): m/z = 502, 504 [M+H]⁺
(40) 4-[(3-Chlor-4-fluor-phenyl)amino]-6-{1-[(piperidin-1-yl)carbonyl]-piperidin-4-yloxy}-7-methoxy-chinazolin
   Die Umsetzung erfolgt mit (Piperidin-1-yl)carbonylchlorid.
   R_{f}-Wert: 0.51 (Kieselgel, Essigester/Methanol/konz. wässriges Ammoniak = 95:5:1)
   Massenspektrum (ESI⁻): m/z = 512, 514 [M-H]⁻
(41) 4-[(3-Chlor-4-fluor-phenyl)amino]-6-{1-[(pyrrolidin-1-yl)carbonyl]-piperidin-4-yloxy}-7-methoxy-chinazolin
   Die Umsetzung erfolgt mit (Pyrrolidin-1-yl)carbonylchlorid.
   Schmelzpunkt: 237°C
   Massenspektrum (ESI⁺): m/z = 500, 502 [M+H]⁺
(42) 4-[(3-Chlor-4-fluor-phenyl)amino]-6-{1-[(4-methyl-piperazin-1-yl)carbonyl]-piperidin-4-yloxy}-7-methoxy-chinazolin
   Die Umsetzung erfolgt mit (4-Methyl-piperazin-1-yl)carbonylchlorid-hydrochlorid.
   R_{f}-Wert: 0.28 (Kieselgel, Methylenchlorid/Methanol/konz. wässriges Ammoniak = 90:10:1)
   Massenspektrum (ESI⁻): m/z = 527, 529 [M-H]⁻
(43) 4-[(3-Chlor-4-fluor-phenyl)amino]-6-[cis-4-(N-methansulfonyl-N-methyl-amino)-cyclohexan-1-yloxy]-7-methoxy-chinazolin
   Die Umsetzung erfolgt in Methylenchlorid.
   R_{f}-Wert: 0.71 (Kieselgel, Essigester/Methanol = 9:1)
   Massenspektrum (ESI⁺): m/z = 509, 51 [M+H]⁺
(44) 4-[(3-Chlor-4-fluor-phenyl)amino]-6-[cis-4-(N-acetyl-N-methyl-amino)-cyclohexan-1-yloxy]-7-methoxy-chinazolin
   Die Umsetzung erfolgt mit Acetanhydrid.
   Schmelzpunkt: 234°C
   Massenspektrum (ESI⁺): m/z = 473, 475 [M+H]⁺
(45) 4-[(3-Chlor-4-fluor-phenyl)amino]-6-(cis-4-[N-(2-methoxy-acetyl)-N-methyl-amino]-cyclohexan-1-yloxy)-7-methoxy-chinazolin
   Die Umsetzung erfolgt mit Methoxyessigsäurechlorid.
   R_{f}-Wert: 0.40 (Kieselgel, Essigester/Methanol = 9:1)
   Massenspektrum (ESI⁺): m/z = 503, 505 [M+H]⁺
(46) 4-[(3-Chlor-4-fluor-phenyl)amino]-6-[cis-4-(N-dimethylaminocarbonyl-N-methylamino)-cyclohexan-1-yloxy]-7-methoxy-chinazolin
   Die Umsetzung erfolgt mit N,N-Dimethylcarbamoylchlorid.
   R_{f}-Wert: 0.51 (Kieselgel, Essigester/Methanol = 9:1)
   Massenspektrum (ESI⁺): m/z = 502, 504 [M+H]⁺
(47) 4-[(3-Chlor-4-fluor-phenyl)amino]-6-(cis-4-{N-[(morpholin-4-yl)carbonyl]-N-methylamino}-cyclohexan-1-yloxy)-7-methoxy-chinazolin
   Die Umsetzung erfolgt mit (Morpholin-4-yl)carbonylchlorid.
   R_{f}-Wert: 0.50 (Kieselgel, Essigester/Methanol = 9:1)
   Massenspektrum (ESI⁺): m/z = 544, 546 [M+H]⁺
(48) 4-[(3-Chlor-4-fluor-phenyl)amino]-6-(cis-4-{N-[(morpholin-4-yl)sulfonyl]-N-methylamino}-cyclohexan-1-yloxy)-7-methoxy-chinazolin
   Die Umsetzung erfolgt mit (Morpholin-4-yl)sulfonylchlorid in Acetonitril.
   R_{f}-Wert: 0.24 (Reversed Phase DC-Fertigplatte (E. Merck), Acetonitril/Wasser/ Trifluoressigsäure = 50:50:1)
   Massenspektrum (ESI⁺): m/z = 580, 582 [M+H]⁺
(49) 4-[(3-Chlor-4-fluor-phenyl)amino]-6-[cis-4-(N-dimethylaminosulfonyl-N-methylamino)-cyclohexan-1-yloxy]-7-methoxy-chinazolin
   Die Umsetzung erfolgt mit N,N-Dimethylsulfamoylchlorid in Acetonitril.
   R_{f}-Wert: 0.53 (Kieselgel, Essigester)
   Massenspektrum (ESI⁺): m/z = 538, 540 [M+H]⁺
(50) 4-[(3-Chlor-4-fluor-phenyl)amino]-6-(trans-4-ethansulfonylamino-cyclohexan-1-yloxy)-7-methoxy-chinazolin
   Die Umsetzung erfolgt mit Ethansulfonsäurechlorid in Methylenchlorid.
   R_{f}-Wert: 0.41 (Kieselgel, Essigester)
   Massenspektrum (ESI⁺): m/z = 509, 511 [M+H]⁺
(51) 4-[(3-Chlor-4-fluor-phenyl)amino]-6-{1-[(morpholin-4-yl)carbonyl]-piperidin-4-yloxy}-7-ethoxy-chinazolin
   Die Umsetzung erfolgt mit (Morpholin-4-yl)carbonylchlorid.
   R_{f}-Wert: 0.48 (Kieselgel, Methylenchlorid/Methanol = 9:1)
   Massenspektrum (ESI⁺): m/z = 530, 532 [M+H]⁺
(52) 4-[(3-Chlor-4-fluor-phenyl)amino]-6-(1-methansulfonyl-piperidin-4-yloxy)-7-ethoxy-chinazolin
   R_{f}-Wert: 0.50 (Kieselgel, Methylenchlorid/Methanol = 9:1)
   Massenspektrum (ESI⁺): m/z = 495, 497 [M+H]⁺
(53) 4-[(3-Chlor-4-fluor-phenyl)amino]-6-[1-(2-methoxy-acetyl)-piperidin-4-yloxy]-7-ethoxy-chinazolin
   Die Umsetzung erfolgt mit Methoxyessigsäurechlorid.
   R_{f}-Wert: 0.40 (Kieselgel, Methylenchlorid/Methanol = 20:1)
   Massenspektrum (ESI⁺): m/z = 489, 491 [M+H]⁺
(54) 4-[(3-Chlor-4-fluor-phenyl)amino]-6-(1-methansulfonyl-piperidin-4-yloxy)-7-(2-methoxy-ethoxy)-chinazolin
   R_{f}-Wert: 0.47 (Kieselgel, Methylenchlorid/Methanol = 9:1)
   Massenspektrum (ESI⁺): m/z = 525, 527 [M+H]⁺
(55) 4-[(3-Chlor-4-fluor-phenyl)amino]-6-{1-[(morpholin-4-yl)carbonyl]-piperidin-4-yloxy}-7-(2-methoxy-ethoxy)-chinazolin
   Die Umsetzung erfolgt mit (Morpholin-4-yl)carbonylchlorid.
   R_{f}-Wert: 0.48 (Kieselgel, Methylenchlorid/Methanol = 9:1)
   Massenspektrum (ESI⁺): m/z = 560, 562 [M+H]⁺
(56) 4-[(3-Chlor-4-fluor-phenyl)amino]-6-[1-(2-methoxy-acetyl)-piperidin-4-yloxy]-7-(2-methoxy-ethoxy)-chinazolin
   Die Umsetzung erfolgt mit Methoxyessigsäurechlorid.
   R_{f}-Wert: 0.48 (Kieselgel, Methylenchlorid/Methanol = 9:1)
   Massenspektrum (ESI⁺): m/z = 519, 521 [M+H]⁺
(57) 4-[(3-Chlor-4-fluor-phenyl)amino]-6-(cis-4-acetylamino-cyclohexan-1-yloxy)-7-methoxy-chinazolin
   Die Umsetzung erfolgt mit Acetanhydrid.
   Schmelzpunkt: 281°C
   Massenspektrum (ESI⁺): m/z = 459, 461 [M+H]⁺
(58) 4-[(3-Chlor-4-fluor-phenyl)amino]-6-[cis-4-(2-methoxy-acetylamino)-cyclohexan-1-yloxy]-7-methoxy-chinazolin
   Die Umsetzung erfolgt mit Methoxyessigsäurechlorid.
   Schmelzpunkt: 264°C
   Massenspektrum (ESI⁺): m/z = 489, 491 [M+H]⁺
(59) 4-[(3-Chlor-4-fluor-phenyl)amino]-6-(cis-4-{N-[(piperidin-1-yl)carbonyl]-N-methylamino}-cyclohexan-1-yloxy)-7-methoxy-chinazolin
   Die Umsetzung erfolgt mit (Piperidin-1-yl)carbonylchlorid.
   Schmelzpunkt: 253°C
   Massenspektrum (ESI⁺): m/z = 542, 544 [M+H]⁺
(60) 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
   Die Umsetzung erfolgt mit (4-Methyl-piperazin-1-yl)carbonylchlorid-hydrochlorid.
   Schmelzpunkt: 262°C
   Massenspektrum (ESI⁺): m/z = 557, 559 [M+H]⁺
(61) 4-[(3-Chlor-4-fluor-phenyl)amino]-6-[cis-4-(N-ethansulfonyl-N-methyl-amino)-cyclohexan-1-yloxy]-7-methoxy-chinazolin
   Die Umsetzung erfolgt mit Ethansulfonsäurechlorid in Methylenchlorid.
   R_{f}-Wert: 0.19 (Reversed Phase DC-Fertigplatte (E. Merck), Acetonitril/Wasser/ Trifluoressigsäure = 50:50:1)
   Massenspektrum (ESI⁺): m/z = 523, 525 [M+H]⁺
(62) 4-[(3-Chlor-4-fluor-phenyl)amino]-6-{cis-4-[(morpholin-4-yl)carbonylamino]-cyclohexan-1-yloxy}-7-methoxy-chinazolin
   Die Umsetzung erfolgt mit (Morpholin-4-yl)carbonylchlorid.
   R_{f}-Wert: 0.33 (Reversed Phase DC-Fertigplatte (E. Merck), Acetonitril/Wasser/ Trifluoressigsäure = 50:50:1)
   Massenspektrum (ESI⁺): m/z = 530, 532 [M+H]⁺
(63) 4-[(3-Chlor-4-fluor-phenyl)amino]-6-{cis-4-[(morpholin-4-yl)sulfonylamino]-cyclohexan-1-yloxy}-7-methoxy-chinazolin
   Die Umsetzung erfolgt mit (Morpholin-4-yl)sulfonylchlorid in Acetonitril.
   R_{f}-Wert: 0.81 (Kieselgel, Essigester/Methanol = 9:1)
   Massenspektrum (ESI⁺): m/z = 566, 568 [M+H]⁺
(64) 4-[(3-Ethinyl-phenyl)amino]-6-(1-acetyl-piperidin-4-yloxy)-7-methoxy-chinazolin
   Die Umsetzung erfolgt mit Acetanhydrid.
   R_{f}-Wert: 0.30 (Kieselgel, Essigester/Methanol/konz. wässriges Ammoniak = 90:10:1)
   Massenspektrum (ESI⁺): m/z = 417 [M+H]⁺
(65) 4-[(3-Ethinyl-phenyl)amino]-6-[1-(2-methoxy-acetyl)-piperidin-4-yloxy]-7-methoxy-chinazolin
   Die Umsetzung erfolgt mit Methoxyessigsäurechlorid.
   R_{f}-Wert: 0.37 (Kieselgel, Methylenchlorid/Methanol/konz. wässriges Ammoniak = 90:10:1)
   Massenspektrum (ESI⁺): m/z = 447 [M+H]⁺
(66) 4-[(3-Ethinyl-phenyl)amino]-6-(1-methansulfonyl-piperidin-4-yloxy)-7-methoxy-chinazolin
   R_{f}-Wert: 0.59 (Kieselgel, Essigester/Methanol/konz. wässriges Ammoniak = 90:10:1)
   Massenspektrum (ESI⁺): m/z = 453 [M+H]⁺
(67) 4-[(3-Ethinyl-phenyl)amino]-6-{1-[(morpholin-4-yl)carbonyl]-piperidin-4-yloxy}-7-methoxy-chinazolin
   Die Umsetzung erfolgt mit (Morpholin-4-yl)carbonylchlorid.
   R_{f}-Wert: 0.43 (Kieselgel, Essigester/Methanol/konz. wässriges Ammoniak = 90:10:1)
   Massenspektrum (ESI⁺): m/z = 488 [M+H]⁺
(68) 4-[(3-Chlor-4-fluor-phenyl)amino]-6-[trans-4-(N-methansulfonyl-N-methyl-amino)-cyclohexan-1-yloxy]-7-methoxy-chinazolin
   R_{f}-Wert: 0.50 (Kieselgel, Methylenchlorid /Methanol = 9:1)
   Massenspektrum (ESI⁺): m/z = 509, 511 [M+H]⁺
(69) 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
   Die Umsetzung erfolgt mit (Morpholin-4-yl)carbonylchlorid.
   R_{f}-Wert: 0.54 (Kieselgel, Methylenchlorid /Methanol = 9:1)
   Massenspektrum (ESI⁺): m/z = 544, 546 [M+H]⁺
(70) 4-{[3-Methyl-4-(pyridin-3-yloxy)-phenyl]amino}-6-(1-methansulfonyl-piperidin-4-yloxy)-7-methoxy-chinazolin-methansulfonat
   R_{f}-Wert: 0.58 (Reversed Phase DC-Fertigplatte (E. Merck), Acetonitril/Wasser/ Trifluoressigsäure = 50:50:1)
   Massenspektrum (ESI⁺): m/z = 536 [M+H]⁺
(71) 4-({3-Chlor-4-[(3-fluor-benzyl)oxy]-phenyl}amino)-6-(1-methansulfonyl-piperidin-4-yloxy)-7-methoxy-chinazolin
   R_{f}-Wert: 0.80 (Kieselgel, Methylenchlorid/Methanol = 9:1)
   Massenspektrum (ESI⁺): m/z = 587, 589 [M+H]⁺

### Beispiel 4

### 4-[(3-Chlor-4-fluor-phenyl)amino]-6-(cis-4-{[3-(morpholin-4-yl)-propyl]sulfonylamino)-cyclohexan-1-yloxy)-7-methoxy-chinazolin

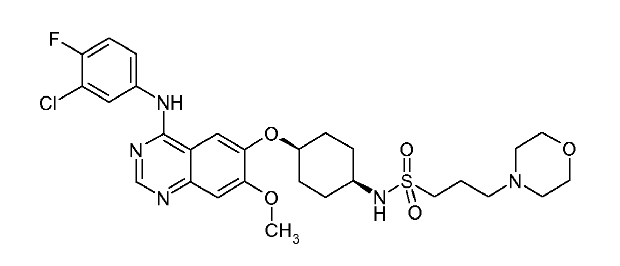

Zu 60 mg 4-[(3-Chlor-4-fluor-phenyl)amino]-6-{cis-4-[(3-chlor-propyl)sulfonylamino]-cyclohexan-1-yloxy}-7-methoxy-chinazolin in 2 ml Acetonitril werden 23 µl Morpholin gegeben und das Reaktionsgemisch wird über Nacht unter Rückfluß erhitzt. Zur Aufarbeitung wird das Gemisch in Essigester aufgenommen und mit Wasser gewaschen. Die organische Phase wird über Magnesiumsulfat getrocknet und eingeengt. Das Rohprodukt wird über eine Kieselgelsäule mit Methylenchlorid/Methanol (9:1) als Laufmittel gereinigt.
Ausbeute: 18 mg (27% der Theorie)
R_{f}-Wert: 0.36 (Kieselgel, Methylenchlorid /Methanol = 9:1)
Massenspektrum (ESI⁺): m/z = 608, 610 [M+H]⁺

Analog Beispiel 4 werden folgende Verbindungen erhalten:
(1) 4-[(3-Chlor-4-fluor-phenyl)amino]-6-(trans-4-{[3-(morpholin-4-yl)-propyl]sulfonyl-amino}-cyclohexan-1-yloxy)-7-methoxy-chinazolin 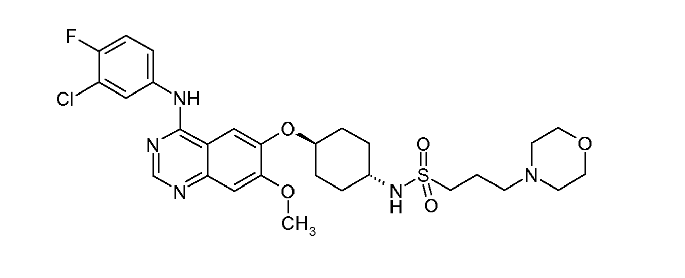
   R_{f}-Wert: 0.16 (Kieselgel, Essigester/Methanol/konz. wässriges Ammoniak = 90:10:1)
   Massenspektrum (ESI⁺): m/z = 608, 610 [M+H]⁺
(2) 4-[(3-Chlor-4-fluor-phenyl)amino]-6-(tetrahydropyran-4-yloxy)-7-[4-(morpholin-4-yl)-butyloxy]-chinazolin
   Durchführung in Gegenwart von Natriumcarbonat und Natriumiodid in N-Methylpyrrolidon bei 100°C.
   R_{f}-Wert: 0.18 (Kieselgel, Essigester/Methanol/konz. wässriges Ammoniak = 40:10:0.5)
   Massenspektrum (ESI⁺): m/z = 531, 533 [M+H]⁺
(3) 4-[(3-Chlor-4-fluor-phenyl)amino]-6-((S)-tetrahydrofuran-3-yloxy)-7-[4-(morpholin-4-yl)-butyloxy]-chinazolin
   Durchführung in Gegenwart von Natriumcarbonat und Natriumiodid in N-Methylpyrrolidon bei 100°C.
   R_{f}-Wert: 0.32 (Kieselgel, Essigester/Methanol/konz. wässriges Ammoniak = 80:20:1)
   Massenspektrum (ESI⁺): m/z = 517, 519 [M+H]⁺
(4) 4-[(3-Chlor-4-fluor-phenyl)amino]-6-{1-[(morpholin-4-yl)acetyl]-piperidin-4-yloxy}-chinazolin
   Durchführung in Gegenwart von Hünigbase in Tetrahydrofuran bei Raumtemperatur.
   R_{f}-Wert: 0.30 (Kieselgel, Methylenchlorid /Methanol = 9:1)
   Massenspektrum (ESI⁺): m/z = 500, 502 [M+H]⁺
(5) 4-[(3-Chlor-4-fluor-phenyl)amino]-6-(1-dimethylaminoacetyl-piperidin-4-yloxy)-chinazolin
   Durchführung in Gegenwart von Hünigbase in Tetrahydrofuran bei Raumtemperatur.
   R_{f}-Wert: 0.11 (Kieselgel, Methylenchlorid/Methanol/konz. wässriges Ammoniak = 90:10:1)
   Massenspektrum (ESI⁺): m/z = 458, 460 [M+H]⁺
(6) 4-[(3-Chlor-4-fluor-phenyl)amino]-6-(1-dimethylaminoacetyl-piperidin-4-yloxy)-7-methoxy-chinazolin
   Durchführung in Gegenwart von Hünigbase in Tetrahydrofuran bei Raumtemperatur.
   R_{f}-Wert: 0.19 (Kieselgel, Essigester/Methanol/konz. wässriges Ammoniak = 90:10:1)
   Massenspektrum (ESI⁺): m/z = 488, 490 [M+H]⁺
(7) 4-[(3-Chlor-4-fluor-phenyl)amino]-6-{1-[(morpholin-4-yl)acetyl]-piperidin-4-yloxy}-7-methoxy-chinazolin
   Durchführung in Gegenwart von Hünigbase in Tetrahydrofuran bei Raumtemperatur.
   Massenspektrum (ESI⁺): m/z = 530, 532 [M+H]⁺

### Beispiel 5

### 4-[(3-Chlor-4-fluor-phenyl)amino]-6-((S)-pyrrolidin-3-yloxy)-7-methoxy-chinazolin-dihydrochlorid

Eine Lösung aus 370 mg 4-[(3-Chlor-4-fluor-phenyl)amino]-6-[(S)-1-(tert.-butyloxycarbonyl)-pyrrolidin-3-yloxy]-7-methoxy-chinazolin in 5 ml Dioxan wird mit 0.32 ml konzentrierter Salzsäure versetzt und über Nacht bei Raumtemperatur gerührt. Der ausgefallene Niederschlag wird abgesaugt und mit reichlich Dioxan nachgewaschen. Das Rohprodukt wird in wenig Methanol gelöst und durch Zugabe der gleichen Menge Essigester wieder ausgefällt. Der so erhaltene weiße Feststoff wird abgesaugt und getrocknet.
Ausbeute: 200 mg (57 % derTheorie)
Schmelzpunkt: 281°C
Massenspektrum (ESI⁺): m/z = 389, 391 [M+H]⁺

Analog Beispiel 5 werden folgende Verbindungen erhalten:
(1) 4-[(3-Chlor-4-fluor-phenyl)amino]-6-(piperidin-3-yloxy)-7-methoxy-chinazolin-dihydrochlorid
   Schmelzpunkt: 263°C
   Massenspektrum (ESI⁺): m/z = 403, 505 [M+H]⁺
(2) 4-[(3-Chlor-4-fluor-phenyl)amino]-6-[1-(2-amino-ethyl)-piperidin-4-yloxy]-7-methoxy-chinazolin-dihydrochlorid
   Schmelzpunkt: 277°C
   Massenspektrum (ESI⁺): m/z = 446, 448 [M+H]⁺
(3) 4-[(3-Chlor-4-fluor-phenyl)amino]-6-(3-amino-cyclohexan-1-yloxy)-7-methoxy-chinazolin-dihydrochlorid
   Massenspektrum (ESI⁺): m/z = 417, 419 [M+H]⁺
(4) 4-[(3-Chlor-4-fluor-phenyl)amino]-6-(tetrahydropyran-4-yloxy)-7-(2-amino-ethoxy)-chinazolin-dihydrochlorid
   Durchführung mit isopropanolischer Salzsäure (5-6 M) in Methylenchlorid.
   R_{f}-Wert: 0.58 (Reversed Phase DC-Fertigplatte (E. Merck), Acetonitril/Wasser/ Trifluoressigsäure = 50:50:1)
   Massenspektrum (ESI⁺): m/z = 433, 435 [M+H]⁺
(5) 4-[(3-Chlor-4-fluor-phenyl)amino]-6-(tetrahydropyran-4-yloxy)-7-(3-amino-propyloxy)-chinazolin-dihydrochlorid
   Durchführung mit isopropanolischer Salzsäure (5-6 M) in Methylenchlorid.
   R_{f}-Wert: 0.44 (Reversed Phase DC-Fertigplatte (E. Merck), Methanol/5%ige wässrige Natriumchlorid-Lösung = 7:3)
   Massenspektrum (ESI⁺): m/z = 447, 449 [M+H]⁺
(6) 4-[(3-Chlor-4-fluor-phenyl)amino]-6-[1-(2-amino-ethyl)-piperidin-4-yloxy]-chinazolin-dihydrochlorid
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 416, 418 [M+H]⁺
(7) 4-[(3-Chlor-4-fluor-phenyl)amino]-6-(cis-4-methylamino-cyclohexan-1-yloxy)-7-methoxy-chinazolin-dihydrochlorid
   Durchführung mit isopropanolischer Salzsäure (5-6 M) in Methylenchlorid.
   R_{f}-Wert: 0.35 (Reversed Phase DC-Fertigplatte (E. Merck), Acetonitril/Wasser/ Trifluoressigsäure = 50:50:1)
   Massenspektrum (ESI⁺): m/z = 431, 433 [M+H]⁺
(8) 4-[(3-Ethinyl-phenyl)amino]-6-(piperidin-4-yloxy)-7-methoxy-chinazolin-dihydrochlorid
   Durchführung mit isopropanolischer Salzsäure (5-6 M) in Methylenchlorid.
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 375 [M+H]⁺
(9) 4-[(3-Chlor-4-fluor-phenyl)amino]-6-(trans-4-methylamino-cyclohexan-1-yloxy)-7-methoxy-chinazolin-dihydrochlorid
   Schmelzpunkt: 251°C
   Massenspektrum (ESI⁺): m/z = 431, 433 [M+H]⁺
(10) 4-{[3-Methyl-4-(pyridin-3-yloxy)-phenyl]amino}-6-(piperidin-4-yloxy)-7-methoxy-chinazolin-dihydrochlorid
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 458 [M+H]⁺
(11) 4-({3-Chlor-4-[(3-fluor-benzyl)oxy]-phenyl}amino)-6-(piperidin-4-yloxy)-7-methoxy-chinazolin-dihydrochlorid
   R_{f}-Wert: 0.38 (Reversed Phase DC-Fertigplatte (E. Merck), Acetonitril/Wasser/ Trifluoressigsäure = 50:50:1)
   Massenspektrum (ESI⁻): m/z = 507, 509 [M-H]⁻
(12) 4-{[3-Methyl-4-(pyridin-3-yloxy)-phenyl]amino}-6-(trans-4-amino-cyclohexan-1-yloxy)-7-methoxy-chinazolin-dihydrochlorid
   R_{f}-Wert: 0.58 (Reversed Phase DC-Fertigplatte (E. Merck), Acetonitril/Wasser/ Trifluoressigsäure = 50:50:1)
   Massenspektrum (ESI⁺): m/z = 472 [M+H]⁺
(13) 4-({3-Chlor-4-[(3-fluor-benzyl)oxy]-phenyl}amino)-6-(trans-4-amino-cyclohexan-1-yloxy)-7-methoxy-chinazolin-dihydrochlorid
   R_{f}-Wert: 0.48 (Reversed Phase DC-Fertigplatte (E. Merck), Acetonitril/Wasser/ Trifluoressigsäure = 50:50:1)
   Massenspektrum (ESI⁺): m/z = 523, 525 [M+H]⁺

### Beispiel 6

### 4-[(3-Chlor-4-fluor-phenyl)amino]-6-(tetrahydropyran-4-yloxy)-7-hydroxy-chinazolin

Ein Gemisch aus 9.00 g 4-[(3-Chlor-4-fluor-phenyl)amino]-6-(tetrahydropyran-4-yloxy)-7-benzyloxy-chinazolin-hydrochlorid und 50 ml Trifluoressigsäure wird 1.5 Stunden auf 100°C erhitzt. Anschließend wird das Reaktionsgemisch eingeengt und der Rückstand in 10 ml Acetonitril aufgenommen. Diese Lösung wird unter kräftigem Rühren auf 100 ml gesättigte Natriumhydrogencarbonat-Lösung getropft. Nach 1.5 Stunden wird der entstandene Niederschlag abgesaugt und mehrmals mit Wasser nachgewaschen. Das Rohprodukt wird mit Diethylether verrührt, abgesaugt und getrocknet.
Ausbeute: 5.90 g (87 % der Theorie)
R_{f}-Wert: 0.21 (Kieselgel, Essigester)
Massenspektrum (ESI⁺): m/z = 390, 392 [M+H]⁺

Analog Beispiel 6 werden folgende Verbindungen erhalten:
(1) 4-[(3-Chlor-4-fluor-phenyl)amino]-6-((*S*)-tetrahydrofuran-3-yloxy)-7-hydroxy-chinazolin
   R_{f}-Wert: 0.44 (Kieselgel, Essigester/Methanol = 9:1)
   Massenspektrum (ESI⁺): m/z = 376, 378 [M+H]⁺

### Beispiel 7

### 4-[(3-Chlor-4-fluor-phenyl)amino]-6-(tetrahydropyran-4-yloxy)-7-[3-(morpholin-4-yl)-propyloxy]-chinazolin

Ein Gemisch aus 300 mg 4-[(3-Chlor-4-fluor-phenyl)amino]-6-(tetrahydropyran-4-yloxy)-7-hydroxy-chinazolin, 130 mg 3-(Morpholin-4-yl)-propylchlorid und 530 mg Kaliumcarbonat in 5 ml N,N-Dimethylformamid wird über Nacht bei 80°C gerührt. Zur Aufarbeitung wird das Reaktionsgemisch mit 25 ml Wasser verdünnt und mit Essigester extrahiert. Die vereinigten organischen Phasen werden mit gesättigter Natriumchloridlösung gewaschen, über Magnesiumsulfat getrocknet und eingeengt. Der Rückstand wird mit Diethylether verrührt, abgesaugt und getrocknet.
Ausbeute: 250 mg (63 % der Theorie)
Schmelzpunkt: 205°C
Massenspektrum (ESI⁺): m/z = 517, 519 [M+H]⁺

Analog Beispiel 7 werden folgende Verbindungen erhalten:
(1) 4-[(3-Chlor-4-fluor-phenyl)amino]-6-(tetrahydropyran-4-yloxy)-7-[2-(morpholin-4-yl)-ethoxy]-chinazolin
   R_{f}-Wert: 0.33 (Kieselgel, Essigester/Methanol/konz. wässriges Ammoniak = 40:10:0.5)
   Massenspektrum (ESI⁺): m/z = 503, 505 [M+H]⁺
(2) 4-[(3-Chlor-4-fluor-phenyl)amino]-6-(tetrahydropyran-4-yloxy)-7-ethoxy-chinazolin
   R_{f}-Wert: 0.76 (Kieselgel, Essigester/Methanol/konz. wässriges Ammoniak = 90:10:1)
   Massenspektrum (ESI⁺): m/z = 418, 420 [M+H]⁺
(3) 4-[(3-Chlor-4-fluor-phenyl)amino]-6-((S)-tetrahydrofuran-3-yloxy)-7-[3-(morpholin-4-yl)-propyloxy]-chinazolin
   R_{f}-Wert: 0.20 (Kieselgel, Essigester/Methanol/konz. wässriges Ammoniak = 90:10:1)
   Massenspektrum (ESI⁻): m/z = 501, 503[M-H]⁻
(4) 4-[(3-Chlor-4-fluor-phenyl)amino]-6-((S)-tetrahydrofuran-3-yloxy)-7-[2-(morpholin-4-yl)-ethoxy]-chinazolin
   R_{f}-Wert: 0.19 (Kieselgel, Essigester/Methanol/konz. wässriges Ammoniak = 90:10:1)
   Massenspektrum (ESI⁺): m/z = 489, 491 [M+H]⁺
(5) 4-[(3-Chlor-4-fluor-phenyl)amino]-6-(tetrahydropyran-4-yloxy)-7-(2-methoxy-ethoxy)-chinazolin
   R_{f}-Wert: 0.57 (Kieselgel, Methylenchlorid /Methanol = 9:1)
   Massenspektrum (ESI⁺): m/z = 448, 450 [M+H]⁺
(6) 4-[(3-Chlor-4-fluor-phenyl)amino]-6-(tetrahydropyran-4-yloxy)-7-[2-(tert.-butyloxycarbonylamino)-ethoxy]-chinazolin
   R_{f}-Wert: 0.64 (Kieselgel, Essigester/Methanol/konz. wässriges Ammoniak = 95:5:0.1)
   Massenspektrum (ESI⁺): m/z = 533, 535 [M+H]⁺
(7) 4-[(3-Chlor-4-fluor-phenyl)amino]-6-(tetrahydropyran-4-yloxy)-7-[3-(tert.-butyloxycarbonylamino)-propyloxy]-chinazolin
   R_{f}-Wert: 0.74 (Kieselgel, Essigester/Methanol/konz. wässriges Ammoniak = 95:5:0.1)
   Massenspektrum (ESI⁺): m/z = 547, 549 [M+H]⁺

### Beispiel 8

### 4-[(3-Chlor-4-fluor-phenyl)amino]-6-(piperidin-4-yloxy)-chinazolin

Eine Lösung aus 4.55 g 4-[(3-Chlor-4-fluor-phenyl)amino]-6-(1-trifluoracetyl-piperidin-4-yloxy)-chinazolin-hydrochlorid in 35 ml Methanol wird mit 13 ml 3 N Natronlauge versetzt und ca. eine halbe Stunde bei Raumtemperatur gerührt. Zur Aufarbeitung wird das Reaktionsgemisch mit Wasser verdünnt und mit Essigester extrahiert. Die vereinigten organischen Phasen werden mit gesättigter Natriumchlorid-Lösung gewaschen, über Magnesiumsulfat getrocknet und eingeengt. Der Rückstand wird mit Diethylether verrührt, abgesaugt und getrocknet.
Ausbeute: 3.00 g (89 % der Theorie)
R_{f}-Wert: 0.48 (Reversed Phase DC-Fertigplatte (E. Merck), Acetonitril/Wasser/ Trifluoressigsäure = 50:50:1)
Massenspektrum (ESI⁺): m/z = 373, 375 [M+H]⁺

Analog Beispiel 8 werden folgende Verbindungen erhalten:
(1) 4-[(3-Chlor-4-fluor-phenyl)amino]-6-(piperidin-4-yloxy)-7-ethoxy-chinazolin
   R_{f}-Wert: 0.20 (Kieselgel, Methylenchlorid/Methanol/konz. wässriges Ammoniak = 90:10:1)
   Massenspektrum (ESI⁺): m/z = 417, 419 [M+H]⁺
(2) 4-[(3-Chlor-4-fluor-phenyl)amino]-6-(piperidin-4-yloxy)-7-(2-methoxy-ethoxy)-chinazolin
   R_{f}-Wert: 0.10 (Kieselgel, Methylenchlorid/Methanol/konz. wässriges Ammoniak = 90:10:1)
   Massenspektrum (ESI⁺): m/z = 447, 449 [M+H]⁺

### Beispiel 9

### 4-[(3-Chlor-4-fluor-phenyl)amino]-6-{1-[(ethylamino)carbonyl]-piperidin-4-yloxy}-7-methoxy-chinazolin

Hergestellt durch Umsetzung von 4-[(3-Chlor-4-fluor-phenyl)amino]-6-(piperidin-4-yloxy)-7-methoxy-chinazolin mit Ethylisocyanat in Tetrahydrofuran bei Raumtemperatur.
R_{f}-Wert: 0.53 (Kieselgel, Essigester/Methanol/konz. wässriges Ammoniak = 90:10:1)
Massenspektrum (ESI⁺): m/z = 474, 476 [M+H]⁺

Analog Beispiel 9 werden folgende Verbindungen erhalten:
(1) 4-[(3-Chlor-4-fluor-phenyl)amino]-6-{1-[(isopropylamino)carbonyl]-piperidin-4-yloxy}-7-methoxy-chinazolin
   Schmelzpunkt: 236°C
   Massenspektrum (ESI⁻): m/z = 486, 488 [M-H]⁻
(2) 4-[(3-Chlor-4-fluor-phenyl)amino]-6-{1-[(phenylamino)carbonyl]-piperidin-4-yloxy}-7-methoxy-chinazolin
   R_{f}-Wert: 0.70 (Kieselgel, Essigester/Methanol/konz. wässriges Ammoniak = 95:5:0.1)
   Massenspektrum (ESI⁺): m/z = 522, 524 [M+H]⁺
(3) 4-[(3-Chlor-4-fluor-phenyl)amino]-6-(cis-4-{N-[(ethylamino)carbonyl]-N-methylamino}-cyclohexan-1-yloxy)-7-methoxy-chinazolin
   R_{f}-Wert: 0.38 (Kieselgel, Methylenchlorid/Methanol = 9:1)
   Massenspektrum (ESI⁺): m/z = 502, 504 [M+H]⁺

### Beispiel 10

### 4-[(3-Chlor-4-fluor-phenyl)amino]-6-{1-[(dimethylamino)carbonylmethyl]-piperidin-4-yloxy}-chinazolin

Hergestellt durch Umsetzung von 4-[(3-Chlor-4-fluor-phenyl)amino]-6-(piperidin-4-yloxy)-chinazolin mit 2-Chlor-N,N-dimethylacetamid in Gegenwart von Kaliumcarbonat in N,N-Dimethylformamid bei Raumtemperatur.
R_{f}-Wert: 0.24 (Kieselgel, Methylenchlorid/Methanol = 9:1)
Massenspektrum (ESI⁺): m/z = 458, 460 [M+H]⁺

Analog Beispiel 10 werden folgende Verbindungen erhalten:
(1) 4-[(3-Chlor-4-fluor-phenyl)amino]-6-{1-[(morpholin-4-yl)carbonylmethyl]-piperidin-4-yloxy}-chinazolin
   R_{f}-Wert: 0.42 (Reversed Phase DC-Fertigplatte (E. Merck), Acetonitril/Wasser/ Trifluoressigsäure = 50:50:1)
   Massenspektrum (ESI⁺): m/z = 500, 502 [M+H]⁺
(2) 4-[(3-Chlor-4-fluor-phenyl)amino]-6-(1-aminocarbonylmethyl-piperidin-4-yloxy)-7-methoxy-chinazolin
   Schmelzpunkt: 251°C
   Massenspektrum (ESI⁺): m/z = 460, 462 [M+H]⁺
(3) 4-[(3-Chlor-4-fluor-phenyl)amino]-6-{1-[(dimethylamino)carbonylmethyl]-piperidin-4-yloxy}-7-methoxy-chinazolin
   Schmelzpunkt: 233°C
   Massenspektrum (ESI⁺): m/z = 488, 490 [M+H]⁺
(4) 4-[(3-Chlor-4-fluor-phenyl)amino]-6-{1-[(morpholin-4-yl)carbonylmethyl]-piperidin-4-yloxy}-7-methoxy-chinazolin
   Schmelzpunkt: 245°C
   Massenspektrum (ESI⁺): m/z = 530, 532 [M+H]⁺
(5) 4-[(3-Chlor-4-fluor-phenyl)amino]-6-[1-(2-methoxyethyl)-piperidin-4-yloxy]-7-methoxy-chinazolin
   Schmelzpunkt: 178°C
   Massenspektrum (ESI⁺): m/z = 461, 463 [M+H]⁺
(6) 4-[(3-Chlor-4-fluor-phenyl)amino]-6-{1-[2-(2-oxopyrrolidin-1-yl)ethyl]-piperidin-4-yloxy}-7-methoxy-chinazolin
   Schmelzpunkt: 234°C
   R_{f}-Wert: 0.28 (Kieselgel, Methylenchlorid/Methanol/konz. wässriges Ammoniak = 90:10:1)
   Massenspektrum (ESI⁺): m/z = 514, 516 [M+H]⁺

### Beispiel 11

### 4-[(3-Chlor-4-fluor-phenyl)amino]-6-{1-[(tetrahydropyran-4-yl)carbonyl]-piperidin-4-yloxy}-7-methoxy-chinazolin

Zu einem Gemisch aus 300 mg 4-[(3-Chlor-4-fluor-phenyl)amino]-6-(piperidin-4-yloxy)-7-methoxy-chinazolin-dihydrochlorid, 82 mg Tetrahydropyran-4-carbonsäure und 0.54 ml Hünigbase in 5 ml N,N-Dimethylformamid werden 90 mg 1-Hydroxy-1H-benzotriazol und 250 mg 2-(1H-Benzotriazol-1-yl)-1,1,3,3-tetramethyluronium-tetrafluoroborat gegeben. Das Reaktionsgemisch wird über Nacht bei Raumtemperatur gerührt. Zur Aufarbeitung wird es mit 25 ml Essigester versetzt und mit Wasser, 10%iger Kaliumcarbonat-Lösung und gesättigter Natriumchlorid-Lösung gewaschen. Die organische Phase wird über Magnesiumsulfat getrocknet und eingeengt. Der Rückstand wird mit wenig Essigester verrührt, abgesaugt und getrocknet.
Ausbeute: 250 mg (77 % der Theorie)
R_{f}-Wert: 0.43 (Kieselgel, Essigester/Methanol/konz. wässriges Ammoniak = 90:10:1)
Massenspektrum (ESI⁺): m/z = 515, 517 [M+H]⁺

Analog Beispiel 11 werden folgende Verbindungen erhalten:
(1) 4-[(3-Chlor-4-fluor-phenyl)amino]-6-{trans-4-[(tetrahydropyran-4-yl)carbonylamino]-cyclohexan-1-yloxy}-7-methoxy-chinazolin
   R_{f}-Wert: 0.44 (Kieselgel, Essigester/Methanol/konz. wässriges Ammoniak = 90:10:1)
   Massenspektrum (ESI⁺): m/z = 529, 531 [M+H]⁺
(2) 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
   R_{f}-Wert: 0.31 (Kieselgel, Essigester/Methanol = 9:1)
   Massenspektrum (ESI⁺): m/z = 543, 545 [M+H]⁺
(3) 4-[(3-Chlor-4-fluor-phenyl)amino]-6-{1-[(R)-(tetrahydrofuran-2-yl)carbonyl]-piperidin-4-yloxy}-7-methoxy-chinazolin
   Schmelzpunkt: 243°C
(4) 4-[(3-Chlor-4-fluor-phenyl)amino]-6-{1-[(S)-(tetrahydrofuran-2-yl)carbonyl]-piperidin-4-yloxy}-7-methoxy-chinazolin
   R_{f}-Wert: 0.34 (Kieselgel, Essigester/Methanol/konz. wässriges Ammoniak = 90:10:1)
(5) 4-[(3-Chlor-4-fluor-phenyl)amino]-6-{1-[(2-methoxyethyl)carbonyl]-piperidin-4-yloxy}-7-methoxy-chinazolin
   R_{f}-Wert: 0.31 (Kieselgel, Methylenchlorid/Methanol/konz. wässriges Ammoniak = 90:10:1)
   Massenspektrum (ESI⁻): m/z = 487, 489 [M-H]⁻

### Beispiel 12

### 4-[(3-Chlor-4-fluor-phenyl)amino]-6-{1-[([1,4]oxazepan-4-yl)carbonyl]-piperidin-4-yloxy}-7-methoxy-chinazolin

Zu 900 mg 1-Methyl-3-[([1,4]oxazepan-4-yl)carbonyl]-3H-imidazol-1-ium-iodid in 10 ml Methylenchlorid werden 4-[(3-Chlor-4-fluor-phenyl)amino]-6-(piperidin-4-yloxy)-7-methoxy-chinazolin-dihydrochlorid und 1.05 ml Triethylamin gegeben. Die gelbliche Suspension wird ca. 24 Stunden bei Raumtemperatur gerührt. Zur Aufarbeitung wird das Reaktionsgemisch mit 50 ml Methylenchlorid versetzt und mit Wasser sowie 10%iger Zitronensäure extrahiert. Die organische Phase wird mit gesättigter Natriumchlorid-Lösung gewaschen, über Magnesiumsulfat getrocknet und eingeengt. Der Rückstand wird über eine Kieselgelsäule mit Methylenchlorid/Methanol/konz. Ammoniak als Laufmittel chromatographiert. Das gewünschte Produkt wird mit Diethylether verrührt, abgesaugt und getrocknet.
Ausbeute: 800 mg (80 % der Theorie)
R_{f}-Wert: 0.30 (Kieselgel, Methylenchlorid/Methanol/konz. wässriges Ammoniak = 90:10:1)
Massenspektrum (ESI⁺): m/z = 530, 532 [M+H]⁺

Analog Beispiel 12 werden folgende Verbindungen erhalten:
(1) 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
   R_{f}-Wert: 0.41 (Kieselgel, Essigester/Methanol/konz. wässriges Ammoniak = 90:10:1)
   Massenspektrum (ESI⁺): m/z = 544, 546 [M+H]⁺
(2) 4-[(3-Chlor-4-fluor-phenyl)amino]-6-{1-[(2-methyl-morpholin-4-yl)carbonyl]-piperidin-4-yloxy}-7-methoxy-chinazolin
   R_{f}-Wert: 0.50 (Kieselgel, Essigester/Methanol/konz. wässriges Ammoniak = 90:10:1)
   Massenspektrum (ESI⁺): m/z = 530, 532 [M+H]⁺
(3) 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
   Schmelzpunkt: 193°C
(4) 4-[(3-Chlor-4-fluor-phenyl)amino]-6-{1-[(N-methyl-N-2-methoxyethyl-amino)carbonyl]-piperidin-4-yloxy}-7-methoxy-chinazolin
   Schmelzpunkt: 171°C
(5) 4-[(3-Chlor-4-fluor-phenyl)amino]-6-{1-[(N-methyl-N-3-methoxypropyl-amino)-carbonyl]-piperidin-4-yloxy}-7-methoxy-chinazolin
   R_{f}-Wert: 0.23 (Kieselgel, Methylenchlorid/Methanol/konz. wässriges Ammoniak = 90:10:1)
   Massenspektrum (ESI⁺): m/z = 532, 534 [M+H]⁺

### Beispiel 13

### 4-[(3-Chlor-4-fluor-phenyl)amino]-6-(1-methyl-piperidin-4-yloxy)-7-ethoxy-chinazolin

Zu 175 mg 4-[(3-Chlor-4-fluor-phenyl)amino]-6-(piperidin-4-yloxy)-7-ethoxy-chinazolin in 1 ml Tetrahydrofuran werden 35 µl 37 %ige wässrige Formalinlösung und 110 mg Natriumtriacetoxyborhydrid gegeben. Das Reaktionsgemisch wird ca. vier Stunden bei Raumtemperatur gerührt. Zur Aufarbeitung werden 5 ml gesättigte Natriumhydrogencarbonat-Lösung zugegeben und das Gemisch wird gründlich durchgerührt. Dann werden 20 ml Essigester zugegeben und die wässrige Phase wird abgetrennt. Die organische Phase wird mit Wasser und gesättigter Natriumchlorid-Lösung gewaschen, über Magnesiumsulfat getrocknet und eingeengt. Der Rückstand wird mit Diisopropylether verrührt, abgesaugt und getrocknet.
Ausbeute: 144 mg (80 % der Theorie)
R_{f}-Wert: 0.80 (Kieselgel, Methylenchlorid/Methanol/konz. wässriges Ammoniak = 60:10:1)
Massenspektrum (ESI⁺): m/z = 431, 433 [M+H]⁺

Analog Beispiel 13 werden folgende Verbindungen erhalten:
(1) 4-[(3-Chlor-4-fluor-phenyl)amino]-6-(1-methyl-piperidin-4-yloxy)-7(2-methoxy-ethoxy)-chinazolin
   R_{f}-Wert: 0.85 (Kieselgel, Methylenchlorid/Methanol/konz. wässriges Ammoniak = 60:10:1)
   Massenspektrum (ESI⁺): m/z = 461, 463 [M+H]⁺
(2) 4-[(3-Ethinyl-phenyl)amino]-6-(1-methyl-piperidin-4-yloxy)-7-methoxy-chinazolin-hydrochlorid
   R_{f}-Wert: 0.26 (Kieselgel, Methylenchlorid/Methanol/konz. wässriges Ammoniak = 90:10:1)
   Massenspektrum (ESI⁺): m/z = 389 [M+H]⁺
(3) 4-[(3-Chlor-4-fluor-phenyl)amino]-6-(trans-4-dimethylamino-cyclohexan-1-yloxy)-7-methoxy-chinazolin
   R_{f}-Wert: 0.80 (Aluminiumoxid, Methylenchlorid/Methanol = 9:1)
   Massenspektrum (ESI⁺): m/z = 445, 447 [M+H]⁺
(4) 4-[(3-Chlor-4-fluor-phenyl)amino]-6-(1-ethyl-piperidin-4-yloxy)-7-methoxy-chinazolin-hydrochlorid
   Durchführung mit Acetaldehyd
   R_{f}-Wert: 0.44 (Reversed Phase DC-Fertigplatte (E. Merck), Acetonitril/Wasser/ Trifluoressigsäure = 50:50:1)
   Massenspektrum (ESI⁺): m/z = 431, 433 [M+H]⁺
(5) 4-{[3-Methyl-4-(pyridin-3-yloxy)-phenyl]amino}-6-(1-methyl-piperidin-4-yloxy)-7-methoxy-chinazolin
   R_{f}-Wert: 0.68 (Kieselgel, Methylenchlorid/Methanol/konz. wässriges Ammoniak = 60:10:1)
   Massenspektrum (ESI⁺): m/z = 472 [M+H]⁺
(6) 4-({3-Chlor-4-[(3-fluor-benzyl)oxy]-phenyl}amino)-6-(1-methyl-piperidin-4-yloxy)-7-methoxy-chinazolin
   R_{f}-Wert: 0.70 (Kieselgel, Methylenchlorid/Methanol/konz. wässriges Ammoniak = 90:10:1)
   Massenspektrum (ESI⁺): m/z = 523, 525 [M+H]⁺

### Beispiel 14

### 4-[(3-Ethinyl-phenyl)amino]-6-[1-(tert.-butyloxycarbonyl)-piperidin-4-yloxy]-7-methoxy-chinazolin

Ein Gemisch aus 3.00 g 4-[(3-Ethinyl-phenyl)amino]-6-hydroxy-7-methoxy-chinazolin, 4.50 g 1-(tert.-Butyloxycarbonyl)-4-(p-toluolsulfonyloxy)-piperidin und 2.90 g Kaliumcarbonat in 30 ml N,N-Dimethylformamid wird zwei Tage bei 60 °C gerührt. Zur Aufarbeitung wird das Gemisch mit 200 ml Essigester versetzt und mit Wasser extrahiert. Die organische Phase wird mit gesättigter Natriumchlorid-Lösung gewaschen, über Magnesiumsulfat getrocknet und eingeengt. Das Rohprodukt wird über eine Kieselgel-Säule mit Methylenchlorid/Methanol/konz. Ammoniak als Laufmittel gereinigt.
Ausbeute: 3.25 g (67 % der Theorie)
R_{f}-Wert: 0.25 (Kieselgel, Methylenchlorid/Methanol/konz. wässriges Ammoniak = 95:5:1)
Massenspektrum (ESI⁺): m/z = 475 [M+H]⁺

Analog Beispiel 14 werden folgende Verbindungen erhalten:
(1) 4-[(3-Ethinyl-phenyl)amino]-6-(tetrahydropyran-4-yloxy]-7-methoxy-chinazolin
   R_{f}-Wert: 0.40 (Kieselgel, Methylenchlorid/Methanol/konz. wässriges Ammoniak = 90:10:1)
   Massenspektrum (ESI⁺): m/z = 376 [M+H]⁺
(2) 4-({3-Chlor-4-[(3-fluor-benzyl)oxy]-phenyl}amino)-6-(tetrahydropyran-4-yloxy)-7-methoxy-chinazolin
   R_{f}-Wert: 0.32 (Kieselgel, Methylenchlorid/Methanol = 20:1)
   Massenspektrum (ESI⁺): m/z = 510, 512 [M+H]⁺
(3) 4-{[3-Methyl-4-(pyridin-3-yloxy)-phenyl]amino}-6-(tetrahydropyran-4-yloxy)-7-methoxy-chinazolin
   R_{f}-Wert: 0.55 (Kieselgel, Methylenchlorid/Methanol = 9:1)
   Massenspektrum (ESI⁺): m/z = 459 [M+H]⁺
(4) 4-{[3-Methyl-4-(pyridin-3-yloxy)-phenyl]amino}-6-(1-tert.-butyloxycarbonyl-piperidin-4-yloxy)-7-methoxy-chinazolin
   R_{f}-Wert: 0.40 (Kieselgel, Methylenchlorid/Methanol = 9:1)
   Massenspektrum (ESI⁺): m/z = 558 [M+H]⁺
(5) 4-({3-Chlor-4-[(3-fluor-benzyl)oxy]-phenyl}amino)-6-(1-tert.-butyloxycarbonyl-piperidin-4-yloxy)-7-methoxy-chinazolin
   R_{f}-Wert: 0.65 (Kieselgel, Methylenchlorid/Methanol = 9:1)
   Massenspektrum (ESI⁺): m/z = 609, 611 [M+H]⁺
(6) 4-{[3-Methyl-4-(pyridin-3-yloxy)-phenyl]amino}-6-[trans-4-(tert.-butyloxycarbonylamino)-cyclohexan-1-yloxy]-7-methoxy-chinazolin
   Das eingesetzte Alkylierungsmittel, cis-4-(tert.-Butyloxycarbonylamino)-1-(4-methylphenylsulfonyloxy)-cyclohexan (Massenspektrum (ESI⁺): m/z = 370 [M+H]⁺), wurde durch Umsetzung von cis-4-(tert.-Butyloxycarbonylamino)-cyclohexanol mit 4-Methylphenyl-sulfonylchlorid in Pyridin hergestellt.
   Massenspektrum (ESI⁺): m/z = 572 [M+H]⁺
(7) 4-({3-Chlor-4-[(3-fluor-benzyl)oxy]-phenyl}amino)-6-[trans-4-(tert.-butyloxycarbonylamino)-cyclohexan-1-yloxy]-7-methoxy-chinazolin
   Das eingesetzte Alkylierungsmittel, cis-4-(tert.-Butyloxycarbonylamino)-1-(4-methylphenylsulfonyloxy)-cyclohexan (Massenspektrum (ESI⁺): m/z = 370 [M+H]⁺), wurde durch Umsetzung von cis-4-(tert.-Butyloxycarbonylamino)-cyclohexanol mit 4-Methylphenyl-sulfonylchlorid in Pyridin hergestellt.
   Massenspektrum (ESI⁺): m/z = 623, 625 [M+H]⁺

### Beispiel 15

### 4-[(3-Chlor-4-fluor-phenyl)amino]-6-{1-[2-(tert.-butyloxycarbonylamino)-ethyl]-piperidin-4-yloxy}-7-methoxy-chinazolin

Ein Gemisch aus 410 mg 4-[(3-Chlor-4-fluor-phenyl)amino]-6-(piperidin-4-yloxy)-7-methoxy-chinazolin-dihydrochlorid, 240 mg N-(tert.-Butyloxycarbonyl)-2-brom-ethylamin und 360 mg Kaliumcarbonat in 5 ml N,N-Dimethylformamid wird über Nacht bei Raumtemperatur gerührt. Dann werden nochmals 80 mg N-(tert.-Butyloxycarbonyl)-2-brom-ethylamin zugesetzt und das Reaktionsgemisch wird weitere vier Stunden bei Raumtemperatur gerührt. Zur Aufarbeitung wird es mit Wasser verdünnt und mit Essigester extrahiert. Die vereinigten organischen Phasen werden mit gesättigter Natriumchloridlösung gewaschen, über Magnesiumsulfat getrocknet und eingeengt. Der Rückstand wird über eine Kieselgelsäule mit Essigester/Methanol (95:5 auf 90:1) als Laufmittel chromatographiert.
Ausbeute: 370 mg (79 % der Theorie)
R_{f}-Wert: 0.33 (Reversed Phase DC-Fertigplatte (E. Merck), Acetonitril/Wasser/ Trifluoressigsäure = 50:50:1)
Massenspektrum (ESI⁻): m/z = 544, 546 [M-H]⁻

Analog Beispiel 15 wird folgende Verbindung erhalten:
(1) 4-[(3-Chlor-4-fluor-phenyl)amino]-6-{1-[2-(tert.-butyloxycarbonylamino)-ethyl]-piperidin-4-yloxy}-chinazolin
   R_{f}-Wert: 0.38 (Reversed Phase DC-Fertigplatte (E. Merck), Acetonitril/Wasser/ Trifluoressigsäure = 50:50:1)
   Massenspektrum (ESI⁺): m/z = 516, 518 [M+H]⁺

### Beispiel 16

### 4-[(3-Chlor-4-fluor-phenyl)amino]-6-{1-[(3-methoxypropyl-amino)-carbonyl]-piperidin-4-yloxy}-7-methoxy-chinazolin

Hergestellt durch Umsetzung von 4-[(3-Chlor-4-fluor-phenyl)amino]-6-{1-[(4-nitrophenyloxy)-carbonyl]-piperidin-4-yloxy}-7-methoxy-chinazolin mit 3-Methoxypropylamin bei 60°C.
R_{f}-Wert: 0.33 (Kieselgel, Methylenchlorid/Methanol/konz. wässriges Ammoniak = 90:10:1)
Massenspektrum (ESI⁺): m/z = 518, 520 [M+H]⁺

Analog Beispiel 16 wird folgende Verbindung erhalten:
(1) 4-[(3-Chlor-4-fluor-phenyl)amino]-6-{1-[(2-methoxyethyl-amino)-carbonyl]-piperidin-4-yloxy}-7-methoxy-chinazolin
   Massenspektrum (ESI⁺): m/z = 504, 506 [M+H]⁺.

Analog den vorstehend genannten Beispielen und anderen literaturbekannten Verfahren können auch folgende Verbindungen hergestellt werden:

| **Beispiel-Nr.** | **Struktur** |
|---|---|
| (1) | 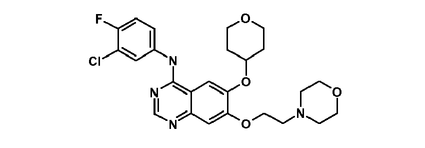 |
| (2) | 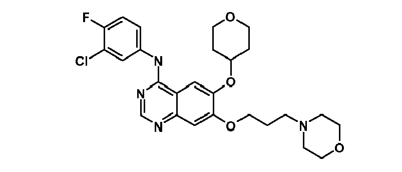 |
| (3) | 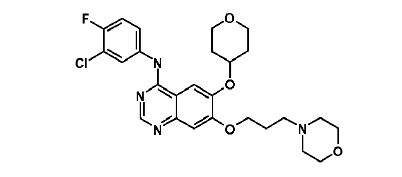 |
| (4) | 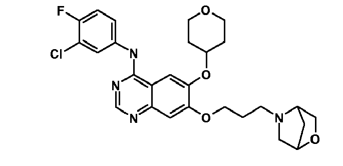 |
| (5) | 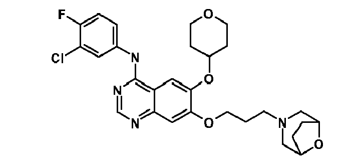 |
| (6) | 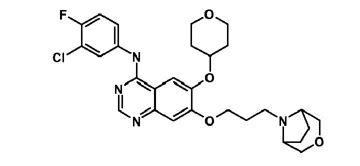 |
| (7) | 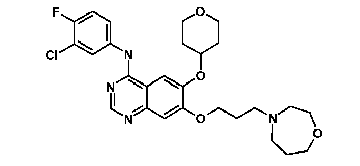 |
| (8) | 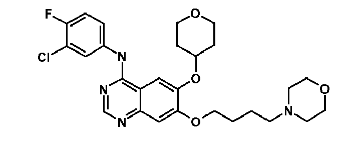 |
| (9) | 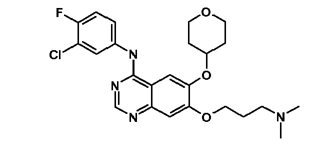 |
| (10) | 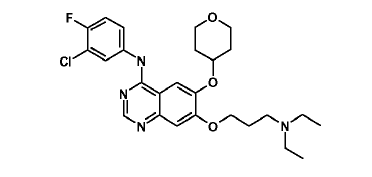 |
| (11) | 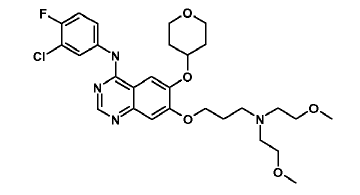 |
| (12) | 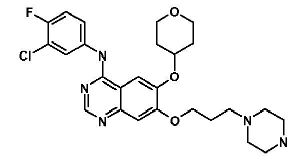 |
| (13) | 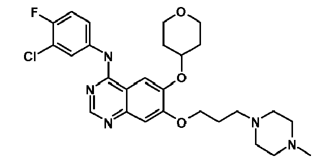 |
| (14) | 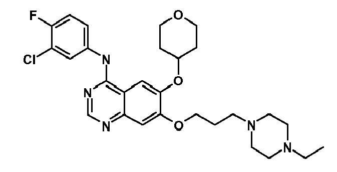 |
| (15) | 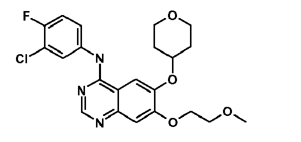 |
| (16) | 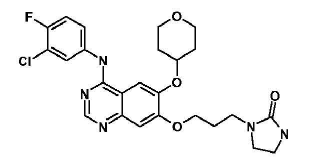 |
| (17) | 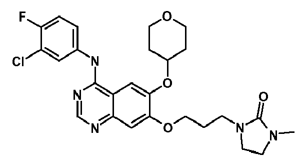 |
| (18) | 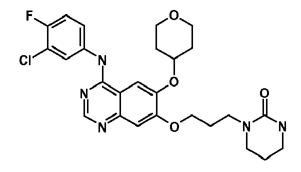 |
| (19) | 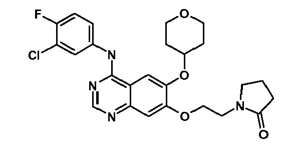 |
| (20) | 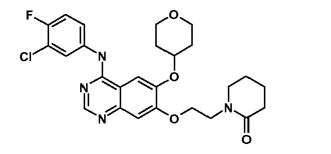 |
| (21) | 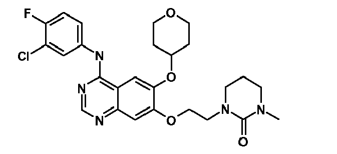 |
| (22) | 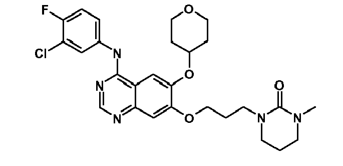 |
| (23) | 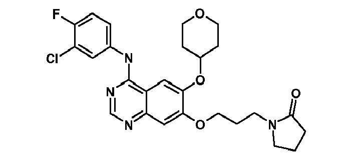 |
| (24) | 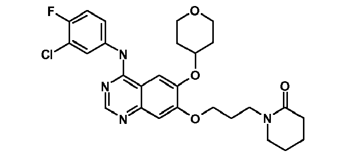 |
| (25) | 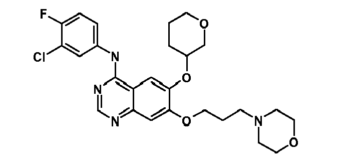 |
| (26) | 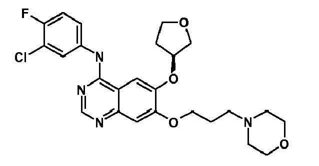 |
| (27) | 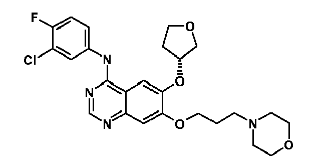 |
| (28) | 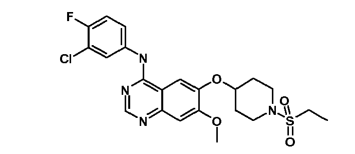 |
| (29) | 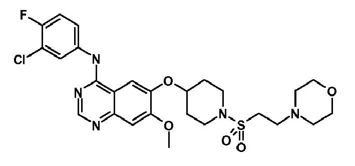 |
| (30) | 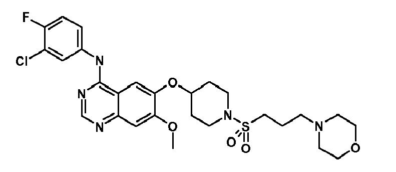 |
| (31) | 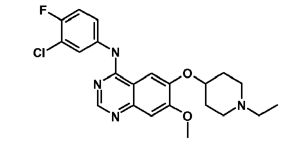 |
| (32) | 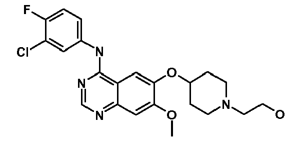 |
| (33) | 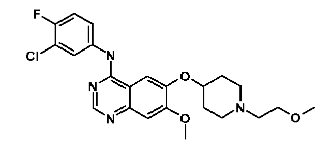 |
| (34) | 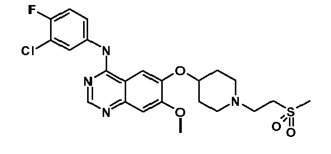 |
| (35) | 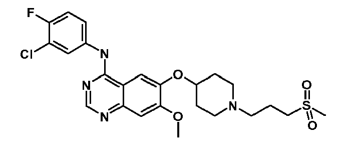 |
| (36) | 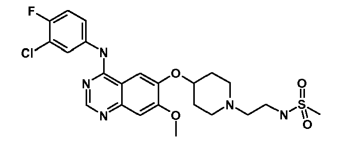 |
| (37) | 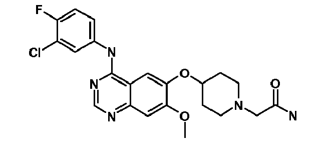 |
| (38) | 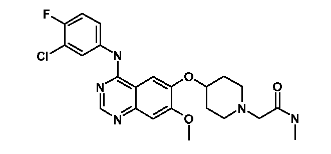 |
| (39) | 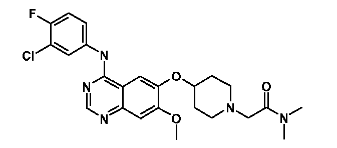 |
| (40) | 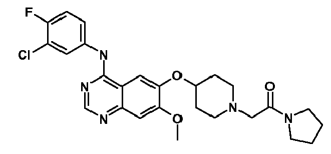 |
| (41) | 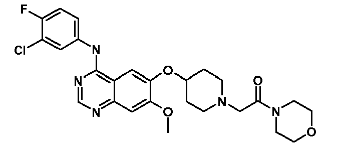 |
| (42) | 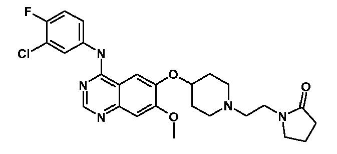 |
| (43) | 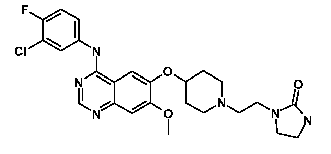 |
| (44) | 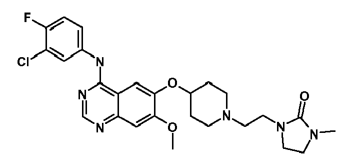 |
| (45) | 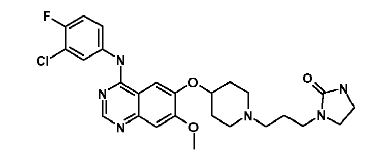 |
| (46) | 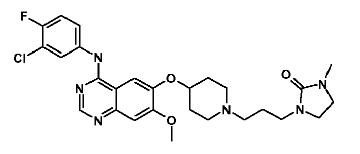 |
| (47) | 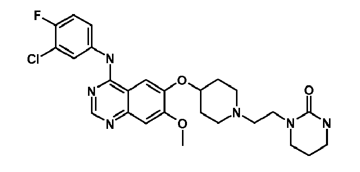 |
| (48) | 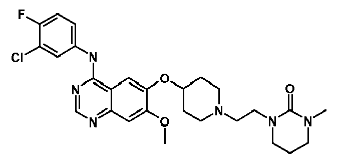 |
| (49) | 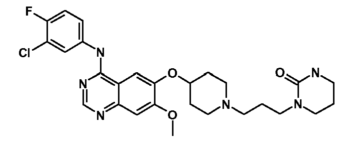 |
| (50) | 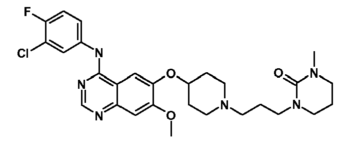 |
| (51) | 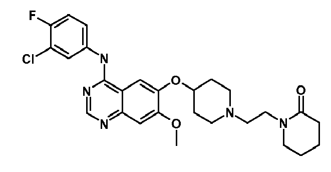 |
| (52) | 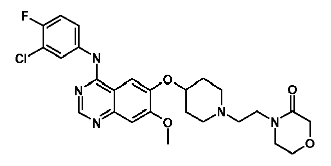 |
| (53) | 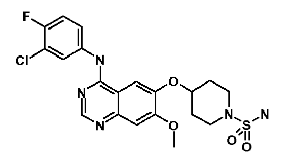 |
| (54) | 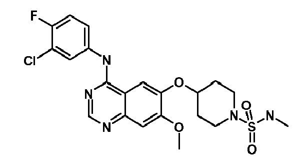 |
| (55) | 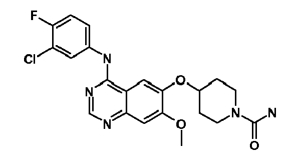 |
| (56) | 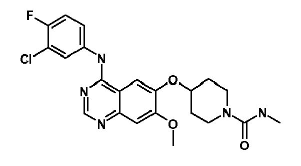 |
| (57) | 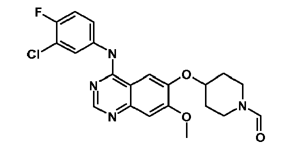 |
| (58) | 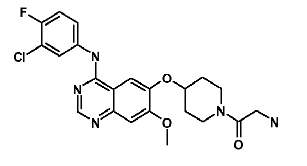 |
| (59) | 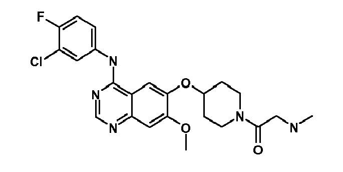 |
| (60) | 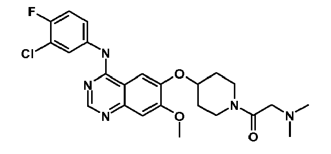 |
| (61) | 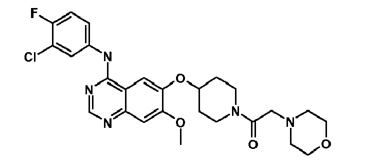 |
| (62) | 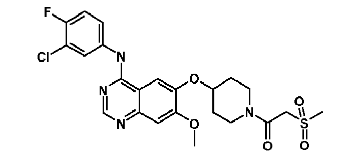 |
| (63) | 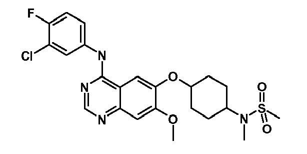 |
| (64) | 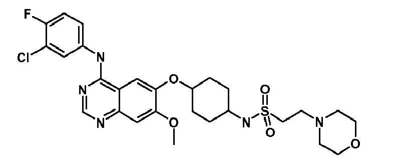 |
| (65) | 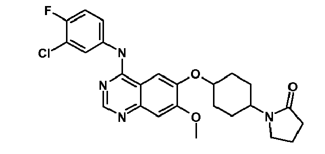 |
| (66) | 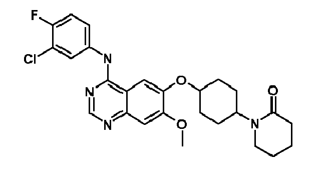 |
| (67) | 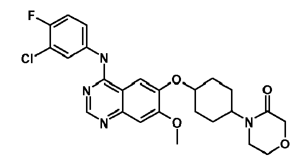 |
| (68) | 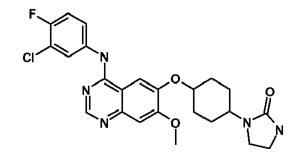 |
| (69) | 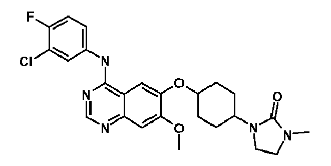 |
| (70) | 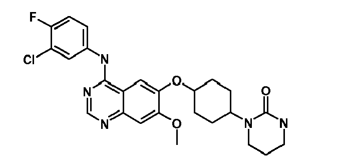 |
| (71) | 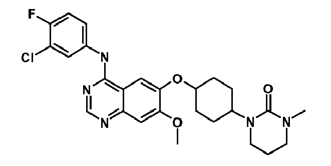 |
| (72) | 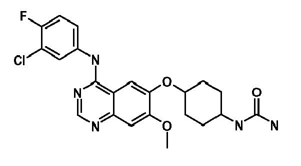 |
| (73) | 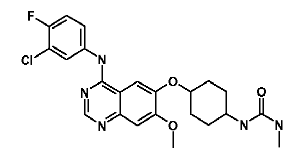 |
| (74) | 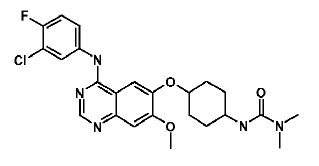 |
| (75) | 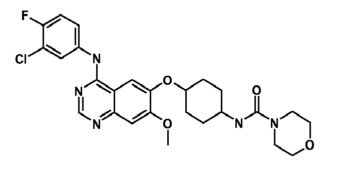 |
| (76) | 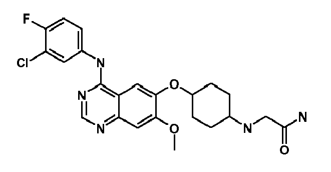 |
| (77) | 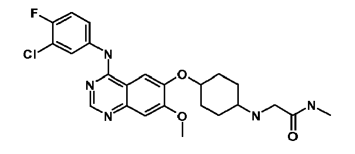 |
| (78) | 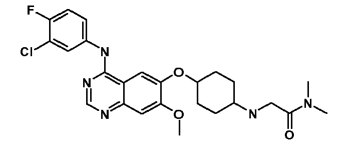 |
| (79) | 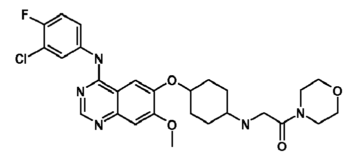 |
| (80) | 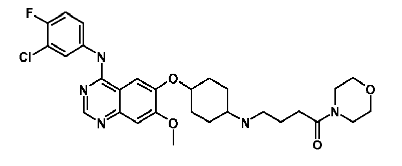 |
| (81) | 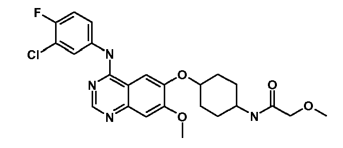 |
| (82) | 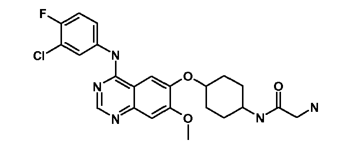 |
| (83) | 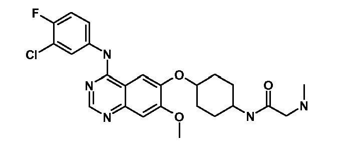 |
| (84) | 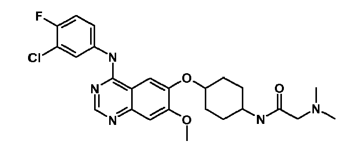 |
| (85) | 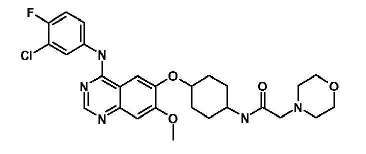 |
| (86) | 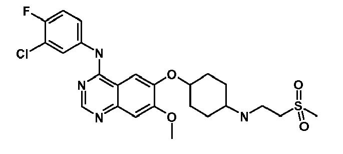 |
| (87) | 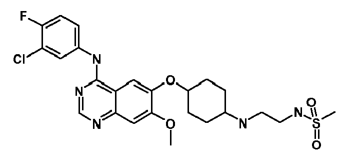 |
| (88) | 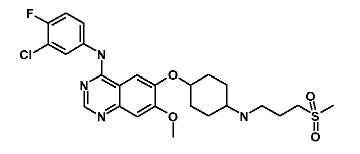 |
| (89) | 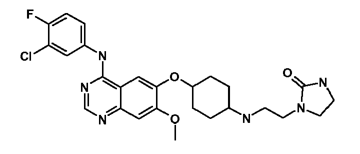 |
| (90) | 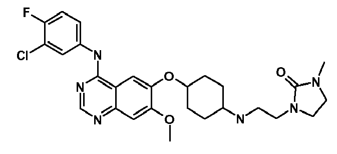 |
| (91) | 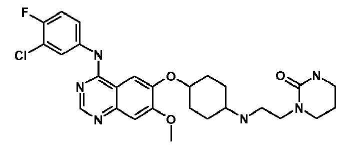 |
| (92) | 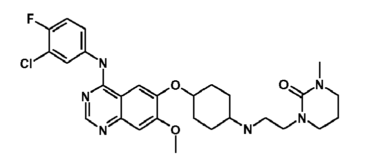 |
| (93) | 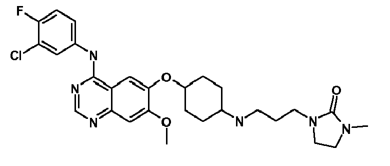 |
| (94) | 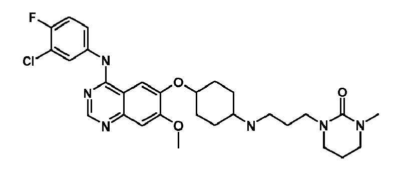 |
| (95) | 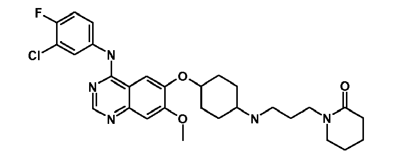 |
| (96) | 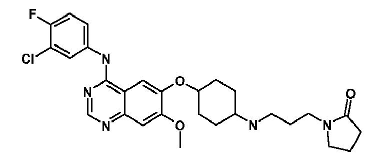 |
| (97) | 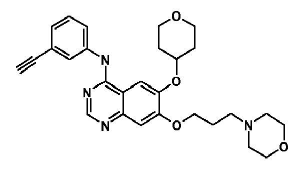 |
| (98) | 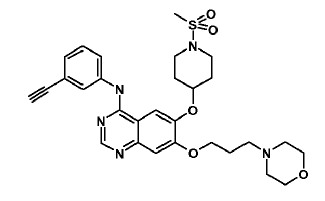 |
| (99) | 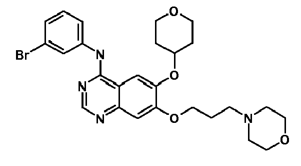 |
| (100) | 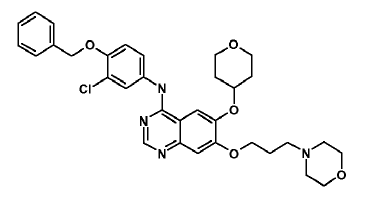 |
| (101) | 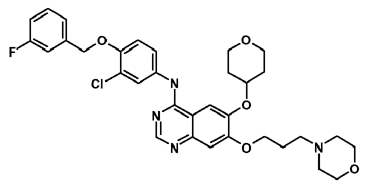 |
| (102) | 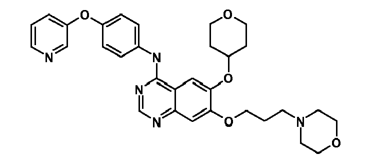 |
| (103) | 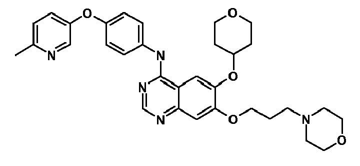 |
| (104) | 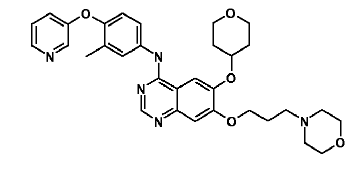 |
| (105) | 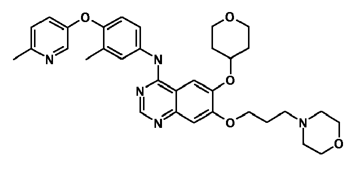 |
| (106) | 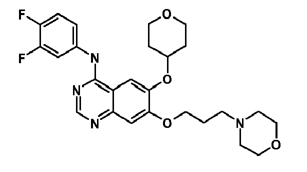 |
| (107) | 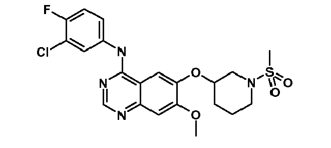 |
| (108) | 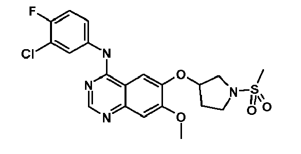 |
| (109) | 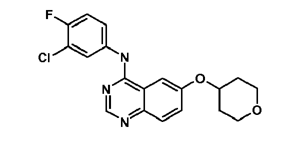 |
| (110) | 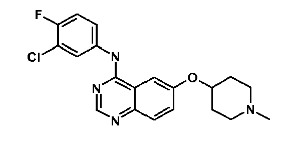 |
| (111) | 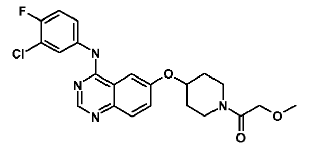 |
| (112) | 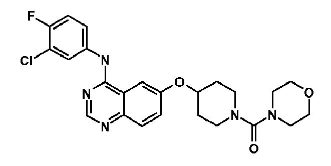 |
| (113) | 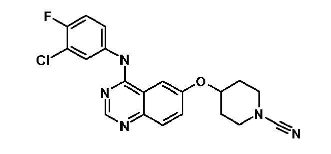 |
| (114) | 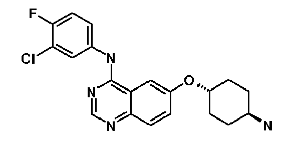 |
| (115) | 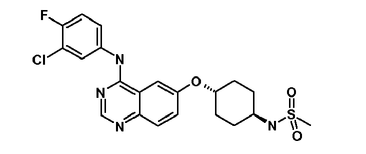 |
| (116) | 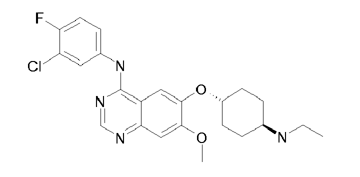 |
| (117) | 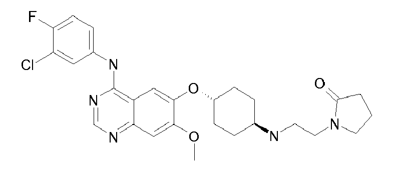 |
| (118) | 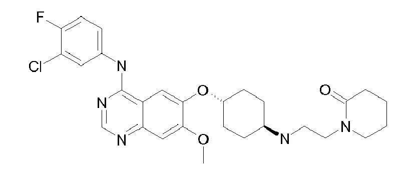 |
| (119) | 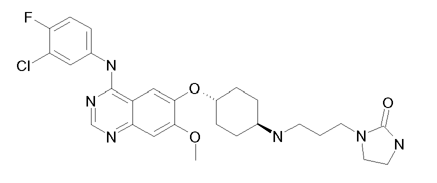 |
| (120) | 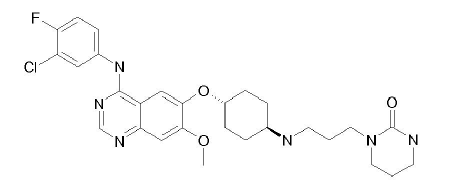 |
| (121) | 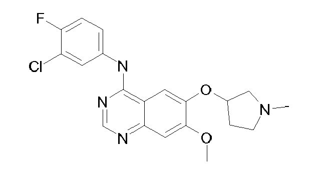 |
| (122) | 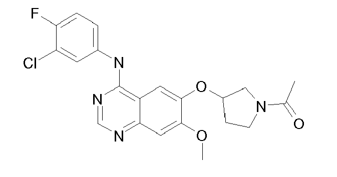 |
| (123) | 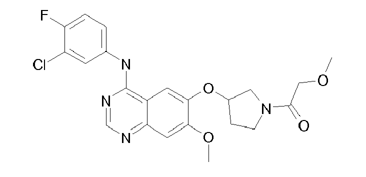 |
| (124) | 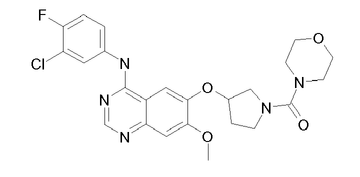 |
| (125) | 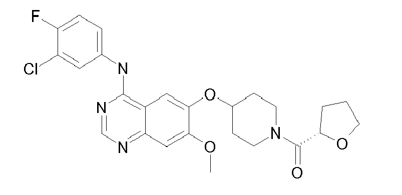 |
| (126) | 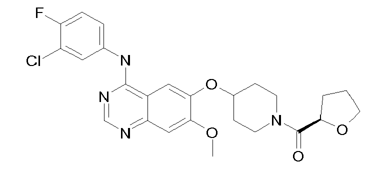 |
| (127) | 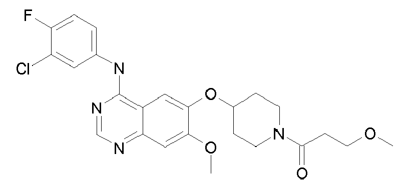 |
| (128) | 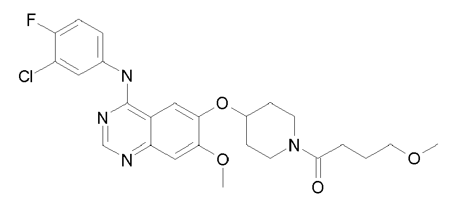 |
| (129) | 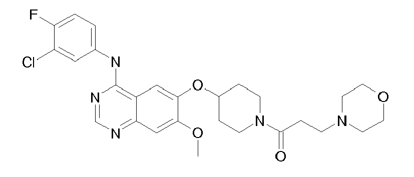 |
| (130) | 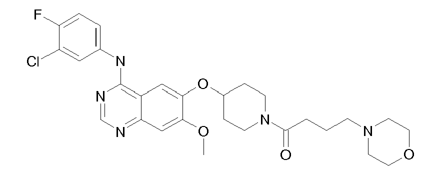 |
| (131) | 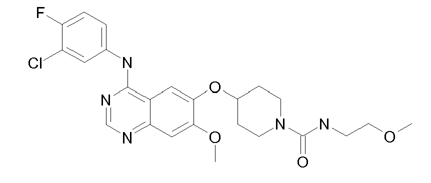 |
| (132) | 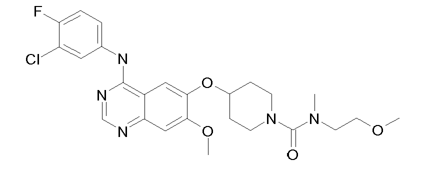 |
| (133) | 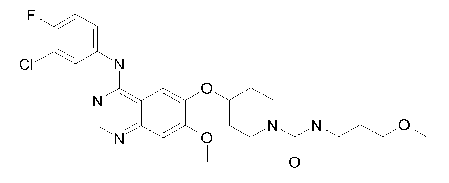 |
| (134) | 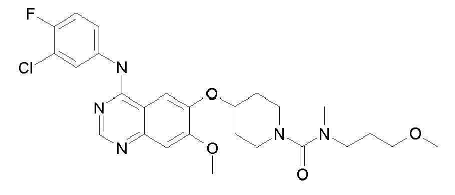 |
| (135) | 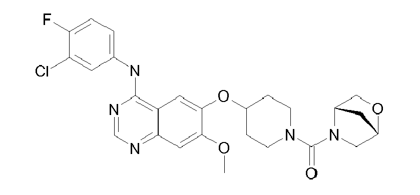 |
| (136) | 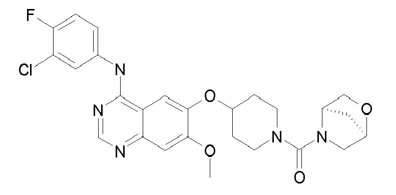 |
| (137) | 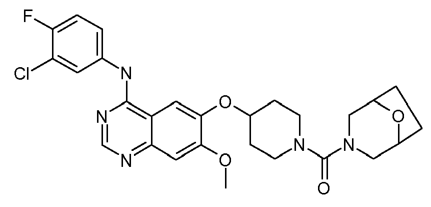 |
| (138) | 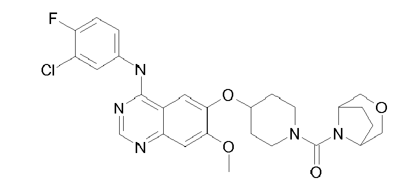 |
| (139) | 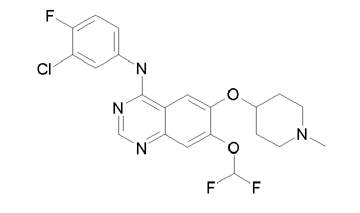 |
| (140) | 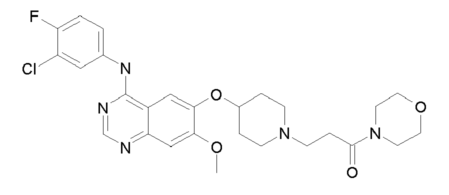 |
| (141) | 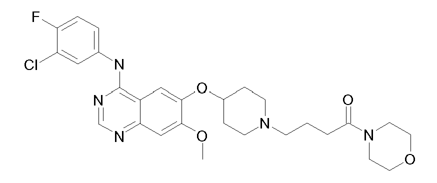 |
| (142) | 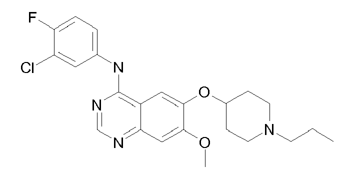 |
| (143) | 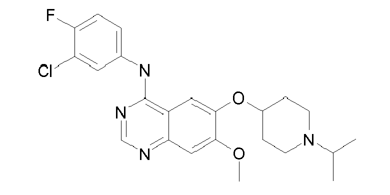 |
| (144) | 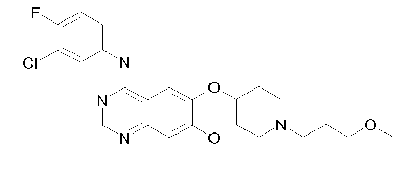 |
| (145) | 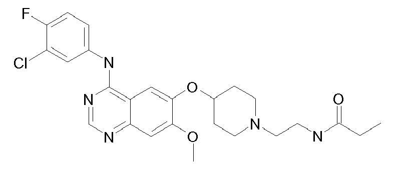 |
| (146) | 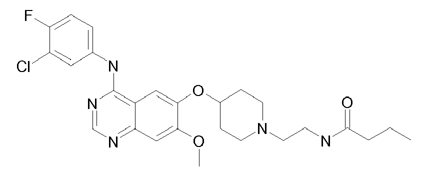 |
| (147) | 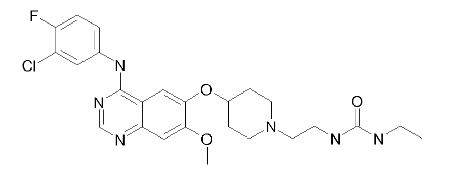 |
| (148) | 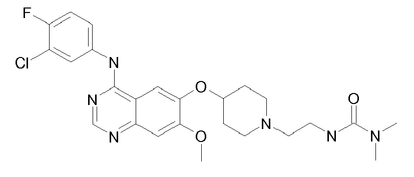 |
| (149) | 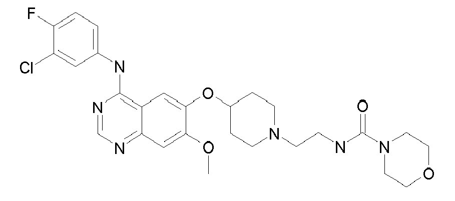 |
| (150) | 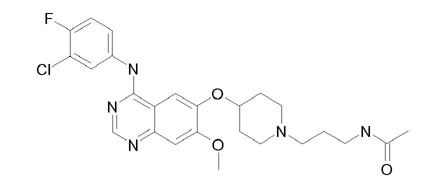 |
| (151) | 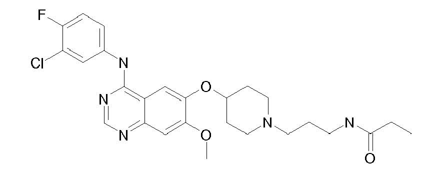 |
| (152) | 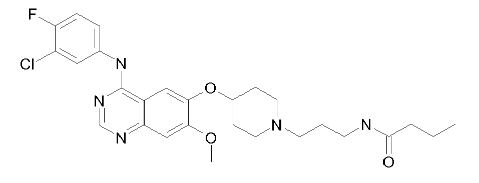 |
| (153) | 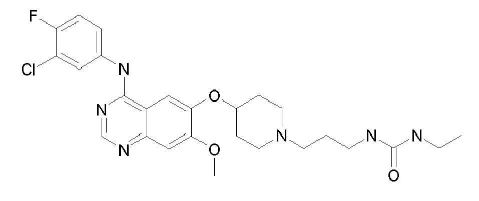 |
| (154) | 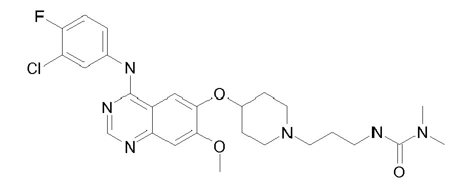 |
| (156) | 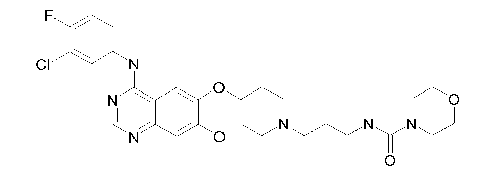 |
| (157) | 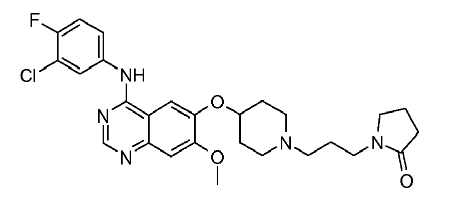 |
| (158) | 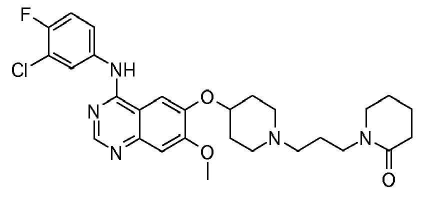 |
| (159) | 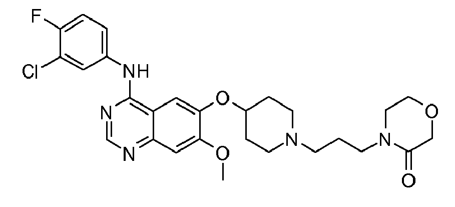 |
| (160) | 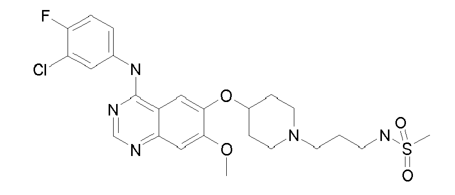 |
| (161) | 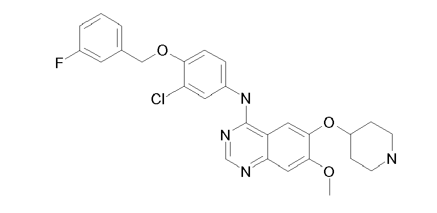 |
| (162) | 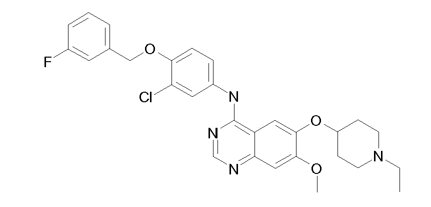 |
| (163) | 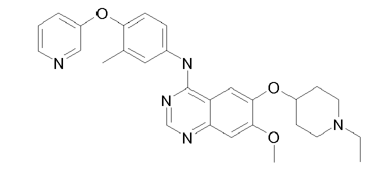 |
| (164) | 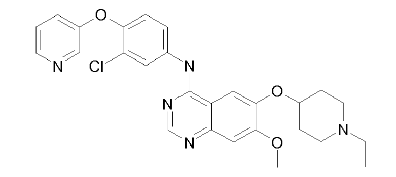 |
| (165) | 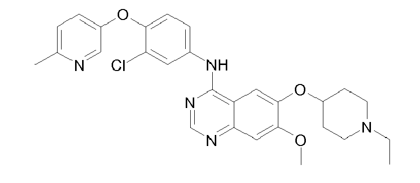 |
| (166) | 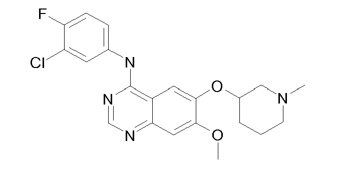 |
| (167) | 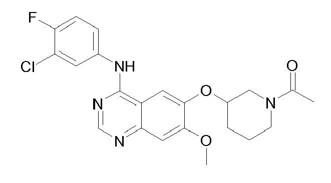 |
| (168) | 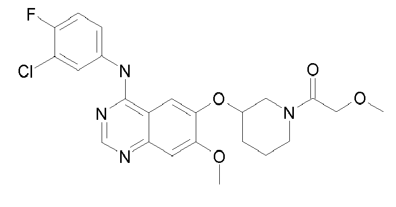 |
| (169) | 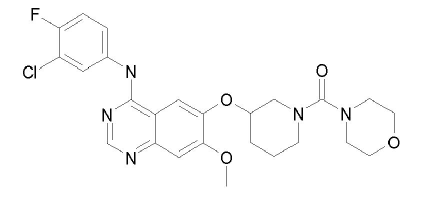 |
| (170) | 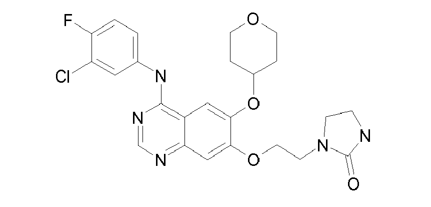 |
| (171) | 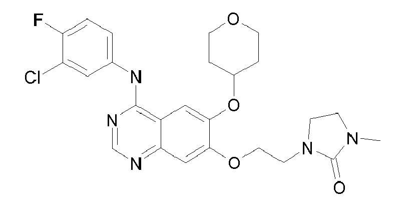 |
| (172) | 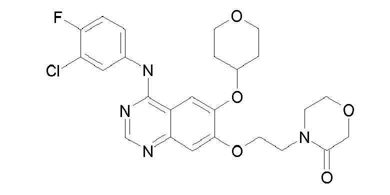 |
| (173) | 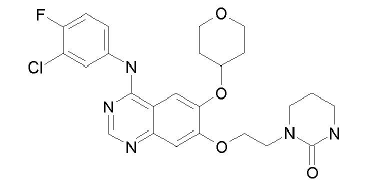 |
| (174) | 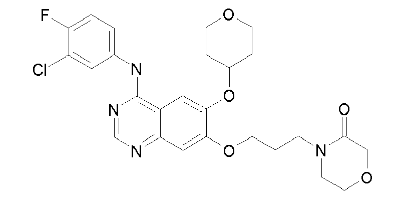 |
| (175) | 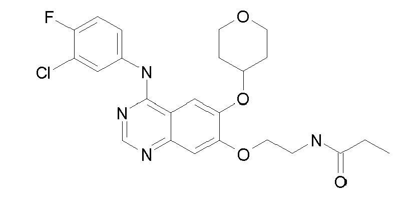 |
| (176) | 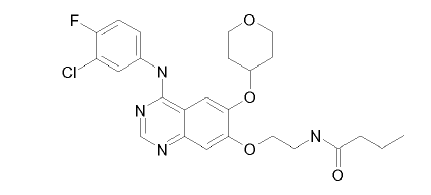 |
| (178) | 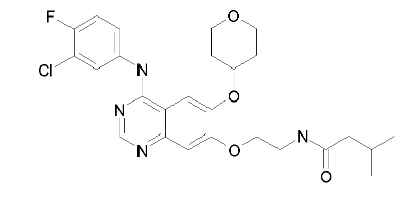 |
| (179) | 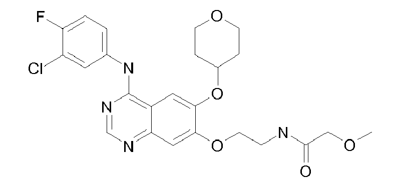 |
| (180) | 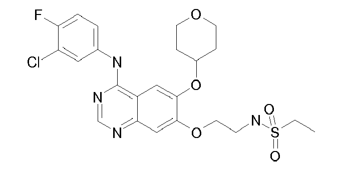 |
| (181) | 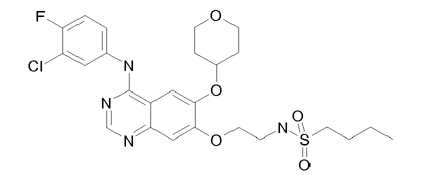 |
| (182) | 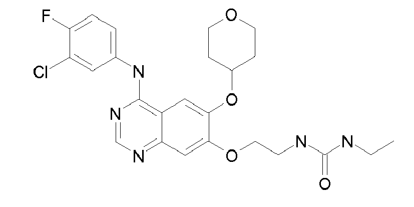 |
| (183) | 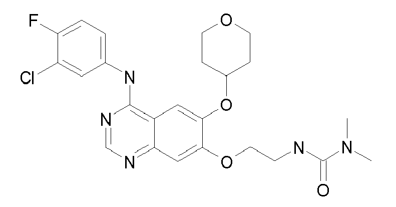 |
| (184) | 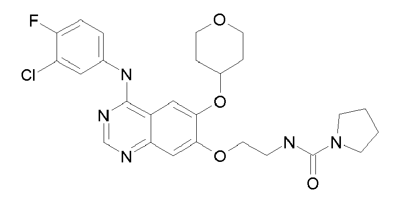 |
| (185) | 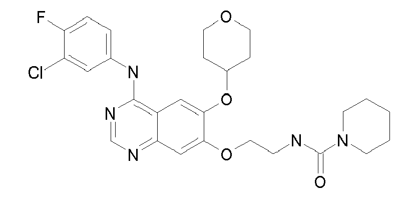 |
| (186) | 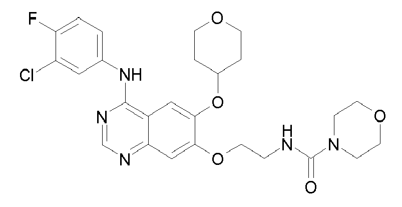 |

### Beispiel 16

### Dragees mit 75 mg Wirksubstanz

### Zusammensetzung:

### 1 Dragéekern enthält:

| | |
|---|---|
| Wirksubstanz | 75.0 mg |
| Calciumphosphat | 93.0 mg |
| Maisstärke | 35.5 mg |
| Polyvinylpyrrolidon | 10.0 mg |
| Hydroxypropylmethylcellulose | 15.0 mg |
| Magnesiumstearat | 1.5 mg |
| | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### Beispiel 17

### Tabletten mit 100 mg Wirksubstanz

### Zusammensetzung:

### 1 Tablette enthält:

| | |
|---|---|
| Wirksubstanz | 100.0 mg |
| Milchzucker | 80.0 mg |
| Maisstärke | 34.0 mg |
| Polyvinylpyrrolidon | 4.0 mg |
| Magnesiumstearat | 2.0 mg |
| | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette |
| | | und einseitiger Teilkerbe. |

### Beispiel 18

### Tabletten mit 150 mg Wirksubstanz

### Zusammensetzung:

### 1 Tablette enthält:

| | |
|---|---|
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 19

### Hartgelatine-Kapseln mit 150 mg Wirksubstanz

### Zusammensetzung:

### 1 Kapsel enthält:

| | |
|---|---|
| Wirkstoff | 150.0 mg |
| Maisstärke getr. ca. | 180.0 mg |
| Milchzucker pulv. ca. | 87.0 mg |
| Magnesiumstearat | 3.0 mg |
| ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 20

### Suppositorien mit 150 mg Wirksubstanz

### Zusammensetzung:

### 1 Zäpfchen enthält:

| | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel 21

### Suspension mit 50 mg Wirksubstanz

### Zusammensetzung:

### 100 ml Suspension enthalten:

| | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest.ad 100.00 ml | |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 22

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel 23

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCI gelöst, mit Kochsalz isotonisch gestellt, sterilfiltriert und in 10 ml Ampullen abgefüllt.

### Beispiel 24

### Kapseln zur Pulverinhalation mit 5 mg Wirksubstanz

1 Kapsel enthält:

| | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### Beispiel 25

### Inhalationslösung für Handvernebler mit 2.5 mg Wirksubstanz

1 Hub enthält:

| | |
|---|---|
| Wirksubstanz | 2.500 mg |
| Benzalkoniumchlorid | 0.001 mg |
| 1N-Salzsäure q.s. | |
| Ethanol/Wasser (50/50) ad | 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

1. Bicyclische Heterocyclen der allgemeinen Formel 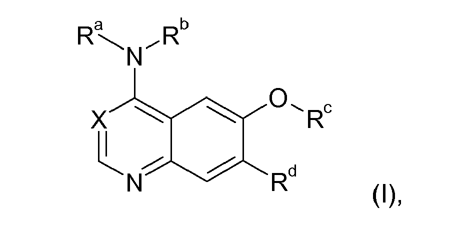 in denen
R^{a} ein Wasserstoffatom oder eine C₁-₄-Alkylgruppe,
R^{b} eine Phenyl- oder 1-Phenylethylgruppe, in denen der Phenylkern jeweils durch die Reste R¹ bis R³ substituiert ist, wobei
R¹ und R², die gleich oder verschieden sein können, jeweils ein Wasserstoff-, Fluor-, Chlor-, Brom- oder Jodatom,
eine C₁₋₄-Alkyl-, Hydroxy-, C₁₋₄-Alkoxy-, C₂₋₃-Alkenyl- oder C₂₋₃-Alkinylgruppe, eine Aryl-, Aryloxy-, Arylmethyl- oder Arylmethoxygruppe,
eine Heteroaryl-, Heteroaryloxy-, Heteroarylmethyl- oder Heteroarylmethoxygruppe,
eine durch 1 bis 3 Fluoratome substituierte Methyl- oder Methoxygruppe oder eine Cyano-, Nitro- oder Aminogruppe, und
R³ ein Wasserstoff-, Fluor-, Chlor- oder Bromatom,
eine Methyl- oder Trifluormethylgruppe darstellen,
R^{c} eine Cyclobutyl-, Cyclopentyl- oder Cyclohexylgruppe, die jeweils durch eine Gruppe R⁴-N-R⁵ substituiert ist, wobei
R⁴ ein Wasserstoffatom oder eine C₁₋₃-Alkylgruppe und
R⁵ ein Wasserstoffatom oder eine C₁₋₃-Alkylgruppe,
eine Aminocarbonyl-C₁₋₃-alkyl-, C₁₋₃-Alkylaminocarbonyl-C₁₋₃-alkyl-, Di-(C₁₋₃-alkyl)aminocarbonyl-C₁₋₃-alkyl-, Pyrrolidin-1-ylcarbonyl-C₁₋₃-alkyl-, Piperidin-1-ylcarbonyl-C₁₋₃-alkyl-, Homopiperidin-1-ylcarbonyl-C₁₋₃-alkyl-, Morpholin-4-yl-carbonyl-C₁₋₃-alkyl-, Homomorpholin-4-ylcarbonyl-C₁₋₃-alkyl-, Piperazin-1-yl-carbonyl-C₁₋₃-alkyl-, 4-C₁₋₃-Alkyl-piperazin-1-ylcarbonyl-C₁₋₃-alkyl-, Homo-piperazin-1-ylcarbonyl-C₁₋₃-alkyl- oder eine 4-C₁₋₃-Alkyl-homopiperazin-1-yl-carbonyl-C₁₋₃-alkylgruppe,
eine Hydroxy-C₂₋₄-alkyl-, C₁₋₃-Alkyloxy-C₂₋₄-alkyl-, C₁₋₄-Alkyloxy-carbonylamino-C₂₋₄-alkyl-, Amino-C₂₋₄-alkyl-, C₁₋₃-Alkylamino-C₂₋₄-alkyl-, Di-(C₁₋₃-alkyl)amino-C₂₋₄-alkyl-, C₁₋₃-Alkylcarbonylamino-C₂₋₄-alkyl-, Aminocarbonylamino-C₂₋₄-alkyl-, C₁₋₃-Alkylaminocarbonylamino-C₂₋₄-alkyl-, D i-(C₁₋₃-alkyl)amino-carbonylamino-C₂₋₄-alkyl-, Pyrrolidin-1-ylcarbonylamino-C₂₋₄-alkyl-, Piperidin-1-ylcarbonylamino-C₂₋₄-alkyl-, Morpholin-4-ylcarbonylamino-C₂₋₄-alkyl-, C₁₋₃-Alkylsulfonyl-C₂₋₄-alkyl- oder eine C₁₋₃-Alkylsulfonylamino-C₂₋₄-alkylgruppe,
eine (2-Oxo-pyrrolidin-1-yl)-C₂₋₄-alkyl-, (2-Oxopiperidin-1-yl)-C₂₋₄-alkyl-, (3-Oxo-morpholin-4-yl)-C₂₋₄-alkyl-, (2-Oxo-imidazolidin-1-yl)-C₂₋₄-alkyl-, (2-Oxo-3-C₁₋₃-alkyl-imidazolidin-1-yl)-C₂₋₄-alkyl-, (2-Oxo-hexahydropyrimidin-1-yl)-C₂₋₄-alkyl- oder eine (2-Oxo-3-C₁₋₃-alkyl-hexahydropyrimidin-1-yl)-C₂₋₄-alkylgruppe,
eine C₁₋₄-Alkylsulfonyl-, Chlor-C₁₋₄-alkylsulfonyl-, Brom-C₁₋₄-alkylsulfonyl-, Amino-C₁₋₄-alkylsulfonyl-, C₁₋₃-Alkylamino-C₁₋₄-alkylsulfonyl-, Di-(C₁₋₃-alkyl)amino-C₁₋₄-alkylsulfonyl-, (Pyrrolidin-1-yl)-C₁₋₄-alkylsulfonyl-, (Piperidin-1-yl)-C₁₋₄-alkylsulfonyl-, (Homopiperidin-1-yl)-C₁₋₄-alkylsulfonyl-, (Morpholin-4-yl)-C₁₋₄-alkylsulfonyl-, (Homomorpholin-4-yl)-C₁₋₄-alkylsulfonyl-, (Piperazin-1-yl)-C₁₋₄-alkylsulfonyl-, (4-C₁₋₃-Alkyl-piperazin-1-yl)-C₁₋₄-alkylsulfonyl-, (Homopiperazin-1-yl)-C₁₋₄-alkylsulfonyl- oder eine (4-C₁₋₃-Alkyl-homopiperazin-1-yl)-C₁₋₄-alkylsulfonylgruppe,
eine C₁₋₄-Alkyloxycarbonylgruppe,
eine Formyl-, C₁₋₄-Alkyl-carbonyl-, C₁₋₃-Alkyloxy-C₁₋₄-alkyl-carbonyl-, Tetrahydrofuranylcarbonyl-, Tetrahydropyranylcarbonyl-, Amino-C₁₋₄-alkylcarbonyl-, C₁₋₃-Alkylamino-C₁₋₄-alkyl-carbonyl-, Di-(C₁₋₃-alkyl)amino-C₁₋₄-alkylcarbonyl-, Pyrrolidin-1-yl-C₁₋₄-alkyl-carbonyl-, Piperidin-1-yl-C₁₋₄-alkyl-carbonyl-, (Homopiperidin-1-yl)-C₁₋₄-alkyl-carbonyl-, Morpholin-4-yl-C₁₋₄-alkyl-carbonyl-, (Homomorpholin-4-yl)-C₁₋₄-alkyl-carbonyl-, (Piperazin-1-yl)-C₁₋₄-alkyl-carbonyl-, (4-C₁₋₃-Alkyl-piperazin-1-yl)-C₁₋₄-alkyl-carbonyl-, (Homopiperazin-1-yl)-C₁₋₄-alkylcarbonyl-, ( 4-C₁₋₃-Alkyl-homopiperazin-1-yl)-C₁₋₄-alkyl-carbonyl- oder eine C₁₋₃-Alkylsulfonyl-C₁₋₄-alkyl-carbonylgruppe, oder
eine Cyano-, Aminocarbonyl-, C₁₋₃-Alkyl-aminocarbonyl-, Di-(C₁₋₃-alkyl)aminocarbonyl-, (C₁₋₃-Alkyloxy-C₂₋₄-alkyl)aminocarbonyl-, N-(C₁₋₃-Alkyl)-N-(C₁₋₃-alkyloxy-C₂₋₄-alkyl)aminocarbonyl-, Arylaminocarbonyl-, Pyrrolidin-1-ylcarbonyl-, Piperidin-1-ylcarbonyl-, Homopiperidin-1-ylcarbonyl-, Morpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1 ]oct-8-ylcarbonyl-, 8-Oxa-3-aza-bicyclo[3.2.1 ]oct-3-ylcarbonyl-, Piperazin-1-ylcarbonyl-, 4-C₁₋₃-Alkyl-piperazin-1-ylcarbonyl-, Homopiperazin-1-ylcarbonyl-, 4-C₁₋₃-Alkyl-homopiperazin-1-ylcarbonyl-, Aminosulfonyl-, C₁₋₃-Alkyl-aminosulfonyl-, D i-(C₁₋₃-alkyl)amino-sulfonyl-, Pyrrolidin-1-yl-sulfonyl-, Piperidin-1-ylsulfonyl-, Homopiperidin-1-ylsulfonyl-, Morpholin-4-ylsulfonyl-, Homomorpholin-4-ylsulfonyl-, Piperazin-1-ylsulfonyl-, 4-C₁₋₃-Alkyl-piperazin-1-ylsulfonyl-, Homopiperazin-1-ylsulfonyl- oder eine 4-C₁₋₃-Alkyl-homopiperazin-1-ylsulfonylgruppe darstellen,
eine Cyclobutyl-, Cyclopentyl- oder Cyclohexylgruppe, die jeweils durch eine Gruppe R⁶ substituiert ist, wobei
R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-C₁₋₃-alkyl-imidazolidin-1-yl-, 2-Oxo-hexahydro-pyrimidin-1-yl- oder eine 2-Oxo-3-C₁₋₃-alkyl-hexahydropyrimidin-1-ylgruppe darstellt,
eine Azetidin-3-ylgruppe, die in 1-Stellung durch den Rest R⁵ substituiert ist, wobei R⁵ wie vorstehend erwähnt definiert ist,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist,
oder eine Tetrahydrofuran-3-yl-, Tetrahydropyran-3-yl- oder Tetrahydropyran-4-yl-gruppe,
R^{d} ein Wasserstoffatom oder ein Fluor-, Chlor- oder Bromatom,
eine Hydroxygruppe,
eine C₁₋₄-Alkyloxygruppe,
eine durch 1 bis 3 Fluoratome substituierte Methoxygruppe,
eine durch 1 bis 5 Fluoratome substituierte Ethyloxygruppe,
eine C₂₋₄-Alkyloxygruppe, die durch einen Rest R⁶ oder R⁷ substituiert ist, wobei
R⁶ wie vorstehend erwähnt definiert ist und
R⁷ eine Hydroxy-, C₁₋₃-Alkyloxy-, C₃₋₆-Cycloalkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Homopiperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo-[3.2.1]oct-3-yl-, Piperazin-1-yl-, 4-C₁₋₃-Alkyl-piperazin-1-yl-, Homopiperazin-1-yl- oder C₁₋₃-Alkyl-homopiperazin-1-ylgruppe, oder
eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino- Morpholin-4-ylcarbonylamino- oder eine C₁₋₄-Alkylsulfonylamino-Gruppe darstellt,
eine C₃₋₇Cycloalkyloxy- oder C₃₋₇-Cycloalkyl-C₁₋₄-alkyloxygruppe,
eine Tetrahydrofuran-3-yloxy-, Tetrahydropyran-3-yloxy- oder Tetrahydropyran-4-yloxy-gruppe,
eine Tetrahydrofuranyl-C₁₋₄-alkyloxy- oder Tetrahydropyranyl-C₁₋₄-alkyloxygruppe,
eine C₁₋₄-Alkoxygruppe, die durch eine in 1-Stellung durch den Rest R⁸ substituierte Pyrrolidinyl-, Piperidinyl- oder Homopiperidinylgruppe substituiert ist, wobei
R⁸ ein Wasserstoffatom oder eine C₁₋₃-Alkylgruppe darstellt,
oder eine C₁₋₄-Alkoxygruppe, die durch eine in 4-Stellung durch den Rest R⁸ substituierte Morpholinylgruppe substituiert ist, wobei R⁸ wie vorstehend erwähnt definiert ist, und
X eine durch eine Cyanogruppe substituierte Methingruppe oder ein Stickstoffatom bedeuten,
wobei unter den bei der Definition der vorstehend genannten Reste erwähnten Arylgruppen jeweils eine Phenylgruppe zu verstehen ist, die durch R⁹ mono- oder disubstituiert ist, wobei die Substituenten gleich oder verschieden sein können und
R⁹ ein Wasserstoffatom, ein Fluor-, Chlor-, Brom- oder lodatom oder eine C₁₋₃-Alkyl-, Hydroxy-, C₁₋₃-Alkyloxy-, Difluormethyl- Trifluormethyl-, Difluormethoxy-, Trifluormethoxy- oder Cyanogruppe darstellt,
unter den bei der Definition der vorstehend genannten Reste erwähnten Heteroarylgruppen eine Pyridyl-, Pyridazinyl-, Pyrimidinyl- oder Pyrazinylgruppe zu verstehen ist, wobei die vorstehend erwähnten Heteroarylgruppen jeweils durch den Rest R⁹ mono- oder disubstituiert sind, wobei die Substituenten gleich oder verschieden sein können und R⁹ wie vorstehend erwähnt definiert ist, und
die vorstehend erwähnten Pyrrolidinyl-, Piperidinyl-, Piperazinyl- und Morpholinylgruppen jeweils durch eine oder zwei C₁₋₃-Alkylgruppen substituiert sein können, und
soweit nichts anderes erwähnt wurde, die vorstehend erwähnten Alkylgruppen geradkettig oder verzweigt sein können,
mit der Maßgabe, daß die Verbindung 4-[(3-Chlor-4-fluor-phenyl)amino]-6-((S)-tetrahydrofuran-3-yloxy)-7-hydroxy-chinazolin ausgeschlossen ist,
deren Tautomere, deren Stereoisomere, deren Gemische und deren Salze.

2. Bicyclische Heterocyclen der allgemeinen Formel I gemäß Anspruch 1, in denen
R^{a} ein Wasserstoffatom,
R^{b} eine durch die Reste R¹ bis R³ substituierte Phenylgruppe, wobei
R¹ ein Wasserstoff-, Fluor-, Chlor- oder Bromatom,
eine Methyl-, Trifluormethyl- oder Ethinylgruppe,
eine Phenyloxy- oder Phenylmethoxygruppe, wobei der Phenylteil der vorstehend erwähnten Gruppen gegebenenfalls durch ein Fluor- oder Chloratom substituiert ist, oder
eine Pyridyloxy- oder Pyridinylmethoxygruppe, wobei der Pyridinylteil der vorstehend erwähnten Gruppen gegebenenfalls durch eine Methyl- oder Trifluormethylgruppe substituiert ist,
R² ein Wasserstoff-, Fluor- oder Chloratom oder eine Methylgruppe und
R³ ein Wasserstoffatom darstellen,
R^{c} eine Cyclopentylgruppe, die in 3-Stellung durch eine Gruppe R⁴-N-R⁵ substituiert ist, wobei
R⁴ ein Wasserstoffatom oder eine C₁₋₃-Alkylgruppe und
R⁵ ein Wasserstoffatom oder eine C₁₋₃-Alkylgruppe,
eine Aminocarbonyl-C₁₋₃-alkyl-, C₁₋₃-Alkylaminocarbonyl-C₁₋₃-alkyl-, Di-(C₁₋₃-alkyl)aminocarbonyl-C₁-₃-alkyl-, Pyrrolidin-1-ylcarbonyl-C₁-₃-alkyl-, Piperidin-1-yl-carbonyl-C₁₋₃-alkyl-, Piperazin-1-ylcarbonyl-C₁₋₃-alkyl-, 4-C₁₋₃-Alkyl-piperazin-1-yl-carbonyl-C₁₋₃-alkyl- oder Morpholin-4-ylcarbonyl-C₁₋₃-alkylgruppe,
eine Hydroxy-C₂₋₄-alkyl-, C₁₋₃-Alkyloxy-C₂₋₄-alkyl-, C₁₋₄-Alkyloxy-carbonylamino-C₂₋₄-alkyl-, Amino-C₂₋₄-al kyl-, C₁₋₃-Alkylamino-C₂₋₄-alkyl-, Di-(C₁₋₃-alkyl)amino-C₂₋₄-alkyl-, C₁₋₃-Alkylcarbonylamino-C₂₋₄-alkyl-, Aminocarbonylamino-C₂₋₄-alkyl-, C₁₋₃-Alkylaminocarbonylamino-C₂₋₄-alkyl-, Di-(C₁₋₃-alkyl)amino-carbonylamino-C₂₋₄-alkyl-, Morpholin-4-ylcarbonylamino-C₂₋₄-alkyl-, C₁₋₃-Alkylsulfonyl-C₂₋₄-alkyl - oder C₁₋₃-Alkylsulfonylamino-C₂₋₄-alkylgruppe,
eine (2-Oxo-pyrrolidin-1-yl)-C₂₋₄-alkyl-, (2-Oxopiperidin-1-yl)-C₂₋₄-alkyl-, (3-Oxo-morpholin-4-yl)-C₂₋₄-alkyl-, (2-Oxo-imidazolidin-1-yl)-C₂₋₄-alkyl-, (2-Oxo-3-methyl-imidazolidin-1-yl)-C₂₋₄-alkyl-, (2-Oxo-hexahydropyrimidin-1-yl)-C₂₋₄-alkyl- oder (2-Oxo-3-methyl-hexahydropyrimidin-1-yl)-C₂₋₄-alkylgruppe,
eine C₁₋₃-Alkylsulfonyl-, Chlor-C₂₋₄-alkylsulfonyl-, Brom-C₂₋₄-alkylsulfonyl-, Amino-C₂₋₄-alkylsulfonyl-, C₁₋₃-Alkylamino-C₂₋₄-alkylsulfonyl-, Di-(C₁₋₃-alkyl)amino-C₂₋₄-alkylsulfonyl-, (Pyrrolidin-1-yl)-C₂₋₄-alkylsulfonyl-, (Piperidin-1-yl)-C₂₋₄-alkylsulfonyl- oder (Morpholin-4-yl)-C₂₋₄-alkylsulfonylgruppe,
eine C₁₋₄-Alkyloxycarbonylgruppe,
eine Formyl-, C₁₋₃-Alkyl-carbonyl-, C₁₋₃-Alkyloxy-C₁₋₃-alkyl-carbonyl-, Tetrahydrofuranylcarbonyl-, Tetrahydropyranylcarbonyl-, Amino-C₁₋₃-alkylcarbonyl-, C₁₋₃-Alkylamino-C₁₋₃-alkyl-carbonyl-, Di-(C₁₋₃-alkyl)amino-C₁₋₃-alkylcarbonyl-, Pyrrolidin-1-yl-C₁₋₃-alkyl-carbonyl-, Piperidin-1-yl-C₁₋₃-alkyl-carbonyl-, Piperazin-1-yl-C₁₋₃-alkyl-carbonyl-, 4-C₁₋₃-Alkyl-piperazin-1-yl-C₁₋₃-alkyl-carbonyl-, Morpholin-4-yl-C₁₋₃-alkyl-carbonyl- oder eine C₁₋₃-Alkylsulfonyl-C₁₋₃-alkyl-carbonylgruppe, oder
eine Cyano-, Aminocarbonyl-, C₁₋₃-Alkyl-aminocarbonyl-, Di-(C₁₋₃-alkyl)aminocarbonyl-, (C₁₋₃-Alkyloxy-C₂₋₄-alkyl)aminocarbonyl-, N-(C₁₋₃-Alkyl)-N-(C₁₋₃-Alkyloxy-C₂₋₄-alkyl)aminocarbonyl-, Phenylaminocarbonyl-, Pyrrolidin-1-ylcarbonyl-, Piperidin-1-ylcarbonyl, Morpholin-4-ylcarbonyl-, C₁₋₃-Alkyl-morpholin-4-ylcarbonyl-, Di-(C₁₋₃-alkyl)morpholin-4-ylcarbonyl-, Homomorpholin-4-ylcarbonyl-, 2-Oxa-5-aza-bicyclo[2.2.1 ]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, Piperazin-1-ylcarbonyl-, 4-(C₁₋₃-alkyl)-piperazin-1-ylcarbonyl-, Aminosulfonyl-, C₁₋₃-Alkyl-aminosulfonyl-, Di-(C₁₋₃-alkyl)amino-sulfonyl-, Pyrrolidin-1-yl-sulfonyl-, Piperidin-1-ylsulfonyl- oder eine Morpholin-4-ylsulfonylgruppe darstellen,
eine Cyclopentylgruppe, die in 3-Stellung durch eine Gruppe R⁶ substituiert ist, wobei
R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydro-pyrimidin-1-yl- oder eine 2-Oxo-3-methyl-hexahydropyrimidin-1-ylgruppe darstellt,
eine Cyclohexylgruppe, die in 3-Stellung oder in 4-Stellung durch eine Gruppe R⁴-N-R⁵ substituiert ist, wobei R⁴ und R⁵ wie vorstehend erwähnt definiert sind,
eine Cyclohexylgruppe, die in 3-Stellung oder in 4-Stellung durch eine Gruppe R⁶ substituiert ist, wobei R⁶ wie vorstehend erwähnt definiert ist,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist, oder
eine Tetrahydrofuran-3-yl-, Tetrahydropyran-3-yl- oder Tetrahydropyran-4-ylgruppe,
R^{d} ein Wasserstoffatom,
eine C₁₋₃-Alkyloxygruppe,
eine Methoxygruppe, die durch ein bis drei Fluoratome substituiert ist,
eine Ethyloxygruppe, die in 2-Stellung durch einen Rest R⁶ oder R⁷ substituiert ist, wobei R⁶ wie vorstehend erwähnt definiert ist und
R⁷ eine Hydroxy-, C₁₋₃-Alkyloxy-, Amino-, C₁₋₃-Alkylamino-, Di-(C₁₋₃-alkyl)amino-, Bis-(2-methoxyethyl)-amino-, Pyrrolidin-1-yl-, Piperidin-1-yl-, Morpholin-4-yl-, Homomorpholin-4-yl-, 2-Oxa-5-aza-bicyclo[2.2.1]hept-5-yl-, 3-Oxa-8-aza-bicyclo-[3.2.1]oct-8-yl-, 8-Oxa-3-aza-bicyclo[3.2.1]oct-3-yl-, Piperazin-1-yl- oder eine 4-C₁₋₃-Alkyl-piperazin-1-ylgruppe, oder
eine Formylamino-, C₁₋₄-Alkylcarbonylamino-, C₁₋₃-Alkyloxy-C₁₋₃-alkyl-carbonylamino-, C₁₋₄-Alkyloxycarbonylamino-, Aminocarbonylamino-, C₁₋₃-Alkylaminocarbonylamino-, Di-(C₁₋₃-alkyl)aminocarbonylamino-, Pyrrolidin-1-ylcarbonylamino-, Piperidin-1-ylcarbonylamino-, Piperazin-1-ylcarbonylamino-, 4-C₁₋₃-Alkyl-piperazin-1-ylcarbonylamino- Morpholin-4-ylcarbonylamino- oder eine C₁₋₄-Alkylsulfonylamino-Gruppe darstellt,
eine Propyloxygruppe, die in 3-Stellung durch einen Rest R⁶ oder R⁷ substituiert ist, wobei R⁶ und R⁷ wie vorstehend erwähnt definiert sind, oder
eine Butyloxygruppe, die in 4-Stellung durch einen Rest R⁶ oder R⁷ substituiert ist, wobei R⁶ und R⁷ wie vorstehend erwähnt definiert sind, und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

3. Bicyclische Heterocyclen der allgemeinen Formel 1 gemäß Anspruch 1, in denen
R^{a} ein Wasserstoffatom,
R^{b} eine 3-Ethinylphenyl-, 3-Bromphenyl-, 3,4-Difluorphenyl- oder 3-Chlor-4-fluor-phenylgruppe,
eine 3-Chlor-4-benzyloxy-phenyl-, 3-Chlor-4-[(3-fluor-benzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{c} eine Cyclohexylgruppe, die in 3-Stellung oder in 4-Stellung durch eine Gruppe R⁴-N-R⁵ substituiert ist, wobei
R⁴ ein Wasserstoffatom,eine Methyl- oder Ethylgruppe und
R⁵ ein Wasserstoffatom, eine Methyl-, Aminocarbonylmethyl-, Methylaminocarbonylmethyl-, Dimethylaminocarbonylmethyl-, Pyrrolidin-1-ylcarbonylmethyl-, Piperidin-1-ylcarbonylmethyl-, Piperazin-1-ylcarbonylmethyl-, 4-Methylpiperazin-1-ylcarbonylmethyl-, Morpholin-4-ylcarbonylmethyl-, 2-(Morpholin-4-yl-carbonyl)ethyl- oder 3-(Morpholin-4-yl-carbonyl)propylgruppe,
eine Ethyl-, Propyl-, 2-Hydroxyethyl-, 3-Hydroxypropyl-, 2-Methoxyethyl-, 3-Methoxypropyl-, 2-(Butyloxycarbonylamino)-ethyl-, 2-Aminoethyl-, 3-Aminopropyl-, 2-(Acetylamino)ethyl-, 3-(Acetylamino)propyl-, 2-(Ethylcarbonylamino)ethyl-, 3-(Ethylcarbonylamino)propyl-, 2-(Propylcarbonylamino)ethyl-, 3-(Propylcarbonylamino)propyl-, 2-(Ethylaminocarbonylamino)ethyl-, 3-(Ethylaminocarbonylamino)propyl-, 2-(Dimethylaminocarbonylamino)ethyl-, 3-(Dimethylaminocarbonylamino)propyl-, 2-(Morpholin-4-ylcarbonylamino)ethyl-, 3-(Morpholin-4-ylcarbonylamino)propyl-, 2-(Methylsulfonyl)ethyl-, 3-(Methylsulfonyl)propyl-, 2-(Methylsulfonylamino)ethyl-oder eine 3-(Methylsulfonylamino)propylgruppe,
eine 2-(2-Oxo-pyrrolidin-1-yl)ethyl-, 2-(2-Oxopiperidin-1-yl)ethyl-, 2-(3-Oxo-morpholin-4-yl)ethyl-, 2-(2-Oxo-imidazolidin-1-yl)ethyl-, 2-(2-Oxo-3-methyl-imidazolidin-1-yl)ethyl-, 2-(2-Oxo-hexahydropyrimidin-1-yl)ethyl- oder eine 2-(2-Oxo-3-methyl-hexahydropyrimidin-1-yl )ethylgruppe,
eine 3-(2-Oxo-pyrrolidin-1-yl)propyl-, 3-(2-Oxopiperidin-1-yl)propyl-, 3-(3-Oxo-morpholin-4-yl)propyl-, 3-(2-Oxo-imidazolidin-1-yl)propyl-, 3-(2-Oxo-3-methyl-imidazolidin-1-yl)propyl-, 3-(2-Oxo-hexahydropyrimidin-1-yl)propyl- oder eine 3-(2-Oxo-3-methyl-hexahydropyrimidin-1-yl)propylgruppe,
eine Methylsulfonyl-, Ethylsulfonyl-, 3-Chlorpropylsulfonyl-, 2-(Morpholin-4-yl)-ethylsulfonyl- oder eine 3-(Morpholin-4-yl)-propylsulfonylgruppe,
eine Propyloxycarbonyl- oder Butyloxycarbonylgruppe,
eine Formyl-, Acetyl-, Ethylcarbonyl-, Propylcarbonyl-, Methoxyacetyl-, (2-Methoxyethyl)carbonyl-, (3-Methoxypropyl)carbonyl-, Tetrahydrofuran-2-ylcarbonyl-, Tetrahydropyran-4-ylcarbonyl-, Aminoacetyl-, Methylaminoacetyl-, Dimethylaminoacetyl-, Morpholin-4-ylacetyl-, [2-(Morpholin-4-yl)ethyl]carbonyl-, [3-(Morpholin-4-yl)propyl]carbonyl- oder eine Methylsulfonylacetylgruppe,
eine Cyano-, Aminocarbonyl-, Methylaminocarbonyl-, Dimethylaminocarbonyl-, Ethylaminocarbonyl-, Diethylaminocarbonyl-, Propylaminocarbonyl-, (2-Methoxyethyl)aminocarbonyl-, N-Methyl-N-(2-methoxyethyl)-aminocarbonyl-, (3-Methoxypropyl)aminocarbonyl-, N-Methyl-N-(3-methoxypropyl)-aminocarbonyl-, Phenylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, 4-Methylpiperazin-1-ylcarbonyl-, Aminosulfonyl-, Methylaminosulfonyl-, Dimethylaminosulfonyl- oder eine Morpholin-4-ylsulfonylgruppe darstellen,
eine Cyclohexylgruppe, die in 3-Stellung oder in 4-Stellung durch eine Gruppe R⁶ substituiert ist, wobei
R⁶ eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydropyrimidin-1-yl- oder eine 2-Oxo-3-methyl-hexahydropyrimidin-1-ylgruppe darstellt,
eine Pyrrolidin-3-ylgruppe, die in 1-Stellung durch den Rest R⁵ substituiert ist, wobei R⁵ wie vorstehend erwähnt definiert ist,
eine Piperidin-3-ylgruppe, die in 1-Stellung durch den Rest R⁵ substituiert ist, wobei
R⁵ wie vorstehend erwähnt definiert ist,
eine Piperidin-4-ylgruppe, die in 1-Stellung durch den Rest R⁵ substituiert ist, wobei R⁵ wie vorstehend erwähnt definiert ist, oder
eine Tetrahydrofuran-3-yl-, Tetrahydropyran-3-yl- oder Tetrahydropyran-4-ylgruppe,
R^{d} ein Wasserstoffatom,
eine Methoxy-, Difluormethoxy- oder Ethyloxygruppe,
eine Ethyloxygruppe, die in 2-Stellung durch einen Rest R⁶ oder R⁷ substituiert ist, wobei R⁶ wie vorstehend erwähnt definiert ist und
R⁷ eine Hydroxy-, Methoxy-, Ethoxy-, Amino-, Dimethylamino-, Diethylamino-, Bis-(2-methoxyethyl)-amino-, Pyrrolidin-1-yl-, Piperidin-1-yl-, Morpholin-4-yl-, Homomorpholin-4-yl-, 2-Oxa-5-aza-bicyclo[2.2.1]hept-5-yl-, 3-Oxa-8-aza-bicyclo[3.2.1]oct-8-yl-, 8-Oxa-3-aza-bicyclo[3.2.1]oct-3-yl-, Piperazin-1-yl-, 4-Methylpiperazin-1-yl- oder 4-Ethylpiperazin-1-ylgruppe, oder
eine Acetylamino-, Ethylcarbonylamino-, Propylcarbonylamino-, Butylcarbonylamino-, Methoxyacetylamino-, Butyloxycarbonylamino-, Ethylaminocarbonylamino-, Dimethylaminocarbonylamino-, Pyrrolidin-1-ylcarbonylamino-, Piperidin-1-ylcarbonylamino-, Morpholin-4-ylcarbonylamino-, Methylsulfonylamino-, Ethylsulfonylamino- oder Butylsulfonylamino-Gruppe darstellt,
eine Propyloxygruppe, die in 3-Stellung durch einen Rest R⁶ oder R⁷ substituiert ist, wobei R⁶ und R⁷ wie vorstehend erwähnt definiert sind, oder
eine Butyloxygruppe, die in 4-Stellung durch einen Rest R⁶ oder R⁷ substituiert ist, wobei R⁶ und R⁷ wie vorstehend erwähnt definiert sind, und
X ein Stickstoffatom bedeuten,
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

4. Bicyclische Heterocyclen der allgemeinen Formel I gemäß Anspruch 1, in denen
R^{a} ein Wasserstoffatom,
R^{b} eine 3-Bromphenyl-, 3,4-Difluorphenyl-, 3-Chlor-4-fluor-phenyl- oder eine 3-Ethinylphenylgruppe, oder
eine 3-Chlor-4-benzyloxy-phenyl-, 3-Chlor-4-[(3-fluorbenzyl)oxy]-phenyl-, 4-(Pyridin-3-yloxy)-phenyl-, 4-[(6-Methyl-pyridin-3-yl)oxy]-phenyl-, 3-Methyl-4-(pyridin-3-yloxy)-phenyl-, 3-Methyl-4-[(6-methyl-pyridin-3-yl)oxy]-phenyl-, 3-Chlor-4-(pyridin-3-yloxy)-phenyl- oder 3-Chlor-4-[(6-methyl-pyridin-3-yl)oxy]-phenyl-Gruppe,
R^{c} eine Cyclohexylgruppe, die in 3-Stellung durch eine Amino-, Acetylamino-, tert.-Butyloxycarbonylamino- oder Methylsulfonylaminogruppe substituiert ist,
eine Cyclohexylgruppe, die in 4-Stellung durch eine Amino-, Methylamino-, Ethylamino, Dimethylamino-, Aminocarbonylmethylamino-, Methylaminocarbonylmethylamino-, Dimethylaminocarbonylmethylamino-, Morpholin-4-ylcarbonylmethylamino-, [3-(Morpholin-4-ylcarbonyl)propyl]amino-, [2-(Methylsulfonyl)ethyl]amino-, [3-(Methylsulfonyl)propyl]amino- oder [2-(Methylsulfonylamino)ethyl]amino-Gruppe substituiert ist,
eine Cyclohexylgruppe, die in 4-Stellung durch eine [2-(2-Oxo-pyrrolidin-1-yl)ethyl]amino-, [2-(2-Oxopiperidin-1-yl)ethyl]amino-, [2-(2-Oxo-imidazolidin-1-yl)ethyl]amino-, [2-(2-Oxo-3-methyl-imidazolidin-1-yl)ethyl]amino-, [2-(2-Oxo-hexahydropyrimidin-1-yl)ethyl]amino- oder [2-(2-Oxo-3-methyl-hexahydropyrimidin-1-yl)ethyl]amino-Gruppe substituiert ist,
eine Cyclohexylgruppe, die in 4-Stellung durch eine [3-(2-Oxo-pyrrolidin-1-yl)propyl]amino-, [3-(2-Oxopiperidin-1-yl)propyl]amino-, [3-(2-Oxo-imidazolidin-1-yl)propyl]amino-, [3-(2-Oxo-3-methyl-imidazolidin-1-yl)propyl]amino-, [3-(2-Oxo-hexahydropyrimidin-1-yl)propyl]amino- oder [3-(2-Oxo-3-methyl-hexahydropyrimidin-1-yl)propyl]amino-Gruppe substituiert ist,
eine Cyclohexylgruppe, die in 4-Stellung durch eine Acetylamino-, N-(Acetyl)-methylamino-, Aminomethylcarbonylamino-, Methylaminomethylcarbonylamino-, Dimethylaminomethylcarbonylamino-, Morpholin-4-ylmethylcarbonylamino-, Methoxyacetylamino-, N-(Methoxyacetyl)-methylamino-, Tetrahydropyran-4-ylcarbonylamino-, N-(Tetrahydropyran-4-ylcarbonyl)-methylamino-, tert.-Butyloxycarbonylamino-, N-(tert.-Butyloxycarbonyl)-methylamino-, Aminocarbonylamino-, Methylaminocarbonylamino-, N-(Ethylaminocarbonyl)-methylamino-, Dimethylaminocarbonylamino-, N-(Dimethylaminocarbonyl)-methylamino-, N-(Piperidin-1-ylcarbonyl)-methylamino-, Morpholin-4-ylcarbonylamino-, N-(Morpholin-4-ylcarbonyl)-methylamino- oder N-(4-Methylpiperazin-1-ylcarbonyl)-methylamino-Gruppe substituiert ist,
eine Cyclohexylgruppe, die in 4-Stellung durch eine 2-Oxo-pyrrolidin-1-yl-, 2-Oxopiperidin-1-yl-, 3-Oxo-morpholin-4-yl-, 2-Oxo-imidazolidin-1-yl-, 2-Oxo-3-methyl-imidazolidin-1-yl-, 2-Oxo-hexahydropyrimidin-1-yl- oder eine 2-Oxo-3-methyl-hexahydropyrimidin-1-yl-Gruppe substituiert ist,
eine Cyclohexylgruppe, die in 4-Stellung durch eine Methylsulfonylamino-, N-(Methylsulfonyl)-methylamino-, Ethylsulfonylamino-, N-(Ethylsulfonyl)-methylamino-, Dimethylaminosulfonylamino-, N-(Dimethylaminosulfonyl)-methylamino-, Morpholin-4-ylsulfonylamino-, N-(Morpholin-4-ylsulfonyl)-methylamino- 3-Chlorpropylsulfonylamino-, [2-(Morpholin-4-yl)-ethyl]sulfonylamino- oder [3-(Morpholin-4-yl)-propyl]sulfonylamino-gruppe substituiert ist,
eine Pyrrolidin-3-ylgruppe,
eine Pyrrolidin-3-ylgruppe, die in 1-Stellung durch eine Methyl-, Acetyl-, Methoxyacetyl-, tert.-Butyloxycarbonyl-, Morpholin-4-ylcarbonyl- oder Methylsulfonylgruppe substituiert ist,
eine Piperidin-3-ylgruppe,
eine Piperidin-3-ylgruppe, die in 1-Stellung durch eine Methyl-, Acetyl-, Methoxyacetyl-, tert.-Butyloxycarbonyl-, Morpholin-4-ylcarbonyl- oder Methylsulfonylgruppe substituiert ist,
eine Piperidin-4-ylgruppe, die in 1-Stellung durch eine Methyl-, Ethyl-, Propyl-, Isopropyl-, 2-Hydroxyethyl-, 2-Methoxyethyl-, 3-Methoxypropyl-, 2-(Methylsulfonyl)-ethyl-, 3-(Methylsulfonyl)-propyl-, 2-(tert.-Butyloxycarbonylamino)-ethyl-, 2-Aminoethyl-, 2-(Acetylamino)-ethyl-, 2-(Ethylcarbonylamino)-ethyl-, 2-(Propylcarbonylamino)-ethyl-, 2-(Ethylaminocarbonylamino)-ethyl-, 2-(Dimethylaminocarbonylamino)-ethyl-, 2-(Morpholin-4-ylcarbonylamino)-ethyl-, 3-(Acetylamino)-propyl-, 3-(Ethylcarbonylamino)-propyl-, 3-(Propylcarbonylamino)-propyl-, 3-(Ethylaminocarbonylamino)-propyl-, 3-(Dimethylaminocarbonylamino)-propyl-, 3-(Morpholin-4-ylcarbonylamino)-propyl-, 2-(Methylsulfonylamino)-ethyl-, 3-(Methylsulfonylamino)-propyl-, (Aminocarbonyl)methyl-, (Methylaminocarbonyl)methyl-, (Dimethylaminocarbonyl)methyl-, (Pyrrolidin-1-ylcarbonyl)methyl-, (Morpholin-4-ylcarbonyl)methyl-, 2-(Morpholin-4-ylcarbonyl)-ethyl- oder 3-(Morpholin-4-ylcarbonyl)-propyl-Gruppe substituiert ist,
eine Piperidin-4-ylgruppe, die in 1-Stellung durch eine 2-(2-Oxo-pyrrolidin-1-yl)-ethyl-, 2-(2-Oxopiperidin-1-yl)-ethyl-, 2-(3-Oxomorpholin-4-yl)-ethyl-, 2-(2-Oxo-imidazolidin-1-yl)-ethyl-, 2-(2-Oxo-3-methyl-imidazolidin-1-yl)-ethyl-, 2-(2-Oxo-hexahydropyrimidin-1-yl)-ethyl- oder 2-(2-Oxo-3-methyl-hexahydropyrimidin-1-yl)-ethyl-Gruppe substituiert ist,
eine Piperidin-4-ylgruppe, die in 1-Stellung durch eine 3-(2-Oxo-pyrrolidin-1-yl)-propyl-, 3-(2-Oxopiperidin-1-yl)-propyl-, 3-(3-Oxomorpholin-4-yl)-propyl-, 3-(2-Oxo-imidazolidin-1-yl)-propyl-, 3-(2-Oxo-3-methyl-imidazolidin-1-yl)-propyl-, 3-(2-Oxo-hexahydropyrimidin-1-yl)-propyl- oder 3-(2-Oxo-3-methyl-hexahydropyrimidin-1-yl)-propyl-Gruppe substituiert ist,
eine Piperidin-4-ylgruppe, die in 1-Stellung durch eine Formyl-, Acetyl-, Methoxyacetyl-, (2-Methoxyethyl)carbonyl-, (3-Methoxypropyl)carbonyl-, Methylsulfonylacetyl-, Aminoacetyl-, Methylaminoacetyl-, (Dimethylamino)acetyl-, (Morpholin-4-yl)acetyl-, [2-(Morpholin-4-yl)-ethyl]carbonyl-, [3-(Morpholin-4-yl)-propyl]carbonyl-, Tetrahydrofuran-2-ylcarbonyl- oder Tetrahydropyran-4-ylcarbonyl-Gruppe substituiert ist,
eine Piperidin-4-ylgruppe, die in 1-Stellung durch eine Cyano-, Aminocarbonyl-, Methylaminocarbonyl-, Ethylaminocarbonyl-, (2-Methoxyethyl)aminocarbonyl-, N-Methyl-N-(2-methoxyethyl)-aminocarbonyl-, (3-Methoxypropyl)aminocarbonyl-, N-Methyl-N-(3-methoxypropyl)-aminocarbonyl-, Isopropylaminocarbonyl-, Phenylaminocarbonyl-, Dimethylaminocarbonyl-, Diethylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 2-Oxa-5-aza-bicyclo[2.2.1]hept-5-ylcarbonyl-, 3-Oxa-8-aza-bicyclo[3.2.1]oct-8-ylcarbonyl-, 8-Oxa-3-aza-bicyclo[3.2.1]oct-3-ylcarbonyl-, 4-Methylpiperazin-1-ylcarbonyl-, Isopropyloxycarbonyl- oder tert.-Butyloxycarbonyl-Gruppe substituiert ist,
eine Piperidin-4-ylgruppe, die in 1-Stellung durch eine Methylsulfonyl-, Ethylsulfonyl-, [2-(Morpholin-4-yl)-ethyl]sulfonyl-, [3-(Morpholin-4-yl)-propyl]sulfonyl-, Aminosulfonyl-, Methylaminosulfonyl-, Dimethylaminosulfonyl- oder Morpholin-4-ylsulfonylgruppe substituiert ist, oder
eine Tetrahydrofuran-3-yl-, Tetrahydropyran-3-yl- oder Tetrahydropyran-4-ylgruppe,
R^{d} ein Wasserstoffatom,
eine Methoxy-, Difluormethoxy- oder Ethyloxygruppe,
eine 2-(Morpholin-4-yl)ethyloxy-, 3-(Morpholin-4-yl)propyloxy- oder 4-(Morpholin-4-yl)butyloxygruppe,
eine 3-(Dimethylamino)propyloxy-, 3-(Diethylamino)propyloxy-, 3-[Bis-(2-methoxyethyl)-amino]propyloxy-, 3-(Piperazin-1-yl)propyloxy-, 3-(4-Methylpiperazin-1-yl)propyloxy-oder 3-(4-Ethylpiperazin-1-yl)propyloxy-Gruppe
eine 3-(Homomorpholin-4-yl)-propyloxy-, 3-(2-Oxa-5-aza-bicyclo[2.2.1]hept-5-yl)-propyloxy-, 3-(3-Oxa-8-aza-bicyclo[3.2.1]oct-8-yl)-propyloxy- oder 3-(8-Oxa-3-aza-bicyclo[3.2.1]oct-3-yl)-propyloxy-Gruppe,
eine 2-(2-Oxo-pyrrolidin-1-yl)-ethyloxy-, 2-(2-Oxopiperidin-1-yl)-ethyloxy-, 2-(3-Oxomorpholin-4-yl)-ethyloxy-, 2-(2-Oxo-imidazolidin-1-yl)-ethyloxy-, 2-(2-Oxo-3-methyl-imidazolidin-1-yl)-ethyloxy-, 2-(2-Oxo-hexahydropyrimidin-1-yl)-ethyloxy- oder 2-(2-Oxo-3-methyl-hexahydropyrimidin-1-yl)-ethyloxy-Gruppe,
eine 3-(2-Oxo-pyrrolidin-1-yl)-propyloxy-, 3-(2-Oxopiperidin-1-yl)-propyloxy-, 3-(3-Oxomorpholin-4-yl)-propyloxy-, 3-(2-Oxo-imidazolidin-1 -yl)-propyloxy-, 3-(2-Oxo-3-methyl-imidazolidin-1-yl)-propyloxy-, 3-(2-Oxo-hexahydropyrimidin-1-yl)-propyloxy- oder 3-(2-Oxo-3-methyl-hexahydropyrimidin-1-yl)-propyloxy-Gruppe,
eine 2-(Methoxy)-ethyloxy-, 2-(tert.-Butyloxycarbonylamino)-ethyloxy-, 2-(Amino)-ethyloxy-, 2-(Acetylamino)-ethyloxy-, 2-(Ethylcarbonylamino)-ethyloxy-, 2-(Propylcarbonylamino)-ethyloxy-, 2-(Isobutylcarbonylamino)-ethyloxy-, 2-(Methoxyacetylamino)-ethyloxy-, 2-(Ethylaminocarbonylamino)-ethyloxy-, 2-(Dimethylaminocarbonylamino)-ethyloxy-, 2-(Pyrrolidin-1-ylcarbonylamino)-ethyloxy-, 2-(Piperidin-1-ylcarbonylamino)-ethyloxy-, 2-(Morpholin-4-ylcarbonylamino)-ethyloxy-, 2-(Methylsulfonylamino)-ethyloxygruppe, 2-(Ethylsulfonylamino)-ethyloxy- oder 2-(Butylsulfonylamino)-ethyloxy-Gruppe, oder
eine 3-(tert.-Butyloxycarbonylamino)-propyloxy-, 3-(Amino)-propyloxy-, 3-(Acetylamino)-propyloxy- oder 3-(Methylsulfonylamino)-propyloxy-Gruppe,
und
X ein Stickstoffatom bedeuten,
deren Tautomere, deren Stereoisomere, deren Gemische und deren Salze.

5. Bicyclische Heterocyclen der allgemeinen Formel I gemäß Anspruch 1, in denen
R^{a} ein Wasserstoffatom,
R^{b} eine 3-Chlor-4-fluor-phenylgruppe oder eine 3-Ethinylphenylgruppe,
R^{c} eine Cyclohexylgruppe, die in 3-Stellung durch eine Amino-, Acetylamino-, tert.-Butyloxycarbonylamino- oder Methylsulfonylaminogruppe substituiert ist,
eine Cyclohexylgruppe, die in 4-Stellung durch eine Amino-, Methylamino-, Dimethylamino-, Acetylamino-, N-(Acetyl)-methylamino-, Methoxyacetylamino-, N-(Methoxyacetyl)-methylamino-, Tetrahydropyran-4-ylcarbonylamino-, N-(Tetrahydropyran-4-ylcarbonyl)-methylamino-, tert.-Butyloxycarbonylamino-, N-(tert.-Butyloxycarbonyl)-methylamino-, N-(Ethylaminocarbonyl)-methylamino-, Dimethylaminocarbonylamino-, N-(Dimethylaminocarbonyl)-methylamino-, N-(Piperidin-1-ylcarbonyl)-methylamino-, Morpholin-4-ylcarbonylamino-, N-(Morpholin-4-ylcarbonyl)-methylamino-, N-(4-Methylpiperazin-1-ylcarbonyl)-methylamino-, Methylsulfonylamino-, N-(Methylsulfonyl)-methylamino-, Ethylsulfonylamino-, N-(Ethylsulfonyl)-methylamino-, Dimethylaminosulfonylamino-, N-(Dimethylaminosulfonyl)-methylamino-, Morpholin-4-ylsulfonylamino-, N-(Morpholin-4-ylsulfonyl)-methylamino-, 3-Chlorpropylsulfonylamino-oder [3-(Morpholin-4-yl)-propyl]sulfonylaminogruppe substituiert ist,
eine Pyrrolidin-3-ylgruppe,
eine Pyrrolidin-3-ylgruppe, die in 1-Stellung durch eine tert.-Butyloxycarbonyl- oder Methylsulfonylgruppe substituiert ist,
eine Piperidin-3-ylgruppe,
eine Piperidin-3-ylgruppe, die in 1-Stellung durch eine tert.-Butyloxycarbonyl- oder Methylsulfonylgruppe substituiert ist,
eine Piperidin-4-ylgruppe,
eine Piperidin-4-ylgruppe, die in 1-Stellung durch eine Methyl-, (Aminocarbonyl)methyl-, (Dimethylaminocarbonyl)methyl-, (Morpholin-4-ylcarbonyl)methyl-, 2-(tert.-Butyloxycarbonylamino)ethyl-, 2-Aminoethyl-, 2-(Acetylamino)ethyl-, 2-(Methylsulfonylamino)ethyl-, Cyano-, Acetyl-, Methoxyacetyl-, (Dimethylamino)acetyl-, (Morpholin-4-yl)acetyl-, Tetrahydropyran-4-ylcarbonyl-, Ethylaminocarbonyl-, Isopropylaminocarbonyl-, Phenylaminocarbonyl-, Dimethylaminocarbonyl-, Diethylaminocarbonyl-, Pyrrolidin-1-ylcarbonyl-, Piperidin-1-ylcarbonyl-, Morpholin-4-ylcarbonyl-, 2-Methylmorpholin-4-ylcarbonyl-, 2,6-Dimethylmorpholin-4-ylcarbonyl-, Homomorpholin-4-ylcarbonyl-, 4-Methylpiperazin-1-ylcarbonyl-, Isopropyloxycarbonyl-, tert.-Butyloxycarbonyl-, Methylsulfonyl-, Dimethylaminosulfonyl- oder Morpholin-4-ylsulfonylgruppe substituiert ist, oder
eine Tetrahydrofuran-3-yl-, Tetrahydropyran-3-yl- oder Tetrahydropyran-4-ylgruppe,
R^{d} ein Wasserstoffatom,
eine Methoxy- oder Ethyloxygruppe,
eine 2-(Morpholin-4-yl)ethyloxy-, 3-(Morpholin-4-yl)propyloxy- oder 4-(Morpholin-4-yl)butyloxygruppe,
eine 2-(3-Methyl-2-oxo-hexahydropyrimidin-1-yl)-ethyloxygruppe,
eine 2-(Methoxy)-ethyloxy-, 2-(tert.-Butyloxycarbonylamino)-ethyloxy-, 2-Aminoethyloxy-, 2-(Acetylamino)-ethyloxy- oder 2-(Methylsulfonylamino)-ethyloxygruppe oder eine 3-(tert.-Butyloxycarbonylamino)-propyloxy-, 3-Amino-propyloxy-, 3-(Acetylamino)-propyloxy- oder 3-(Methylsulfonylamino)-propyloxygruppe,
und
X ein Stickstoffatom bedeuten,
deren Tautomere, deren Stereoisomere, deren Gemische und deren Salze.

6. Folgende Verbindungen der allgemeinen Formel I gemäß Anspruch 1:
(a) 4-[(3-Chlor-4-fluor-phenyl)amino]-6-((S)-tetrahydrofuran-3-yloxy)-7-methoxy-chinazolin,
(b) 4-[(3-Chlor-4-fluor-phenyl)amino]-6-(tetrahydropyran-4-yloxy)-7-methoxy-chinazolin,
(c) 4-[(3-Chlor-4-fluor-phenyl)amino]-6-((*R*)-tetrahydrofuran-3-yloxy)-7-methoxy-chinazolin,
(d) 4-[(3-Chlor-4-fluor-phenyl)amino]-6-(trans-4-amino-cyclohexan-1-yloxy)-7-methoxy-chinazolin,
(e) 4-[(3-Chlor-4-fluor-phenyl)amino]-6-(trans-4-methansulfonylamino-cyclohexan-1-yloxy)-7-methoxy-chinazolin,
(f) 4-[(3-Chlor-4-fluor-phenyl)amino]-6-(piperidin-4-yloxy)-7-methoxy-chinazolin,
(g) 4-[(3-Chlor-4-fluor-phenyl)amino]-6-(1-methansulfonyl-piperidin-4-yloxy)-7-methoxy-chinazolin,
(h) 4-[(3-Chlor-4-fluor-phenyl)amino]-6-(cis-4-([3-(morpholin-4-yl)-propyl]sulfonyl-amino)-cyclohexan-1-yloxy)-7-methoxy-chinazolin,
(i) 4-[(3-Chlor-4-fluor-phenyl)amino]-6-(tetrahydropyran-3-yloxy)-7-methoxy-chinazolin,
(k) 4-[(3-Chlor-4-fluor-phenyl)amino]-6-(trans-4-([3-(morpholin-4-yl)-propyl]sulfonyl-amino)-cyclohexan-1-yloxy)-7-methoxy-chinazolin,
(I) 4-[(3-Chlor-4-fluor-phenyl)amino]-6-(1-methyl-piperidin-4-yloxy)-7-methoxy-chinazolin,
(m) 4-[(3-Chlor-4-fluor-phenyl)amino]-6-{1-[(morpholin-4-yl)carbonyl]-piperidin-4-yl-oxy}-7-methoxy-chinazolin,
(n) 4-[(3-Chlor-4-fluor-phenyl)amino]-6-(1-[(methoxymethyl)carbonyl]-piperidin-4-yl-oxy)-7-methoxy-chinazolin,
(o) 4-[(3-Chlor-4-fluor-phenyl)amino]-6-(1-cyano-piperidin-4-yloxy)-7-methoxy-chinazolin,
(p) 4-[(3-Chlor-4-fluor-phenyl)amino]-6-{1-[(morpholin-4-yl)sulfonyl]-piperidin-4-yl-oxy}-7-methoxy-chinazolin,
(q) 4-[(3-Chlor-4-fluor-phenyl)amino]-6-[1-(2-acetylamino-ethyl)-piperidin-4-yloxy]-7-methoxy-chinazolin,
(r) 4-[(3-Chlor-4-fluor-phenyl)amino]-6-{trans-4-[(dimethylamino)sulfonylamino]-cyclohexan-1-yloxy}-7-methoxy-chinazolin,
(s) 4-[(3-Chlor-4-fluor-phenyl)amino]-6-{trans-4-[(morpholin-4-yl)carbonylamino]-cyclohexan-1-yloxy}-7-methoxy-chinazolin,
(t) 4-[(3-Chlor-4-fluor-phenyl)amino]-6-{trans-4-[(morpholin-4-yl)sulfonylamino]-cyclohexan-1-yloxy}-7 -methoxy-chinazolin,
(u) 4-[(3-Chlor-4-fluor-phenyl)amino]-6-(tetrahydropyran-4-yloxy)-7-(2-acetylamino-ethoxy)-chinazolin,
(v) 4-[(3-Chlor-4-fluor-phenyl)amino]-6-(tetrahydropyran-4-yloxy)-7-(2-methansulfonylamino-ethoxy)-chinazolin und
(w) 4-[(3-Chlor-4-fluor-phenyl)amino]-6-(tetrahydropyran-4-yloxy)-7-(2-methoxy-ethoxy)-chinazolin,
(x) 4-[(3-Chlor-4-fluor-phenyl)amino]-6-(cis-4-(N-[(morpholin-4-yl)carbonyl]-N-methylamino)-cyclohexan-1-yloxy)-7-methoxy-chinazolin
(y) 4-[(3-Chlor-4-fluor-phenyl)amino]-6-(cis-4-{N-[(morpholin-4-yl)sulfonyl]-N-methylamino}-cyclohexan-1-yloxy)-7-methoxy-chinazolin
(z) 4-[(3-Chlor-4-fluor-phenyl)amino]-6-[cis-4-(N-acetyl-N-methyl-amino)-cyclohexan-1-yloxy]-7-methoxy-chinazolin
sowie deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

10. Verfahren zur Herstellung eines Arzneimittels gemäß Anspruch 8, **dadurch gekennzeichnet, daß** auf nicht-chemischem Wege eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel I gemäß den Ansprüchen 1 bis 6, **dadurch gekennzeichnet, daß**
a) eine Verbindung der allgemeinen Formel 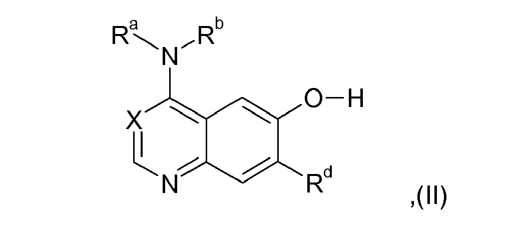 in der
R^{a}, R^{b}, R^{d} und X wie in den Ansprüchen 1 bis 6 erwähnt definiert sind, mit einer Verbindung der allgemeinen Formel
Z¹-R^{c}, (III)
in der
R^{c} wie in den Ansprüchen 1 bis 6 erwähnt definiert ist und Z¹ eine Austrittsgruppe darstellt, umgesetzt wird, oder
b) zur Herstellung von Verbindungen der allgemeinen Formel I, in der R^{d} eine der in den Ansprüchen 1 bis 6 erwähnten, gegebenenfalls substituierten Alkyloxygruppen darstellt,
eine Verbindung der allgemeinen Formel 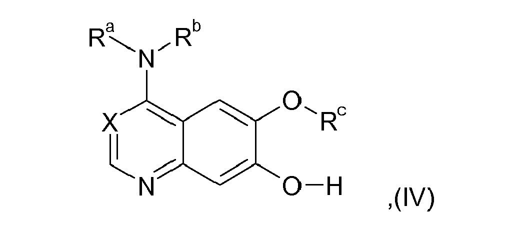 in der R^{a}, R^{b}, R^{c} und X wie in den Ansprüchen 1 bis 6 erwähnt definiert, sind mit einer mit einer Verbindung der allgemeinen Formel
Z²-R^{d'}, (V)
in der R^{d'} eine C₁₋₄-Alkylgruppe, eine durch 1 bis 3 Fluoratome substituierte Methylgruppe, eine durch 1 bis 5 Fluoratome substituierte Ethylgruppe, eine durch einen Rest R⁶ oder R⁷ substituiert C₂₋₄-Alkylgruppe, wobei R⁶ und R⁷ wie in den Ansprüchen 1 bis 6 erwähnt definiert sind, eine C₁₋₄-Alkylgruppe, die durch eine in 1-Stellung durch den Rest R⁸ substituierte Pyrrolidinyl-, Piperidinyl- oder Homopiperidinylgruppe substituiert ist, oder eine C₁₋₄-Alkylgruppe, die durch eine in 4-Stellung durch den Rest R⁸ substituierte Morpholinylgruppe substituiert ist, wobei R⁸ jeweils wie in den Ansprüchen 1 bis 6 erwähnt definiert ist, und
Z² eine Austrittsgruppe darstellt, umgesetzt wird oder
c) zur Herstellung von Verbindungen der allgemeinen Formel I, in der R^{d} eine der in den Ansprüchen 1 bis 6 erwähnten Alkyloxygruppen darstellt, die durch eine gegebenenfalls substituierte Amino-, Alkylamino- oder Dialkylaminogruppe oder durch eine gegebenenfalls substituierte, über ein Iminostickstoffatom gebundene heterocyclischen Gruppe substituiert ist,
miteiner Verbindung der allgemeinen Formel 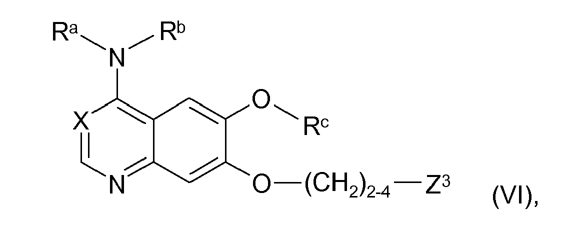
in der R^{a}, R^{b}, R^{c} und X wie in den Ansprüchen 1 bis 6 erwähnt definiert sind und Z³ eine Austrittsgruppe darstellt, mit
Ammoniak, einem entsprechenden, gegebenenfalls substituierten Alkylamin, Dialkylamin oder einer Iminoverbindung oder deren geeigneten Salzen oder Derivaten umgesetzt wird oder
d) zur Herstellung von Verbindungen der allgemeinen Formel I, in der R^{d} eine Hydroxygruppe darstellt,
ein Schutzrestes von einer Verbindung der allgemeinen Formel 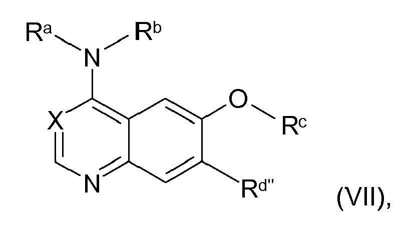 in der R^{a}, R^{b}, R^{c} und X wie in den Ansprüchen 1 bis 6 erwähnt definiert sind und R^{d"} eine in eine Hydroxygruppe überführbare Gruppe darstellt, abgespalten wird oder
e) zur Herstellung von Verbindungen der allgemeinen Formel I, in der R^{c} eine -NH-Gruppe enthält,
ein Schutzrest von einer Verbindung der allgemeinen Formel 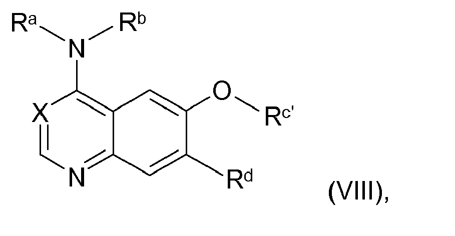 in der R^{a}, R^{b}, R^{d} und X wie in den Ansprüchen 1 bis 6 erwähnt definiert sind und R^{c'} mit der Maßgabe die in den Ansprüchen 1 bis 6 für R^{c} erwähnten Bedeutungen besitzt, daß R^{c} ein geschütztes Stickstoffatom enthält, abgespalten wird oder
f) zur Herstellung von Verbindungen der allgemeinen Formel I, in der R^{c} eine durch eine gegebenenfalls substituierte Amino-, Alkylamino- oder Dialkyaminogruppe oder durch eine über ein Stickstoffatom gebundenene, gegebenenfalls substituierte heterocyclische Gruppe substituierte Alkylgruppe enthält,
ein Verbindung der allgemeinen Formel 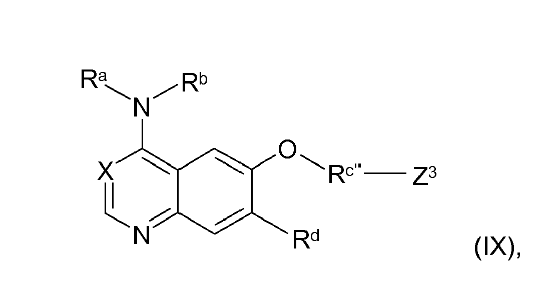
in der R^{a}, R^{b}, R^{d} und X wie in den Ansprüchen 1 bis 6 erwähnt definiert sind, Z³ eine Austrittsgruppe darstellt und R^{c"} mit der Maßgabe, daß ein an ein aliphatisches Kohlenstoffatom gebundenes Wasserstoffatom durch die Gruppe Z³ ersetzt ist, die für R^{c} in den Ansprüchen 1 bis 6 erwähnt Bedeutungen besitzt,
mit Ammoniak, einem entsprechenden, gegenenfalls substituierten Alkylamin, Dialkylamin oder einer Iminoverbindung oder deren geeigneten Salzen oder Derivaten umgesetzt wird und
gewünschtenfalls eine so erhaltene Verbindung der allgemeinen Formel I, die eine Amino-, Alkylamino- oder Iminogruppe enthält, mittels Acylierung, Cyanierung oder Sulfonylierung in eine entsprechende Acyl-, Cyano- oder Sulfonylverbindung der allgemeinen Formel I übergeführt wird und/oder
eine so erhaltene Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt wird und/oder
eine so erhaltene Verbindung der allgemeinen Formel I, die eine Chlor-C₁₋₄-alkylsulfonyl- oder eine Brom-C₁₋₄-alkylsulfonylgruppe enthält, mittels Umsetzung mit einem Amin in eine entsprechende Amino-C₁₋₄-alkylsulfonylverbindung übergeführt wird und/oder
eine so erhaltene Verbindung der allgemeinen Formel I, die eine tert.-Butyloxycarbonylamino-, N-Alkyl-N-(tert.-butyloxycarbonyl)amino- oder eine N-tert.-Butyloxycarbonyliminogruppe enthält, mittels Behandlung mit einer Säure in eine entsprechende Amino-, Alkylamino- oder Iminoverbindung der allgemeinen Formel I übergeführt wird, und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze, übergeführt wird.
